# EUROPÄISCHE PATENTSCHRIFT

(11) **EP 2 467 021 B1**
(45) Veröffentlichungstag und Bekanntmachung des Hinweises auf die Patenterteilung: **28.10.2015**
(21) Anmeldenummer: 10742097.8
(22) Anmeldetag: 07.08.2010
(51) Int. Cl.: A01N 43/653, C07D 249/08, A01P 7/02, A01P 7/04, C07D 403/12, C07C 243/28

(54) **3-[1-(3-HALOALKYL)-TRIAZOLYL]-PHENYL-SULFID-DERIVATE ALS AKARIZIDE UND INSEKTIZIDE**
3-[1-(3-HALOALKYL)-TRIAZOLYL]-PHENYL-SULPHIDE DERIVATIVES AS ACARICIDES AND INSECTICIDES
DÉRIVÉS DE 3-[1-(3-HALOALKYL)-TRIAZOLYL]-PHÉNYLE-SULFURE EN TANT QU'ACARIDES ET INSECTICIDES

(30) Priorität: 20.08.2009 EP 09168287
(43) Veröffentlichungstag der Anmeldung: 27.06.2012
(73) Patentinhaber: Bayer Intellectual Property GmbH, 40789 Monheim am Rhein (DE)
(72) Erfinder: ALIG, Bernd, 53639 Königswinter (DE); ANTONS, Stefan, 51373 Leverkusen (DE); FISCHER, Reiner, 40789 Monheim (DE); LUI, Norbert, 51519 Odenthal (DE); KÖHLER, Adeline, 42105 Wuppertal (DE); VOERSTE, Arnd, 50674 Köln (DE); GÖRGENS, Ulrich, 40882 Ratingen (DE)
(74) Vertreter: BIP Patents
(86) Internationale Anmeldenummer: PCT/EP2010/004843
(87) Internationale Veröffentlichungsnummer: WO 2011/029506

(56) Entgegenhaltungen:
- EP-A2- 0 285 893
- WO-A-99/55668
- WO-A-2006/043635
- WO-A1-2005/097784
- WO-A1-2011/006646
- JP-A- 2007 284 356
- US-A- 4 925 864
- MODERHACK DIETRICH ET AL: "Comparison of N-isomers of 1,2,4-triazolecarboxaldehydes", CHEMIKER ZEITUNG, WILEY - V C H VERLAG GMBH & CO. KGAA, DE, vol. 115, no. 10, 1 January 1991 (1991-01-01), pages 271-275, XP009138488, ISSN: 0009-2894
- DATABASE PUBCHEM [Online] NCBI CID 20661897, 05 Dezember 2007 Database accession no. CID 20661897

## Beschreibung

Die vorliegende Erfindung betrifft neue 3-[1-(3-Haloalkyl)-triazolyl]-phenyl-Sulfid-Derivate, deren Anwendung als Akarizide und Insektizide zur Bekämpfung tierischer Schädlinge und Verfahren zu ihrer Herstellung.

Verschiedene substituierte Phenylheterocyclyl-Sulfid-Derivate und deren insektizide und akarizide Wirkung sind in der Literatur bereits beschrieben in WO 1999/055668, WO 2006/043635 und JP 2007-284356.

Die gemäß den oben genannten Schriften bereits bekannten Wirkstoffe weisen bei ihrer Anwendung Nachteile auf, sei es, dass sie keine oder aber eine nur unzureichende insektizide und/oder akarizide Wirkung gegen tierische Schädlinge insbesondere auch bei niedrigeren Aufwandmengen besitzen.

Aufgabe der vorliegenden Erfindung ist es daher, entsprechende 3-[1-(3-haloalkyl)-triazolyl]-phenyl-Sulfid-Derivate bereitzustellen, welche als Insektizide und/oder Akarizide mit einer zufrieden stellenden oder verbesserten insektiziden und/oder akariziden Wirkung gegen tierische Schädlinge, insbesondere bei niedrigeren Aufwandmengen, mit einer hohen Selektivität und guten Verträglichkeit in Nutzpflanzenkulturen eingesetzt werden können.

Es wurden nun neue Verbindungen der Formel (I) 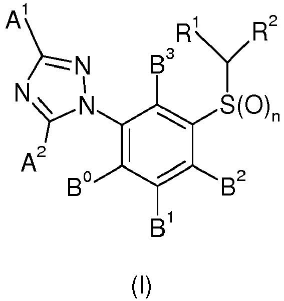 gefunden,
in welcher
- A¹: für CHF₂ steht,
- A²: für Wasserstoff steht,
- B⁰: für Wasserstoff, Amino, Halogen, Cyano, Nitro, Alkyl, Halogenalkyl, Alkylthio, Halogenalkylthio, Alkoxy oder Halogenalkoxy steht,
- B¹, B², B³: unabhängig von einander für Wasserstoff, Halogen, Cyano, Nitro, Alkyl, Halogenalkyl, Cyanoalkyl, Hydroxyalkyl, Alkoxycarbonylalkyl, Alkoxyalkyl, Alkenyl, Halogenalkenyl Cyanoalkenyl, Alkinyl, Halogenalkinyl, Cyanoalkinyl, Alkoxy, Halogenalkoxy, Cyanoalkoxy, Alkoxycarbonylalkoxy, Alkoxyalkoxy, Alkylthio, Halogenalkylthio, Alkoxyalkylthio, Alkylsulfinyl, Halogenalkylsulfinyl, Alkoxyalkylsulfinyl, Alkylsulfonyl, Halogenalkylsulfonyl, Alkoxyalkylsulfonyl, Acyl, Halogenalkylcarbonyl, Carboxyl, Alkoxycarbonyl oder NR³R⁴, wobei R³ und R⁴ unabhängig voneinander für Wasserstoff, Alkyl, Halogenalkyl, Cyanoalkyl, Hydroxyalkyl, Alkoxyalkyl, Alkylthioalkyl, Alkenyl, Halogenalkenyl, Cyanoalkenyl, Alkinyl, Halogenalkinyl, Cyanoalkinyl, Acyl, Alkoxycarbonyl stehen oder R³ und R⁴ gemeinsam mit dem N-Atom, an dem sie gebunden sind, einen gegebenenfalls substituierten und gegebenenfalls durch Heteroatome unterbrochenen gesättigten oder ungesättigten fünf- bis achtgliedrigen Ring bilden können, stehen,
- n: für die Zahl 0, 1 oder 2 steht,
- R¹: für Wasserstoff oder Alkyl steht,
- R²: für Wasserstoff, Halogen, Cyano, Nitro, Alkyl, Halogenalkyl, Cyanoalkyl, Alkoxyalkyl, Alkenyl, Halogenalkenyl, Alkinyl, Halogenalkinyl, Halogenalkoxyalkyl, gegebenenfalls durch ein oder mehrere Heteroatome unterbrochenes gegebenenfalls substituiertes Cycloalkyl oder Cycloalkenyl steht,
wobei die 3-[1-(3-Haloalkyl)-triazolyl]-phenyl-Sulfid-Derivate der Formel (I) nicht 3-(Difluormethyl)-1-{2,4-dimethyl-5-[(2,2,2-trifluorethyl)-sulfanyl]-phenyl}-1H-1,2,4-triazol, 3-(Difluormethyl)-1-{2,4-dimethyl-5-[(2,2,2-trifluorethyl)-sulfinyl]-phenyl}-1H-1,2,4-triazol, 1-{5-[(2,2-Difluorethyl)sulfanyl]-2,4-dimethylphenyl}-3-(difluor-methyl)-1H-1,2,4-triazol, und 1-{5-[(2,2-Difluorethyl)sulfinyl]-2,4-dimethylphenyl}-3-(difluormethyl)-1H-1,2,4-triazol sind.

Die Verbindungen der Formel (I) können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die Verbindungen der Formel (I) umfassen gegebenenfalls Diastereomere oder Enantiomere.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden definiert,
wobei
- A¹: bevorzugt für CHF₂ steht,
- A²: bevorzugt für Wasserstoff steht,
- B⁰: bevorzugt für Wasserstoff, Amino, Halogen, Cyano, Nitro, (C₁-C₆)Alkyl, (C₁-C₆)Halogenalkyl, (C₁-C₆)Alkylthio, (C₁-C₆)Halogenalkylthio, (C₁-C₆)Alkoxy oder (C₁-C₆)Halogenalkoxy steht,
- B¹, B², B³: unabhängig von einander bevorzugt für Wasserstoff, Halogen, Cyano, Nitro, (C₁-C₆)Alkyl, (C₁-C₆)Halogenalkyl, (C₁-C₆)Cyanoalkyl, (C₁-C₆)Hydroxyalkyl, (C₂-C₇)Alkoxycarbonyl-(C₁-C₆)Alkyl, (C₁-C₆)Alkoxy-(C₁-C₆)Alkyl, (C₂-C₆)Alkenyl, (C₂-C₆)Halogenalkenyl (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₂-C₅)Alkoxycarbonyl-(C₁-C₆)Alkoxy, (C₁-C₃)Alkoxy-(C₁-C₆)-Alkoxy, (C₁-C₆)Alkylthio, (C₁-C₆)Halogenalkylthio, (C₁-C₃)Alkoxy-(C₁-C₆)Alkylthio, (C₁-C₆)Alkylsulfinyl, (C₁-C₆)Halogenalkylsulfinyl, (C₁-C₃)Alkoxy-(C₁-C₆)Alkylsulfinyl, (C₁-C₆)Alkylsulfonyl, (C₁-C₆)Halogenalkylsulfonyl, (C₁-C₃)Alkoxy-(C₁-C₆)Alkylsulfonyl, (C₁-C₇)Acyl, (C₂-C₅)Halogenalkylcarbonyl, Carboxyl, (C₂-C₇)Alkoxycarbonyl oder NR³R⁴, wobei R³ und R⁴ unabhängig voneinander für Wasserstoff, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)Alkyl, (C₁-C₆)Alkylthio-(C₁-C₆)Alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₇)Acyl, (C₂-C₇)Alkoxycarbonyl stehen oder R³ und R⁴ gemeinsam mit dem N-Atom, an dem sie gebunden sind, einen gegebenenfalls durch (C₁-C₄)Alkyl, (C₁-C₄)Alkoxy, (C₁-C₄)Halogenalkyl substituierten und gegebenenfalls durch Heteroatome unterbrochenen gesättigten oder ungesättigten fünf- oder sechsgliedrigen Ring bilden können, stehen,
- n: bevorzugt für die Zahl 0, 1 oder 2 steht,
- R¹: bevorzugt für Wasserstoff oder (C₁-C₄)Alkyl steht,
- R²: bevorzugt für Wasserstoff, Halogen, Cyano, Nitro, (C₁-C₆)Alkyl, (C₁-C₆)Halogenalkyl, (C₁-C₆)Cyanoalkyl, (C₂-C₆)Alkenyl, (C₂-C₆)Halogenalkenyl, (C₂-C₆)Alkinyl, (C₂-C₆)Halogenalkinyl, (C₁-C₆)Alkoxy-(C₁-C₆)Alkyl, (C₁-C₆)Halogenalkoxy-(C₁-C₆)Alkyl, gegebenenfalls durch ein oder mehrere Heteroatome unterbrochenes, gegebenenfalls substituiertes (C₃-C₈)Cycloalkyl oder (C₃-C₈)Cycloalkenyl steht.

Die erfindungsgemäßen Verbindungen sind durch die Formel (I) allgemein definiert. Besonders bevorzugte Substituenten bzw. Bereiche der in der oben und nachstehend erwähnten Formeln aufgeführten Reste werden definiert,
wobei
- A¹: besonders bevorzugt für CHF₂ steht,
- A²: besonders bevorzugt für Wasserstoff steht,
- B⁰: besonders bevorzugt für Wasserstoff, Amino, Halogen, Cyano, Nitro, (C₁-C₄)Alkyl, (C₁-C₄)Halogenalkyl, (C₁-C₄)Alkylthio, (C₁-C₄)Halogenalkylthio, (C₁-C₄)Alkoxy oder (C₁-C₄)Halogenalkoxy steht,
- B¹, B², B³: unabhängig von einander besonders bevorzugt für Wasserstoff, Halogen, Cyano, Nitro, (C₁-C₄)Alkyl, (C₁-C₄)Halogenalkyl, (C₂-C₄)Alkenyl, (C₁-C₄)Alkoxy oder (C₁-C₄)Halogenalkoxy stehen,
- n: besonders bevorzugt für die Zahl 0 oder 1 steht,
- R¹: besonders bevorzugt für Wasserstoff oder (C₁-C₂)Alkyl steht,
- R²: besonders bevorzugt für Wasserstoff, Halogen, Cyano, Nitro, (C₁-C₄)Alkyl, (C₁-C₄)Halogenalkyl, (C₁-C₄)Cyanoalkyl, (C₂-C₄)Alkenyl, (C₂-C₄)Halogenalkenyl, (C₂-C₄)Alkinyl, (C₂-C₄)Halogenalkinyl, (C₁-C₄)Alkoxy-(C₁-C₄)Alkyl, (C₁-C₄)Halogenalkoxy-(C₁-C₄)Alkyl, gegebenenfalls durch ein oder mehrere Heteroatome unterbrochenes, gegebenenfalls substituiertes (C₃-C₆)Cycloalkyl oder (C₃-C₆)Cycloalkenyl steht,

Die erfindungsgemäßen Verbindungen sind durch die Formel (I) allgemein definiert. Ganz besonders bevorzugte Substituenten bzw. Bereiche der in der oben und nachstehend erwähnten Formeln aufgeführten Reste werden definiert,
wobei
- A¹: ganz besonders bevorzugt für CHF₂ steht,
- A²: ganz besonders bevorzugt für Wasserstoff steht,
- B⁰: ganz besonders bevorzugt für Wasserstoff, Methyl, Ethyl, Fluor, Chlor, Methoxy, Cyano, CHF₂, CF₃ oder OCF₃ steht,
- B¹: ganz besonders bevorzugt für Wasserstoff steht,
- B²: ganz besonders bevorzugt für Wasserstoff, Methyl, Ethyl, Fluor, Chlor, Methoxy, Cyano, CHF₂, CF₃ oder OCF₃ steht,
- B³: ganz besonders bevorzugt für Wasserstoff steht,
- n: ganz besonders bevorzugt für die Zahl 0 oder 1 steht,
- R¹: ganz besonders bevorzugt für Wasserstoff steht,
- R²: ganz besonders bevorzugt für Wasserstoff, CF₃, Cyano, Methyl, Ethyl, Propyl, Isopropyl, Cyclopropyl, CHF₂, CF₂Cl, CCl₃, CFCl₂, CF₂CF₃, CF₂CHF₂, CH₂CF₃, CH₂CHF₂ oder CF₂CF₂Cl steht.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Gesättigte oder ungesättigte Kohlenwasserstoffreste wie Alkyl, Alkandiyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nicht anderes angegeben ist, einfach oder mehrfach substituiert sein, wobei bei Mehrfachsubstitutionen die Substituenten gleich oder verschieden sein können.

In den als bevorzugt gennanten Restedefinitionen steht Halogen für Fluor, Chlor, Brom und Jod, besonders bevorzugt für Fluor, Chlor und Brom, und ganz besonders bevorzugt für Fluor und Chlor.

Im Einzelnen seien die folgenden Verbindungen der Formel (I-A) genannt (erfindungsgemäß sind Verbindungen mit A¹ = CHF₂, sofern nichts anderers angegeben ist).

**Tabelle 1**

| | | | | |
|---|---|---|---|---|
| 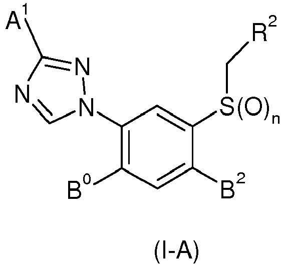 | | | | |

| **A¹** | **B⁰** | **B²** | **R²** | **n** |
|---|---|---|---|---|
| CHF₂ | Me | Me | CF₃ | 0 (nicht erfindungsgemäß) |
| CHF₂ | Me | Me | CHF₂ | 0 (nicht erfindungsgemäß) |
| CHF₂ | Me | Me | CF₂CF₃ | 0 |
| CHF₂ | Me | Me | CF₂CHF₂ | 0 |
| CHF₂ | Me | Me | CF₂CF₂Cl | 0 |
| CHF₂ | Me | Me | CN | 0 |
| CHF₂ | Me | Me | Me | 0 |
| CHF₂ | Me | Me | Pr-i | 0 |
| CHF₂ | Me | Me | Pr-c | 0 |
| CHF₂ | Me | Me | H | 0 |
| CHF₂ | Me | Me | CH₂CF₃ | 0 |
| CHF₂ | Me | Me | CH₂CHF₂ | 0 |
| CHF₂ | Me | Me | CF₃ | 1 (nicht erfindungsgemäß) |
| CHF₂ | Me | Me | CHF₂ | 1 (nicht erfindungsgemäß) |
| CHF₂ | Me | Me | CF₂CF₃ | 1 |
| CHF₂ | Me | Me | CF₂CHF₂ | 1 |
| CHF₂ | Me | Me | CF₂CF₂Cl | 1 |
| CHF₂ | Me | Me | CN | 1 |
| CHF₂ | Me | Me | Me | 1 |
| CHF₂ | Me | Me | Pr-i | 1 |
| CHF₂ | Me | Me | Pr-c | 1 |
| CHF₂ | Me | Me | H | 1 |
| CHF₂ | Me | Me | CH₂CF₃ | 1 |
| CHF₂ | Me | Me | CH₂CHF₂ | 1 |
| CF₂CF₃ | Me | Me | CF₃ | 0 |
| CF₂CF₃ | Me | Me | CHF₂ | 0 |
| CF₂CF₃ | Me | Me | CF₂CF₃ | 0 |
| CF₂CF₃ | Me | Me | CF₂CHF₂ | 0 |
| CF₂CF₃ | Me | Me | CF₂CF₂Cl | 0 |
| CF₂CF₃ | Me | Me | CN | 0 |
| CF₂CF₃ | Me | Me | Me | 0 |
| CF₂CF₃ | Me | Me | Pr-i | 0 |
| CF₂CF₃ | Me | Me | Pr-c | 0 |
| CF₂CF₃ | Me | Me | H | 0 |
| CF₂CF₃ | Me | Me | CH₂CF₃ | 0 |
| CF₂CF₃ | Me | Me | CH₂CHF₂ | 0 |
| CF₂CF₃ | Me | Me | CF₃ | 1 |
| CF₂CF₃ | Me | Me | CHF₂ | 1 |
| CF₂CF₃ | Me | Me | CF₂CF₃ | 1 |
| CF₂CF₃ | Me | Me | CF₂CHF₂ | 1 |
| CF₂CF₃ | Me | Me | CF₂CF₂Cl | 1 |
| CF₂CF₃ | Me | Me | CN | 1 |
| CF₂CF₃ | Me | Me | Me | 1 |
| CF₂CF₃ | Me | Me | Pr-i | 1 |
| CF₂CF₃ | Me | Me | Pr-c | 1 |
| CF₂CF₃ | Me | Me | H | 1 |
| CF₂CF₃ | Me | Me | CH₂CF₃ | 1 |
| CF₂CF₃ | Me | Me | CH₂CHF₂ | 1 |
| CF₂CF₂Cl | Me | Me | CF₃ | 0 |
| CF₂CF₂Cl | Me | Me | CHF₂ | 0 |
| CF₂CF₂Cl | Me | Me | CF₂CF₃ | 0 |
| CF₂CF₂Cl | Me | Me | CF₂CHF₂ | 0 |
| CF₂CF₂Cl | Me | Me | CF₂CF₂Cl | 0 |
| CF₂CF₂Cl | Me | Me | CN | 0 |
| CF₂CF₂Cl | Me | Me | Me | 0 |
| CF₂CF₂Cl | Me | Me | Pr-i | 0 |
| CF₂CF₂Cl | Me | Me | Pr-c | 0 |
| CF₂CF₂Cl | Me | Me | H | 0 |
| CF₂CF₂Cl | Me | Me | CH₂CF₃ | 0 |
| CF₂CF₂Cl | Me | Me | CH₂CHF₂ | 0 |
| CF₂CF₂Cl | Me | Me | CF₃ | 1 |
| CF₂CF₂Cl | Me | Me | CHF₂ | 1 |
| CF₂CF₂Cl | Me | Me | CF₂CF₃ | 1 |
| CF₂CF₂Cl | Me | Me | CF₂CHF₂ | 1 |
| CF₂CF₂Cl | Me | Me | CF₂CF₂Cl | 1 |
| CF₂CF₂Cl | Me | Me | CN | 1 |
| CF₂CF₂Cl | Me | Me | Me | 1 |
| CF₂CF₂Cl | Me | Me | Pr-i | 1 |
| CF₂CF₂Cl | Me | Me | Pr-c | 1 |
| CF₂CF₂Cl | Me | Me | H | 1 |
| CF₂CF₂Cl | Me | Me | CH₂CF₃ | 1 |
| CF₂CF₂Cl | Me | Me | CH₂CHF₂ | 1 |

**Tabelle 2** A¹, R² und n wie in Tabelle 1 angegeben
B⁰ = H; B²= CN

**Tabelle 3** A¹, R² und n wie in Tabelle 1 angegeben
B⁰ = H; B² = Me

**Tabelle 4** A¹, R² und n wie in Tabelle 1 angegeben
B⁰ = H; B² = CHF₂

**Tabelle 5** A¹, R² und n wie in Tabelle 1 angegeben
B⁰ = F; B²= CN

**Tabelle 6** A¹, R² und n wie in Tabelle 1 angegeben
B⁰ = F; B² = Me

**Tabelle 7** A¹, R² und n wie in Tabelle 1 angegeben
B⁰ = F; B² = CHF₂

**Tabelle 8** A¹, R² und n wie in Tabelle 1 angegeben
B⁰=Cl; B²=CN

**Tabelle 9** A¹, R² und n wie in Tabelle 1 angegeben
B⁰=Cl; B²=Me

**Tabelle 10** A¹, R² und n wie in Tabelle 1 angegeben
B⁰ = Cl; B² = CHF₂

**Tabelle 11** A¹, R² und n wie in Tabelle 1 angegeben
B⁰ = Me; B² = Cl

**Tabelle 12** A¹, R² und n wie in Tabelle 1 angegeben
B⁰ = F; B² = Cl

**Tabelle 13** A¹, R² und n wie in Tabelle 1 angegeben
B⁰ = H; B² = Cl

**Tabelle 14** A¹, R² und n wie in Tabelle 1 angegeben
B⁰ = H; B² = CF₃

### Herstellverfahren

Die Verbindungen der allgemeinen Formel (I) können mit den in der Anmeldung WO 1999/055668 beschriebenen Methoden hergestellt werden. Abweichend von diesen beschriebenen Methoden können die Verbindungen der Formel (I) auch nach Verfahren (A) oder (A') hergestellt werden.

### Verfahren A

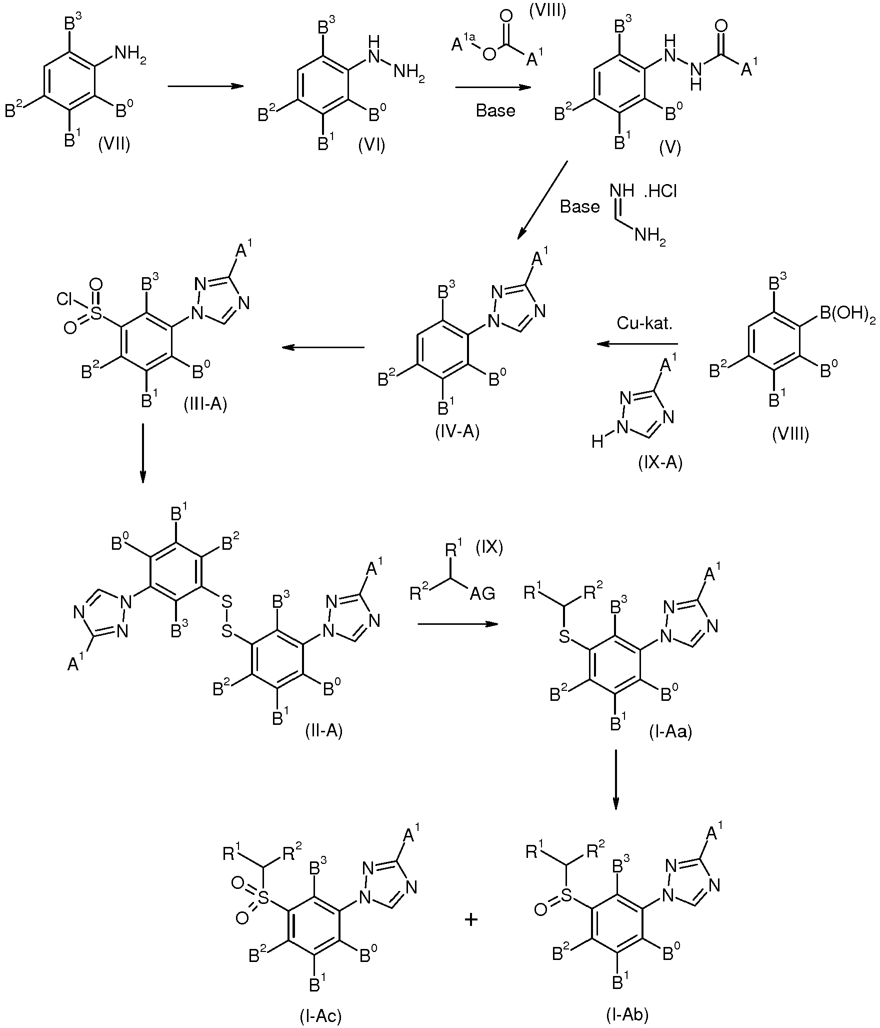

### Verfahren A'

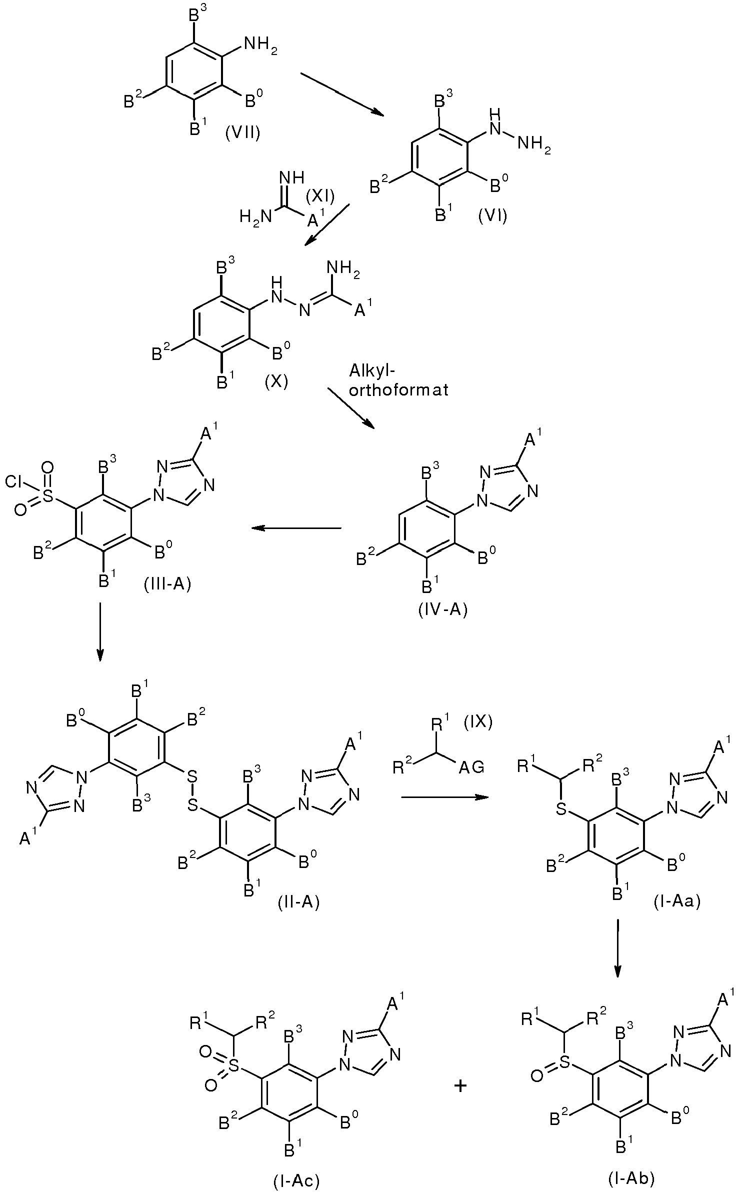

wobei A¹, B⁰, B¹, B², B³, R¹ und R² die oben angegebenen Bedeutungen haben und A^{1a} für Alkyl, bevorzugt für (C₁-C₆)Alkyl steht.

Aniline der Formel (VII) sind entweder kommerziell erhältlich oder können durch bekannte Methoden hergestellt werden. Die Aniline (VII) werden mit Natriumnitrit im Gegenwart von Salzsäure in das entsprechende Diazonium Salze überführt und anschließend mit Zinnchlorid zu Hydrazinen der Formel (VI) reduziert. In Gegenwart von Estern (VIII) werden die Hydrazine (VI) in die entsprechenden Hydrazide (V) überführt. Hydrazide (V) werden mit Formamidin-Hydrochlorid in Gegenwart einer Base wie z. B. Natriumhydrogencarbonat umgesetzt, wobei die Triazole der Formel (IV-A) gebildet werden. Alternativ können die Hydrazine (VI) in die entsprechenden Amidrazone der Formel (X) in Gegenwart von Amidine der Formel (XI) oder Salze davon wie zum Beispiel Amidiniumhydrochloride oder -sulfate überführt werden. Amidrazone der Formel (X) werden mit einem Orthoformat wie zum Beispiel Methylorthoformat oder Ethylorthoformat umgesetzt, wobei die Triazole der Formel (IV-A) entstehen. Die Triazole der Formel (IV-A) können auch über Kupferkatalysierte Kupplungsreaktion mit den Boronsäuren der Formel (VIII) und Triazole der Formel (IX-A) hergestellt werden (vgl. WO 1997055668).Die Sulfochlorierung der Triazole (IV-A) mit Chlorsulfonsäure liefert die entsprechenden Sulfonylchloride (III-A). Die Reduktion der Sulfonylchloride (III-A) in die Disulfide (II-A) ist mit literaturbekannten Methoden wie zum Beispiel Eisen in Salzsäure, Iodwasserstoff oder Iodide möglich. Die Umsetzung der Disulfide mit Elektrophilen der Formel (IX), wobei AG für eine Abgangsgruppe wie Chlor, Brom, Tosylat, Mesylat oder Triflat steht, liefern die Sulfide (I-Aa). Die Thioether werden in die entsprechenden Sulfoxide (I-Ab) und Sulfone (I-Ac) durch Umsetzung mit Oxidationsmitteln wie zum Beispiel *meta-*Chlorperbenzoesäure überführt.

Folgende Zwischenprodukte sind neu und auch Gegenstand der Erfindung.

Verbindungen der Formel (II-A): 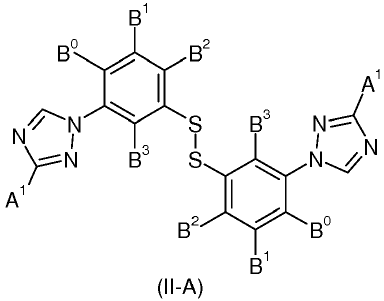 wobei A¹, B⁰ und B² die oben angegebene Bedeutung haben und B¹ und B³ für Wasserstoff stehen.

### Verbindungen der Formel (III-A)

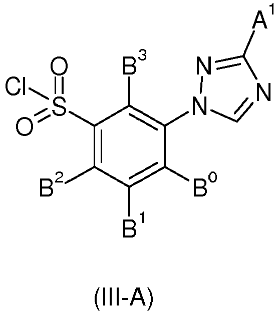

wobei A¹, B⁰ und B² die oben angegebene Bedeutung haben und B¹ und B³ für Wasserstoff stehen.

### Verbindungen der Formel (IV-A)

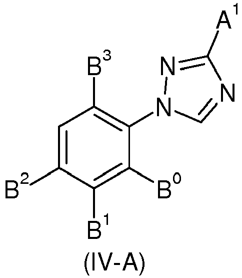

wobei
- B¹ und B³: für Wasserstoff stehen,
- B⁰: steht für Wasserstoff, Methyl, Ethyl, Fluor, Chlor, Methoxy, Cyano, CHF₂, CF₃ oder OCF₃,
- B²: steht für Wasserstoff, Methyl, Ethyl, Fluor, Chlor, Methoxy, Cyano, CHF₂, CF₃ oder OCF₃,

### Verbindungen der Formel (V)

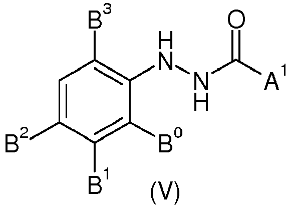

wobei
- B¹ und B³: für Wasserstoff stehen,
- A¹: für CHF₂ steht,
- B⁰: für Methyl, Ethyl, Fluor, Chlor, Methoxy, Cyano, CHF₂, CF₃ oder OCF₃ steht,
- B²: für Wasserstoff, Ethyl, Fluor, Chlor, Cyano, CHF₂, CF₃ oder OCF₃ steht,

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus spp., Aceria sheldoni, Aculops spp., Aculus spp., Amblyomma spp., Amphitetranychus viennensis, Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Halotydeus destructor, Hemitarsonemus spp., Hyalomma spp., Ixodes spp., Latrodectus mactans, Metatetranychus spp., Nuphersa spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acalymma vittatum, Acanthoscelides obtectus, Adoretus spp., Agelastica alni, Agriotes spp., Amphimallon solstitialis, Anobium punctatum, Anoplophora spp., Anthonomus spp., Anthrenus spp., Apion spp., Apogonia spp., Atomaria spp., Attagenus spp., Bruchidius obtectus, Bruchus spp., Cassida spp., Cerotoma trifurcata, Ceutorrhynchus spp., Chaetocnema spp., Cleonus mendicus, Conoderus spp., Cosmopolites spp., Costelytra zealandica, Ctenicera spp., Curculio spp., Cryptorhynchus lapathi, Cylindrocopturus spp., Dermestes spp., Diabrotica spp., Dichocrocis spp., Diloboderus spp., Epilachna spp., Epitrix spp., Faustinus spp., Gibbium psylloides, Hellula undalis, Heteronychus arator, Heteronyx spp., Hylamorpha elegans, Hylotrupes bajulus, Hypera postica, Hypothenemus spp., Lachnosterna consanguinea, Lema spp., Leptinotarsa decemlineata, Leucoptera spp., Lissorhoptrus oryzophilus, Lixus spp., Luperodes spp., Lyctus spp., Megascelis spp., Melanotus spp., Meligethes aeneus, Melolontha spp., Migdolus spp., Monochamus spp., Naupactus xanthographus, Niptus hololeucus, Oryctes rhinoceros, Oryzaephilus surinamensis, Oryzaphagus oryzae, Otiorrhynchus spp., Oxycetonia jucunda, Phaedon cochleariae, Phyllophaga spp., Phyllotreta spp., Popillia japonica, Premnotrypes spp., Psylliodes spp., Ptinus spp., Rhizobius ventralis, Rhizopertha dominica, Sitophilus spp., Sphenophorus spp., Sternechus spp., Symphyletes spp., Tanymecus spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Diplopoda z.B. Blaniulus guttulatus.

Aus der Ordnung der Diptera z.B. Aedes spp., Agromyza spp., Anastrepha spp., Anopheles spp., Asphondylia spp., Bactrocera spp., Bibio hortulanus, Calliphora erythrocephala, Ceratitis capitata, Chironomus spp., Chrysomyia spp., Cochliomyia spp., Contarinia spp., Cordylobia anthropophaga, Culex spp., Cuterebra spp., Dacus oleae, Dasyneura spp., Delia spp., Dermatobia hominis, Drosophila spp., Echinocnemus spp., Fannia spp., Gastrophilus spp., Hydrellia spp., Hylemyia spp., Hyppobosca spp., Hypoderma spp., Liriomyza spp.. Lucilia spp., Musca spp., Nezara spp., Oestrus spp., Oscinella frit, Pegomyia spp., Phorbia spp., Prodiplosis spp., Psila rosae, Rhagoletis spp., Stomoxys spp., Tabanus spp., Tannia spp., Tetanops spp., Tipula spp..

Aus der Klasse der Gastropoda z.B. Arion spp., Biomphalaria spp., Bulinus spp., Deroceras spp., Galba spp., Lynmaea spp., Oncomelania spp., Pomacea spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Collaria spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Monalonion atratum, Nezara spp., Oebalus spp., Pentomidae, Piesma quadrata, Piezodorus spp., Psallus spp., Pseudacysta persea, Rhodnius spp., Sahlbergella singularis, Scaptocoris castanea, Scotinophora spp., Stephanitis nashi, Tibraca spp., Triatoma spp.

Aus der Ordnung der Homoptera z.B. Acyrthosipon spp., Acrogonia spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Drosicha spp., Dysaphis spp., Dysmicoccus spp., Empoasca spp., Eriosoma spp., Erythroneura spp., Euscelis bilobatus, Ferrisia spp., Geococcus coffeae, Hieroglyphus spp., Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva spp., Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes spp., Trioza spp., Typhlocyba spp., Unaspis spp., Viteus vitifolii, Zygina spp..

Aus der Ordnung der Hymenoptera z.B. Athalia spp., Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Acromyrmex spp., Atta spp., Cornitermes cumulans, Microtermes obesi, Odontotermes spp., Reticulitermes spp,

Aus der Ordnung der Lepidoptera z.B. Acronicta major, Adoxophyes spp., Aedia leucomelas, Agrotis spp., Alabama spp., Amyelois transitella, Anarsia spp., Anticarsia spp., Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., Choristoneura spp., Clysia ambiguella, Cnaphalocerus spp., Cnephasia spp., Conopomorpha spp., Conotrachelus spp., Copitarsia spp., Cydia spp., Dalaca noctuides, Diaphania spp., Diatraea saccharalis, Earias spp., Ecdytolopha aurantium, Elasmopalpus lignosellus, Eldana saccharina, Ephestia kuehniella, Epinotia spp., Epiphyas postvittana, Etiella spp., Eulia spp., Eupoecilia ambiguella, Euproctis spp., Euxoa spp., Feltia spp., Galleria mellonella, Gracillaria spp., Grapholitha spp., Hedylepta spp., Helicoverpa spp., Heliothis spp., Hofmannophila pseudospretella, Homoeosoma spp., Homona spp., Hyponomeuta padella, Kakivoria flavofasciata, Laphygma spp., Laspeyresia molesta, Leucinodes orbonalis, Leucoptera spp., Lithocolletis spp., Lithophane antennata, Lobesia spp., Loxagrotis albicosta, Lymantria spp., Lyonetia spp., Malacosoma neustria, Maruca testulalis, Mamestra brassicae, Mocis spp., Mythimna separata, Nymphula spp., Oiketicus spp., Oria spp., Orthaga spp., Ostrinia spp., Oulema oryzae, Panolis flammea, Parnara spp., Pectinophora spp., Perileucoptera spp., Phthorimaea spp., Phyllocnistis citrella, Phyllonorycter spp., Pieris spp., Platynota stultana, Plusia spp., Plutella xylostella, Prays spp., Prodenia spp., Protoparce spp., Pseudaletia spp., Pseudoplusia includens, Pyrausta nubilalis, Rachiplusia nu, Schoenobius spp., Scirpophaga spp., Scotia segetum, Sesamia spp., Sparganothis spp., Spodoptera spp., Stathmopoda spp., Stomopteryx subsecivella, Synanthedon spp., Tecia solanivora, Thermesia gemmatalis, Tinea pellionella, Tineola bisselliella, Tortrix spp., Trichoplusia spp., Tuta absoluta, Virachola spp..

Aus der Ordnung der Orthoptera z.B. Acheta domesticus, Blatta orientalis, Blattella germanica, Dichroplus spp., Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella spp..

Aus der Ordnung der Thysanoptera z.B. Anaphothrips obscurus, Baliothrips biformis, Drepanothris reuteri, Enneothrips flavens, Frankliniella spp., Heliothrips spp., Hercinothrips femoralis, Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Aphelenchoides spp., Bursaphelenchus spp., Ditylenchus spp., Globodera spp., Heterodera spp., Longidorus spp., Meloidogyne spp., Pratylenchus spp., Radopholus similis, Trichodorus spp., Tylenchulus semipenetrans, Xiphinema spp..

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Die vorliegende Erfindung betrifft daher weiterhin Formulierungen und daraus bereitete Anwendungsformen als Pflanzenschutzmittel und/oder Schädlingsbekämpfungsmittel wie z. B. Drench-, Drip- und Spritzbrühen, umfassend mindestens einen der erfindungsgemäßen Wirkstoffe. Gegebenenfalls enthalten die Anwendungsformen weitere Pflanzenschutzmittel und/oder Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und /oder die Retention fördernde Mittel wie z. B. Dioctylsulfosuccinat oder Hydroxypropyl-guar Polymere und/oder Humectants wie z.B. Glycerin und / oder Dünger wie beispielsweise Ammonium-, Kalium- oder Phosphor-enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren erfindungsgemäßen Wirkstoffen weitere agrochemische Wirkstoffe.

Vorzugsweise handelt es sich um Formulierungen oder Anwendungsformen, welche Hilfsstoffe, wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe, wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Grundsätzlich können alle geeigneten Trägerstoffe eingesetzt werden. Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Gegebenenfalls können noch weitere Hilfsstoffe in den Formulierungen und den daraus abgeleiteten Anwendungsformen enthalten sein. Solche Zusatzstoffe sind beispielsweise schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Wirkstoffe mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Betracht, die üblicherweise eingesetzt werden, um das Eindringen agrochemischer Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine ethoxylat (15) oder Ammonium und / oder Phosphonium-Salze wie beispielsweise Ammoniumsulfat oder Dianmionium-hydrogenphosphat.

Die Formulierungen enthalten im allgemeinen zwischen 0,01 und 98 Gew.-% Wirkstoff, vorzugsweise zwischen 0,5 und 90 %.

Die erfindungsgemäßen Wirkstoffe können als solche oder in ihren Formulierungen auch in Mischung mit einem oder mehreren geeigneten Fungiziden, Bakteriziden, Akariziden, Nematiziden, Insektiziden, Mikrobiologika, Düngemittel, Lockstoffen, Phytotonics, Sterilantien, Synergisten, Safenem, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Des Weiteren können solche Kombinationen das Pflanzenwachstum verbessern, die Toleranz gegenüber abiotischen Faktoren wie z. B. hohen oder niedrigen Temperaturen, gegen Trockenheit oder gegen erhöhten Wasser- bzw. Bodensalzgehalt. Auch lässt sich das Blüh- und Fruchtverhalten verbessern, die Keimfähigkeit und Bewurzelung optimieren die Ernte erleichtern und Ernteerträge steigern, die Reife beeinflussen, die Qualität und/oder den Ernährungswert der Ernteprodukte steigern, die Lagerfähigkeit verlängern und/oder die Bearbeitbarkeit der Ernteprodukte verbessern. In der Regel erhält man durch Kombination der erfindungsgemäßen Wirkstoffe und Mischpartnern synergistische Effekte, d.h. die Wirksamkeit der jeweiligen Mischung ist größer als die Wirksamkeit der Einzelkomponenten. Generell können die Kombinationen sowohl in Vor-, Tank- oder Fertigmischungen als auch in Saatgutanwendungen verwendet werden.

Besonders günstige Mischungspartner sind z.B. die folgenden:

### Insektizide/Akarizide/Nematizide

Die hier mit ihrem "common name" genannten Wirkstoffe sind bekannt und beispielsweise im Pestizidhandbuch ("The Pesticide Manual" 14th Ed., British Crop Protection Council 2006) beschrieben oder im Internet recherchierbar (z.B. http://www.alanwood.net/pesticides).
(1) Acetylcholinesterase (AChE) Inhibitoren, wie beispielsweise
   Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; oder
   Organophosphate, z.B. Acephate, Azamethiphos, Azinphos (-methyl, -ethyl), Cadusafos, Chlorethoxyfos, Chlorfenvinphos, Chlormephos, Chlorpyrifos (-methyl), Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Isofenphos, Isopropyl *O*-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion (-methyl), Phenthoate, Phorate, Phosalone, Phosmet, Phosphamidon, Phoxim, Pirimiphos (-methyl), Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Antagonisten, wie beispielsweise
   Organochlorine, z.B. Chlordane und Endosulfan (alpha-); oder
   Fiprole (Phenylpyrazole), z.B. Ethiprole, Fipronil, Pyrafluprole und Pyriprole.
(3) Natrium-Kanal-Modulatoren / Spannungsabhängige Natrium-Kanal-Blocker, wie beispielsweise
   Pyrethroide, z.B. Acrinathrin, Allethrin (d-cis-trans, d-trans), Bifenthrin, Bioallethrin, Bioallethrin-S-cyclopentenyl, Bioresmethrin, Cycloprothrin, Cyfluthrin (beta-), Cyhalothrin (gamma-, lambda-), Cypermethrin (alpha-, beta-, theta-, zeta-), Cyphenothrin [(1*R*)-*trans*-Isomere], Deltamethrin, Dimefluthrin, Empenthrin [(*EZ*)-(1*R*)-Isomere], Esfenvalerate, Etofenprox, Fenpropathrin, Fenvalerate, Flucythrinate, Flumethrin, Fluvalinate (tau-), Halfenprox, Imiprothrin, Metofluthrin, Permethrin, Phenothrin [(1*R*)-trans-Isomer], Prallethrin, Profluthrin, Pyrethrine (pyrethrum), Resmethrin, RU 15525, Silafluofen, Tefluthrin, Tetramethrin [(1*R*)- Isomere], Tralomethrin, Transfluthrin und ZXI 8901; oder
   DDT; oder Methoxychlor.
(4) Nikotinerge Acetylcholin-Rezeptor-Agonisten, wie beispielsweise
   Neonikotinoide, z.B. Acetamiprid, Clothianidin, Dinotefuran, Imidacloprid, Nitenpyram, Thiacloprid, Thiamethoxam; oder
   Nikotin.
(5) Allosterische Acetylcholin-Rezeptor-Modulatoren (Agonisten), wie beispielsweise
   Spinosyne, z.B. Spinetoram und Spinosad.
(6) Chlorid-Kanal-Aktivatoren, wie beispielsweise
   Avermectine/Milbemycine, z.B. Abamectin, Emamectin-benzoate, Lepimectin und Milbemectin.
(7) Juvenilhormon-Analoge, z.B. Hydroprene, Kinoprene, Methoprene; oder Fenoxycarb; Pyriproxyfen.
(8) Wirkstoffe mit unbekannten oder nicht spezifischen Wirkmechanismen, wie beispielsweise
   Begasungsmittel, z.B. Methylbromid und andere Alkylhalogenide; oder
   Chloropicrin; Sulfurylfluorid; Borax; Brechweinstein.
(9) Selektive Fraßhemmer, z.B. Pymetrozine; oder Flonicamid.
(10) Milbenwachstumsinhibitoren, z.B. Clofentezine, Diflovidazin, Hexythiazox, Etoxazole.
(11) Mikrobielle Disruptoren der Insektendarmmembran, wie beispielsweise *Bacillus thuringiensis* Subspezies *israelensis*, *Bacillus sphaericus, Bacillus thuringiensis* Subspezies *aizawai, Bacillus thuringiensis* Subspezies *kurstaki, Bacillus thuringiensis* Subspezies *tenebrionis*, und BT-Pflanzen-Proteine, z.B. Cry1Ab, Cry1Ac, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron; oder
   Organozinnverbindungen, z.B. Azocyclotin, Cyhexatin, Fenbutatin oxide; oder
   Propargite; Tetradifon.
(13) Entkoppler der oxidativen Phoshorylierung durch Unterbrechung des H-Protongradienten, wie beispielsweise Chlorfenapyr und DNOC.
(14) Nikotinerge Acetylcholin-Rezeptor-Antagonisten, wie beispielsweise Bensultap, Cartap (-Hydrochlorid), Thiocylam, und Thiosultap (-sodium).
(15) Inhibitoren der Chitinbiosynthese, Typ 0, wie beispielsweise Benzoylharnstoffe, z.B. Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungsstörende Wirkstoffe, wie beispielsweise Cyromazine.
(18) Ecdysonagonisten/-disruptoren, wie beispielsweise
   Diacylhydrazine, z.B. Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon; Acequinocyl; Fluacrypyrim.
(21) Komplex-I-Elektronentransportinhibitoren, beispielsweise aus der Gruppe der METI-Akarizide, z.B. Fenazaquin, Fenpyroximate, Pyrimidifen, Pyridaben, Tebufenpyrad, Tolfenpyrad; oder Rotenone (Derris).
(22) Spannungsabhängige Natriumkanal-Blocker, z.B. Indoxacarb; Metaflumizone.
(23) Inhibitoren der Acetyl-CoA-Carboxylase, wie beispielsweise Tetronsäure-Derivate, z.B. Spirodiclofen und Spiromesifen; oder Tetramsäure-Derivate, z.B. Spirotetramat.
(24) Komplex-IV-Elektronentransportinhibitoren, wie beispielsweise Phosphine, z.B. Aluminiumphosphid, Kalziumphosphid, Phosphin, Zinkphosphid; oder Cyanid.
(25) Komplex-II-Elektronentransportinhibitoren, wie beispielsweise Cyenopyrafen.
(28) Ryanodinrezeptor-Effektoren, wie beispielsweise Diamide, z.B. Flubendiamide, Chlorantraniliprole (Rynaxypyr), Cyantraniliprole (Cyazypyr) sowie 3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934) oder Methyl-2-[3,5-dibrom-2-({[3-brom-1-(3-chlorpyridin-2-yl)-1H-pyrazol-5-yl]carbonyl}amino)benzoyl]-1,2-dimethylhydrazincarboxylat (bekannt aus WO2007/043677).

Weitere Wirkstoffe mit unbekanntem Wirkmechanismus, wie beispielsweise Azadirachtin, Amidoflumet, Benzoximate, Bifenazate, Chinomethionat, Cryolite, Cyflumetofen, Dicofol, Fluensulfone (5-chloro-2-[(3,4,4-trifluorobut-3-en-1-yl)sulfonyl]-1,3-thiazole), Flufenerim, Pyridalyl und Pyrifluquinazon; desweiteren Präparate auf Basis von Bacillus firmus (I-1582, BioNeem, Votivo) sowie folgende bekannte wirksame Verbindungen

4-{[(6-Brompyrid-3-yl)methyl](2-fluorethyl)amino}furan-2(5H)-on (bekannt aus WO 2007/115644), 4-{[(6-Fluorpyrid-3-yl)methyl](2,2-difluorethyl)amino}furan-2(5H)-on (bekannt aus WO 2007/115644), 4-{[(2-Chlor-1,3-thiazol-5-yl)methyl](2-fluorethyl)amino}furan-2(5H)-on (bekannt aus WO 2007/115644), 4-{[(6-Chlorpyrid-3-yl)methyl](2-fluorethyl)amino}furan-2(5H)-on (bekannt aus WO 2007/ 115644), 4-{[(6-Chlorpyrid-3-yl)methyl](2,2-difluorethyl)amino}furan-2(5H)-on (bekannt aus WO 2007/115644), 4-{[(6-Chlor-5-fluorpyrid-3-yl)methyl](methyl)amino}furan-2(5H)-on (bekannt aus WO 2007/115643), 4-{[(5,6-Dichlorpyrid-3-yl)methyl](2-fluorethyl)amino}furan-2(5H)-on (bekannt aus WO 2007/115646), 4-{[(6-Chlor-5-fluorpyrid-3-yl)methyl](cyclopropyl)amino}furan-2(5H)-on (bekannt aus WO 2007/115643), 4-{[(6-Chlorpyrid-3-yl)methyl](cyclopropyl)amino}furan-2(5H)-on (bekannt aus EP-A-0 539 588), 4-{[(6-Chlorpyrid-3-yl)methyl](methyl)amino}furan-2(5H)-on (bekannt aus EP-A-0 539 588), [(6-Chlorpyridin-3-yl)methyl](methyl)oxido-λ⁴-sulfanylidencyanamid (bekannt aus WO 2007/149134), [1-(6-Chlorpyridin-3-yl)ethyl](methyl)oxido-λ⁴-sulfanylidencyanamid (bekannt aus WO 2007/149134) und seine Diastereomere (A) und (B) 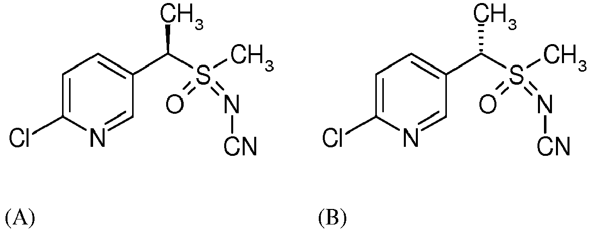 (ebenfalls bekannt aus WO 2007/149134), [(6-Trifluormethylpyridin-3-yl)methyl](methyl)oxido-λ⁴-sulfanylidencyanamid (bekannt aus WO 2007/095229), Sulfoxaflor
(ebenfalls bekannt aus WO 2007/149134), 11-(4-Chlor-2,6-dimethylphenyl)-12-hydroxy-1,4-dioxa-9-azadispiro[4.2.4.2]tetradec-11-en-10-on (bekannt aus WO 2006/089633), 3-(4'-Fluor-2,4-dimethylbiphenyl-3-yl)-4-hydroxy-8-oxa-1-azaspiro[4.5]dec-3-en-2-on (bekannt aus WO 2008/067911),
1-[2-fluoro-4-methyl-5-[(2,2,2-trifluoroethyl)sulfinyl]phenyl]-3-(trifluoromethyl)-1H-1,2,4-Triazol-5-amine (bekannt aus WO 2006/043635),
[(3S,4aR,12R,12aS,12bS)-3-[(Cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methylcyclopropancarboxylat (bekannt aus WO 2006/129714),
2-Cyan-3-(difluormethoxy)-N,N-dimethylbenzolsulfonamid (bekannt aus WO2006/056433),
2-Cyan-3-(difluormethoxy)-N-methylbenzolsulfonamid (bekannt aus WO2006/100288), 2-Cyan-3-(difluormethoxy)-N-ethylbenzolsulfonamid (bekannt aus WO2005/035486), 4-(Difluormethoxy)-N-ethyl-N-methyl-1,2-benzothiazol-3-amin-1,1-dioxid (bekannt aus WO2007/057407) und
N-[1-(2,3-Dimethylphenyl)-2-(3,5-dimethylphenyl)ethyl]-4,5-dihydro-1,3-thiazol-2-amin (bekannt aus WO2008/104503).

### Fungizide

(1) Inhibitoren der Ergosterol-Biosynthese, wie beispielsweise Aldimorph, Azaconazol, Bitertanol, Bromuconazol, Cyproconazol, Diclobutrazol, Difenoconazol, Diniconazol, Diniconazol-M, Dodemorph, Dodemorph Acetat, Epoxiconazol, Etaconazol, Fenarimol, Fenbuconazol, Fenhexamid, Fenpropidin, Fenpropimorph, Fluquinconazol, Flurprimidol, Flusilazol, Flutriafol, Furconazol, Furconazol-Cis, Hexaconazol, Imazalil, Imazalil Sulfat, Imibenconazol, Ipconazol, Metconazol, Myclobutanil, Naftifin, Nuarimol, Oxpoconazol, Paclobutrazol, Pefurazoat, Penconazol, Piperalin, Prochloraz, Propiconazol, Prothioconazol, Pyributicarb, Pyrifenox, Quinconazol, Simeconazol, Spiroxamin, Tebuconazol, Terbinafin, Tetraconazol, Triadimefon, Triadimenol, Tridemorph, Triflumizol, Triforin, Triticonazol, Uniconazol, Uniconazol-p, Viniconazol, Voriconazol, 1-(4-Chlorphenyl)-2-(1H-1,2,4-triazol-1-yl)cycloheptanol, Methyl-1-(2,2-dimethyl-2,3-dihydro-1H-inden-1-yl)-1H-imidazol-5-carboxylat, N'-{5-(Difluormethyl)-2-methyl-4-[3-(trimethylsilyl)propoxy]phenyl}-N-ethyl-N-methylimidoformamid, N-Ethyl-N-methyl-N'-{2-methyl-5-(trifluormethyl)-4-[3-(trimethylsilyl)propoxy]phenyl}imidoformamid und O-[1-(4-Methoxyphenoxy)-3,3-dimethylbutan-2-yl]-1H-imidazol-1-carbothioat.
(2) Inhibitoren der Respiration (Atmungsketten-Inhibitoren), wie beispielsweise Bixafen, Boscalid, Carboxin, Diflumetorim, Fenfuram, Fluopyram, Flutolanil, Fluxapyroxad, Furametpyr, Furmecyclox, Isopyrazam Mischung des syn-epimeren Razemates 1RS,4SR,9RS und des anti-empimeren Razemates 1RS,4SR,9SR, Isopyrazam (anti-epimeres Razemat ), Isopyrazam (anti-epimeres Enantiomer 1R,4S,9S), Isopyrazam (anti-epimeres Enantiomer 1S,4R,9R), Isopyrazam (syn-epimeres Razemat 1RS,4SR,9RS), Isopyrazam (syn-epimeres Enantiomer 1R,4S,9R), Isopyrazam (syn-epimeres Enantiomer 1S,4R,9S), Mepronil, Oxycarboxin, Penflufen, Penthiopyrad, Sedaxane, Thifluzamid, 1-Methyl-N-[2-(1,1,2,2-tetrafluorethoxy)phenyl]-3-(trifluormethyl)-1H-pyrazol-4-carboxamid, 3-(Difluormethyl)-1-methyl-N-[2-(1,1,2,2-tetrafluorethoxy)phenyl]-1H-pyrazol-4-carboxamid, 3-(Difluormethyl)-N-[4-fluor-2-(1,1,2,3,3,3-hexafluorpropoxy)phenyl]-1-methyl-1H-pyrazol-4-carboxamid und N-[1-(2,4-Dichlorphenyl)-1-methoxypropan-2-yl]-3-(difluormethyl)-1-methyl-1H-pyrazol-4-carboxamid.
(3) Inhibitoren der Respiration (Atmungsketten-Inhibitoren) am Komplex III der Atumungskette, wie beispielsweise Ametoctradin, Amisulbrom, Azoxystrobin, Cyazofamid, Dimoxystrobin, Enestroburin, Famoxadon, Fenamidon, Fluoxastrobin, Kresoxim-Methyl, Metominostrobin, Orysastrobin, Picoxystrobin, Pyraclostrobin, Pyrametostrobin, Pyraoxystrobin, Pyribencarb, Trifloxystrobin, (2E)-2-(2-{[6-(3-Chlor-2-methylphenoxy)-5-fluorpyrimidin-4-yl]oxy}phenyl)-2-(methoxyimino)-N-methylethanamid, (2E)-2-(Methoxyimino)-N-methyl-2-(2-{[({(1E)-1-[3-(trifluormethyl)phenyl]ethyliden}amino)oxy]methyl}phenyl)ethanamid, (2E)-2-(Methoxyimino)-N-methyl-2-{2-[(E)-({1-[3-(trifluormethyl)phenyl]ethoxy}imino)methyl]phenyl}ethanamid, (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethanamid, (2E)-2-{2-[({[(2E,3E)-4-(2,6-Dichlorphenyl)but-3-en-2-yliden]amino}oxy)methyl]phenyl}-2-(methoxyimino)-N-methylethanamid, 2-Chlor-N-(1,1,3-trimethyl-2,3-dihydro-1H-inden-4-yl)pyridin-3-carboxamid, 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, Methyl-(2E)-2-{2-[({cyclopropyl[(4-methoxyphenyl)imino]methyl}sulfanyl)methyl]phenyl}-3-methoxyprop-2-enoat, N-(3-Ethyl-3,5,5-trimethylcyclohexyl)-3-(formylamino)-2-hydroxybenzamid, 2-{2-[(2,5-Dimethylphenoxy)methyl]phenyl}-2-methoxy-N-methylacetamid und (2R)-2-{2-[(2,5-Dimethylphenoxy)methyl]phenyl}-2-methoxy-N-methylacetamid.
(4) Inhibitoren der Mitose und Zellteilung, wie beispielsweise Benomyl, Carbendazim, Chlorfenazol, Diethofencarb, Ethaboxam, Fluopicolid, Fuberidazol, Pencycuron, Thiabendazol, Thiophanat-Methyl, Thiophanat, Zoxamid, 5-Chlor-7-(4-methylpiperidin-1-yl)-6-(2,4,6-trifluorphenyl)[1,2,4]triazolo[1,5-a]pyrimidin und 3-Chlor-5-(6-chlorpyridin-3-yl)-6-methyl-4-(2,4,6-trifluorphenyl)pyridazin.
(5) Verbindungen mit Multisite-Aktivität, wie beispielsweise Bordeauxmischung, Captafol, Captan, Chlorothalonil, Kupferzubereitungen wie Kupferhydroxid, Kupfernaphthenat, Kupferoxid, Kupferoxychlorid, Kupfersulfat, Dichlofluanid, Dithianon, Dodine, Dodine freie Base, Ferbam, Fluorofolpet, Folpet, Guazatin, Guazatinacetat, Iminoctadin, Iminoctadinalbesilat, Iminoctadintriacetat, Mankupfer, Mancozeb, Maneb, Metiram, Zinkmetiram, Kupfer-Oxin, Propamidin, Propineb, Schwefel und Schwefelzubereitungen wie beispielsweise Calciumpolysulfid, Thiram, Tolylfluanid, Zineb und Ziram.
(6) Resistenzinduktoren, wie beispielsweise Acibenzolar-S-Methyl, Isotianil, Probenazol und Tiadinil.
(7) Inhibitoren der Aminosäure- und Protein-Biosynthese, wie beispielsweise Andoprim, Blasticidin-S, Cyprodinil, Kasugamycin, Kasugamycin Hydrochlorid Hydrat, Mepanipyrim und Pyrimethanil.
(8) Inhibitoren der ATP Produktion, wie beispielsweise Fentin Acetat, Fentin Chlorid, Fentin Hydroxid und Silthiofam.
(9) Inhibitoren der Zellwandsynthese, wie beispielsweise Benthiavalicarb, Dimethomorph, Flumorph, Iprovalicarb, Mandipropamid, Polyoxins, Polyoxorim, Validamycin A und Valifenalat.
(10) Inhibitoren der Lipid- und Membran-Synthese, wie beispielsweise Biphenyl, Chloroneb, Dicloran, Edifenphos, Etridiazol, Iodocarb, Iprobenfos, Isoprothiolan, Propamocarb, Propamocarb Hydrochlorid, Prothiocarb, Pyrazophos, Quintozen, Tecnazene und Tolclofos-Methyl.
(11) Inhibitoren der Melanin-Biosynthese, wie beispielsweise Carpropamid, Diclocymet, Fenoxanil, Fthalid, Pyroquilon und Tricyclazol.
(12) Inhibitoren der Nukleinsäuresynthese, wie beispielsweise Benalaxyl, Benalaxyl-M (Kiralaxyl), Bupirimat, Clozylacon, Dimethirimol, Ethirimol, Furalaxyl, Hymexazol, Metalaxyl, Metalaxyl-M (Mefenoxam), Ofurace, Oxadixyl, Oxolinsäure.
(13) Inhibitoren der Signaltransduktion, wie beispielsweise Chlozolinat, Fenpiclonil, Fludioxonil, Iprodion, Procymidon, Quinoxyfen und Vinclozolin.
(14) Entkoppler, wie beispielsweise Binapacryl, Dinocap, Ferimzon, Fluazinam und Meptyldinocap.
(15) Weitere Verbindungen, wie beispielsweise Benthiazol, Bethoxazin, Capsimycin, Carvon, Chinomethionat, Chlazafenon, Cufraneb, Cyflufenamid, Cymoxanil, Cyprosulfamide, Dazomet, Debacarb, Dichlorophen, Diclomezin, Difenzoquat, Difenzoquat Methylsulphat, Diphenylamin, Ecomat, Fenpyrazamin, Flumetover, Fluoromid, Flusulfamid, Flutianil, Fosetyl-Aluminium, Fosetyl-Calcium, Fosetyl-Natrium, Hexachlorbenzol, Irumamycin, Methasulfocarb, Methylisothiocyanat, Metrafenon, Mildiomycin, Natamycin, Nickel Dimethyldithiocarbamat, Nitrothal-Isopropyl, Octhilinone, Oxamocarb, Oxyfenthiin, Pentachlorphenol und dessen Salze, Phenothrin, Phosphorsäure und deren Salze, Propamocarb-Fosetylat, Propanosin-Natrium, Proquinazid, Pyrrolnitrin, Tebufloquin, Tecloftalam, Tolnifanid, Triazoxid, Trichlamid, Zarilamid, 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-Methoxyphenoxy)-3,3-dimethylbutan-2-yl-1H-imidazol-1-carboxylat, 2,3,5,6-Tetrachlor-4-(methylsulfonyl)pyridin, 2,3-Dibutyl-6-chlorthieno[2,3-d]pyrimidin-4(3H)-on, 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, 2-Butoxy-6-iod-3-propyl-4H-chromen-4-on, 2-Chlor-5-[2-chlor-1-(2,6-difluor-4-methoxyphenyl)-4-methyl-1H-imidazol-5-yl]pyridin, 2-Phenylphenol und dessen Salze, 3,4,5-Trichlorpyridin-2,6-dicarbonitril, 3-[5-(4-Chlorphenyl)-2,3-dimethyl-1,2-oxazolidin-3-yl]pyridin, 3-Chlor-5-(4-chlorphenyl)-4-(2,6-difluorphenyl)-6-methylpyridazin, 4-(4-Chlorphenyl)-5-(2,6-difluorphenyl)-3,6-dimethylpyridazin, 5-Ammo-1,3,4-thiadiazol-2-thiol, 5-Chlor-N'-phenyl-N'-(prop-2-in-1-yl)thiophen-2-sulfonohydrazid, 5-Methyl-6-octyl[1,2,4]triazolo[1,5-a]pyrimidin-7-amin, Ethyl-(2Z)-3-amino-2-cyan-3-phenylprop-2-enoat, N-(4-Chlorbenzyl)-3-[3-methoxy-4-(prop-2-in-1-yloxy)phenyl]propanamid, N-[(4-Chlorphenyl)(cyan)methyl]-3-[3-methoxy-4-(prop-2-in-1-yloxy)phenyl]propanamid, N-[(5-Brom-3-chlorpyridin-2-yl)methyl]-2,4-dichlorpyridin-3-carboxamid, N-[1-(5-Brom-3-chlorpyridin-2-yl)ethyl]-2,4-dichlorpyridin-3-carboxamid, N-[1-(5-Brom-3-chlorpyridin-2-yl)ethyl]-2-fluor-4-iodpyridin-3-carboxamid, N-{(E)-[(Cyclopropylmethoxy)imino][6-(difluormethoxy)-2,3-difluorphenyl]methyl}-2-phenylacetamid, N-{(Z)-[(Cyclopropylmethoxy)imino][6-(difluormethoxy)-2,3-difluorphenyl]methyl}-2-phenylacetamid, N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazol-4-carboxamid, N-Methyl-2-(1-{[5-methyl-3-(trifluormethyl)-1H-pyrazol-1-yl]acetyl}piperidin-4-yl)-N-[(1R)-1,2,3,4-tetrahydronaphthalen-1-yl]-1,3-thiazol-4-carboxamid, N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazol-4-carboxamid, Pentyl-{6-[({[(1-methyl-1H-tetrazol-5-yl)(phenyl)methyliden]amino}oxy)methyl]pyridin-2-yl}carbamat, Phenazin-1-carbonsäure, Chinolin-8-ol und Chinolin-8-olsulfat(2:1).
(16) Weitere Verbindungen, wie beispielsweise 1-Methyl-3-(trifluormethyl)-N-[2'-(trifluormethyl)biphenyl-2-yl]-1H-pyrazol-4-carboxamid, N-(4'-Chlorbiphenyl-2-yl)-3-(difluormethyl)-1-methyl-1H-pyrazol-4-carboxamid, N-(2',4'-Dichlorbiphenyl-2-yl)-3-(difluormethyl)-1-methyl-1H-pyrazol-4-carboxamid, 3-(Difluormethyl)-1-methyl-N-[4'-(trifluormethyl)biphenyl-2-yl]-1H-pyrazol-4-carboxamid, N-(2',5'-Difluorbiphenyl-2-yl)-1-methyl-3-(trifluormethyl)-1H-pyrazol-4-carboxamid, 3-(Difluormethyl)-1-methyl-N-[4'-(prop-1-in-1-yl)biphenyl-2-yl]-1H-pyrazol-4-carboxamid, 5-Fluor-1,3-dimethyl-N-[4'-(prop-1-in-1-yl)biphenyl-2-yl]-1H-pyrazol-4-carboxamid, 2-Chlor-N-[4'-(prop-1-in-1-yl)biphenyl-2-yl]pyridin-3-carboxamid, 3-(Difluormethyl)-N-[4'-(3,3-dimethylbut-1-in-1-yl)biphenyl-2-yl]-1-methyl-1H-pyrazol-4-carboxamid, N-[4'-(3,3-Dimethylbut-1-in-1-yl)biphenyl-2-yl]-5-fluor-1,3-dimethyl-1H-pyrazol-4-carboxamid, 3-(Difluormethyl)-N-(4'-ethinylbiphenyl-2-yl)-1-methyl-1H-pyrazol-4-carboxamid, N-(4'-Ethinylbiphenyl-2-yl)-5-fluor-1,3-dimethyl-1H-pyrazol-4-carboxamid, 2-Chlor-N-(4'-ethinylbiphenyl-2-yl)pyridin-3-carboxamid, 2-Chlor-N-[4'-(3,3-dimethylbut-1-in-1-yl)biphenyl-2-yl]pyridin-3-carboxamid, 4-(Difluormethyl)-2-methyl-N-[4'-(trifluormethyl)biphenyl-2-yl]-1,3-thiazol-5-carboxamid, 5-Fluor-N-[4'-(3-hydroxy-3-methylbut-1-in-1-yl)biphenyl-2-yl]-1,3-dimethyl-1H-pyrazol-4-carboxamid, 2-Chlor-N-[4'-(3-hydroxy-3-methylbut-1-in-1-yl)biphenyl-2-yl]pyridin-3-carboxamid, 3-(Difluormethyl)-N-[4'-(3-methoxy-3-methylbut-1-in-1-yl)biphenyl-2-yl]-1-methyl-1H-pyrazol-4-carboxamid, 5-Fluor-N-[4'-(3-methoxy-3-methylbut-1-in-1-yl)biphenyl-2-yl]-1,3-dimethyl-1H-pyrazol-4-carboxamid, 2-Chlor-N-[4'-(3-methoxy-3-methylbut-1-in-1-yl)biphenyl-2-yl]pyridin-3-carboxamid, (5-Brom-2-methoxy-4-methylpyridin-3-yl)(2,3,4-trimethoxy-6-methylphenyl)methanon und N-[2-(4-{[3-(4-Chlorphenyl)prop-2-in-1-yl]oxy}-3-methoxyphenyl)ethyl]-N2-(methylsulfonyl)valinamid.

Alle genannten Mischpartner der Klassen (1) bis (16) können, wenn sie auf Grund ihrer funktionellen Gruppen dazu imstande sind, gegebenenfalls mit geeigneten Basen oder Säuren Salze bilden.

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Emährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Emährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Erläuterung der Herstellverfahren und Zwischenprodukte

Die folgenden Herstellungs- und Verwendungsbeispiele illustrieren die Erfindung, ohne sie zu beschränken.

Bei den folgenden Beispielen und Verbindungen, die nicht in den Schutzumfang der Ansprüche fallen, handelt es sich um Vergleichsbeispiele und Vergleichsverbindungen.

### Herstellbeispiel:

### 3-(Difluormethyl)-1-{2,4-dimethyl-5-[(2,2,2-Trifluorethyl)sulfinyl]phenyl}-1H-1,2,4-triazol

### Stufe 1: 2,4-Dimethylhydrazin

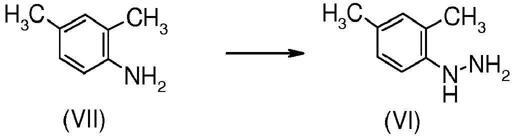

Zu 89 g Salzsäure (32 %ig) und 40 mL Wasser werden 43 g 2,4-Dimethylanilin zugesetzt, nach Abkühlen auf -5 bis -10 °C, werden zu der Suspension eine Lösung aus 25 g Natriumnitrit und 104 mL Wasser zugetropft, hierbei geht das Anilinhydrochlorid langsam in Lösung. Die klare rotbraune Lösung wird mit etwas Amidosulfonsäure nitritfrei gestellt. Diese -10 °C kalte Diazoniumsalzlösung wird zügig zu einer Lösung aus 200 g SnCl₂.H₂0 und 235 g Salzsäure (32 %ig) getropft. Die resultierende weiße Suspension wird bei dieser Temperatur mehrere Stunden nachgerührt und das Zinn-Doppelsalz abgesaugt.

Das Zinn-Doppelsalz wird in mehreren Portionen auf vorgelegte 100 g Natronlauge (45 %ig) und 100 g Wasser gegeben, der pH wird auf 14 gestellt und das Hydrazin durch mehrfache Extraktion mit MTBE/ Ethylacetat Extraktion von der wässrigen Phase abgetrennt. Das Lösungsmittel wird im Vakuum ab destilliert, um das Hydrazin zu erhalten. Nach Umkristallisation aus MTBE werden 45 g (93 % d. Th.) des reinen 2,4-Dimethylhydrazins erhalten
M⁺ : 136
¹H-NMR (D6-DMSO): 6,85-6,97 (m, 3H), 3,7 (m, 3H), 2,25 (s, 3H), 2,1 (s, 3H)

### Stufe 2: N'-(2,4-Dimethylphenyl)-2,2 -difluoracetohydrazid

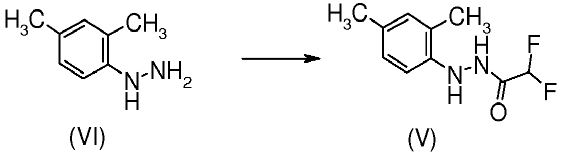

In 100 mL Ethanol werden 26,5 g Difluoressigsäureethylester und 27 g 2,4-Dimethylphenylhydrazin vorgelegt und bei 40 °C gerührt. Nach Abdestillieren der Lösungsmittel wird der Rückstand umkristallisiert. 27,24 g (67 % d. Th.) (V) werden erhalten.
logP: 1,8
M⁺ : 214
¹H-NMR (CDCl₃); 8,05 (s, 1H), 6,93-6,95 (m, 2H); 6,70-6,72 (d, 1H), 5,90-6,17 (t, 1H), 2,25 (s, 3H), 2,24 (s, 3H)

### Stufe 3: 3-(Difluormethyl)-1-(2,4-dimethylphenyl)-1H-1,2,4-triazol

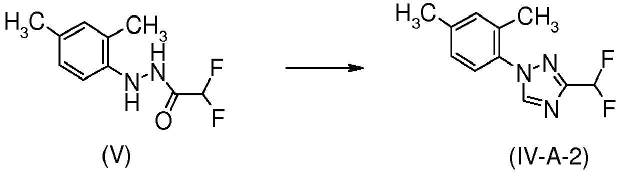

50 g Orthoameisensäuretriethylester und 13,54 g Anmioniumformiat werden vorgelegt und 10 g N'-(2,4-Dimethylphenyl)-2,2-difluoracetohydrazid zugesetzt. Nach 24 Stunden werden die organischen Lösungsmittel abdestilliert und der Rückstand mit verdünnter Salzsäure und Methylenchlorid verrührt. Die Methylenchloridphase wird einrotiert und der kristalline Rückstand aus Cyclohexan umkristallisiert. Ausbeute: 5,6 g (57 % d. Th.)
logP(HCOOH): 2,43
M⁺ :223
¹H-NMR (CDCl₃); 8,27 (s, 1H), 7,12-7,26 (m, 3H), 6,67-6,93 (t, 1H), 2,4 (s, 3H), 2,19 (s, 3H)

### Stufe 4: 5-[3-(Difluormethyl)-1H-1,2,4-triazol-1-yl]-2,4-dimethylbenzolsulfonylchlorid

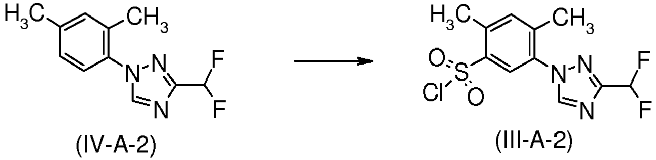

Unter Stickstoff-Atmosphäre werden 14,2 g Chlorsulfonsäure vorgelegt und bei Raumtemperatur 4 g 3-(Difluormethyl)-1-(2,4-dimethylphenyl)-1H-1,2,4-triazol in Portionen zugesetzt. Durch die exotherme Reaktion erwärmt sich die Mischung auf ca 45 °C. Bei 70 °C wird noch 5 h nachgerührt und anschließend abgekühlt und mit 20 mL Methylenchlorid verdünnt. Die Mischung wird unter Rühren auf 40 g Eis gegeben und anschließend die Phasen getrennt und zweimal mit 20 mL Methylenchlorid extrahiert. Die vereinigten organischen Phasen werden einrotiert. Es werden 4,5 g braunes Öl erhalten. Dieses wird mittels CH₂Cl₂ chromatographiert. 2,9 g weißer Feststoff (52 % d. Th) werden isoliert.
M⁺+1: 322
¹H-NMR (CD₃CN): 8,61 (s, 1H), 8,11 (s, 1H), 7,61(s,1H), 6,92 (t, 1H), 2,79 (s, 3H), 2,32 (s,3H)

### Stufe 5: 1,1'-[Disulfanediylbis(4,6-dimethylbenzol-3,1-diyl)]bis[3-(difluormethyl)-1H-1,2,4-triazol]

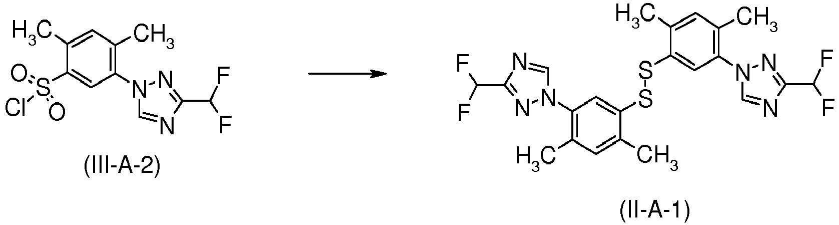

5,4 g 5-[3-(Difluormethyl)-1H-1,2,4-triazol-1-yl]-2,4-dimethylbenzolsulfonylchlorid werden in 25 mL Eisessig gelöst und 4,6 mL Salzsäure (32 %ig) zugesetzt. Die Mischung wird bei 120 °C (Reflux) erhitzt. Anschließend werden 2,53 g Eisenpulver in Portionen zugesetzt. Nach vollständiger Umsetzung wird der Hauptteil an Eisessig abdestilliert und Wasser und Dichlormethan zugesetzt. Nach der Phasentrennung und Einrotieren der organischen Phase werden nach chromatographischer Reinigung (CH₂Cl₂) 1,9 g (49,5 % d. Th) eines weißen Feststoffes erhalten.
M⁺ :508
¹H-NMR (D6-DMSO): 9,00 (s, 2H), 7,62(s, 2H), 7,40 (s, 2H), 7,09- 7,27 (t, 2H), 2,4 (s, 6H), 2,14 (s, 6H)

### Stufe 6: 3-(Difluormethyl)-1-{2,4-dimethyl-5-[(2,2,2-trifluorethyl)sulfanyl]phenyl}-1H-1,2,4-triazol (Verbindung I-A-2)

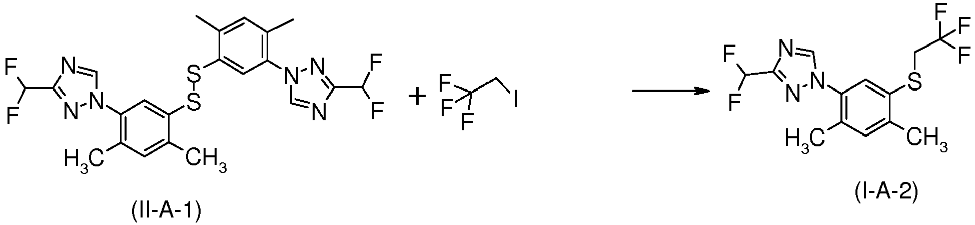

Unter Stickstoff werden 1 g 1,1'-[Disulfanediylbis(4,6-dimethylbenzol-3,1-diyl)]bis[3-(difluormethyl)-1H-1,2,4-triazol] in 25 mL DMF gelöst und mit 1,36 g Natriumdithionit, 0,7 g K₂CO₃ und 0,25 g Rongalit versetzt und 2 Stunden bei 60 °C verrührt, dann werden 0,9 g 2,2,2-Trifluorethanjodid zugegeben und bis zur vollständigen Umsetzung nachgerührt. Die Hauptmenge des DMF's wird im Vakuum abdestilliert und der Rückstand mit Wasser und Methylenchlorid verrührt. Nach Einrotieren der organischen Phase wird der Rückstand chromatographiert. 0,75 g (57 % d. Th.) weißer Feststoff werden erhalten.
logP (HCOOH): 3,33
M⁺: 337
¹H-NMR (D6-DMSO): 9,04 (s,1H), 7,68 (s,1H), 7,38 (s,1H), 7,17- 7,30 (t,1H), 4,03-4,08 (m, 2H), 2,4 (s,3H), 2,17 (s,3H)

### Stufe 7: 3-(Difluormethyl)-1-{2,4-dimethyl-5-[(2,2,2-Trifluorethyl)sulfinyl]phenyl}-1H-1,2,4-triazol (Verbindung I-A-4)

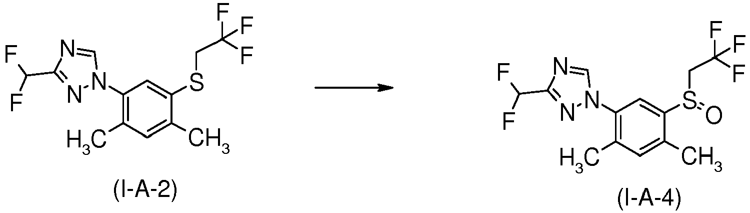

0,5 g 3-(Difluormethyl)-1-{2,4-dimethyl-5-[(2,2,2-trifluorethyl)sulfanyl]phenyl}-1H-1,2,4-triazol werden in 10 mL Dichlormethan und 0,33 g *meta*-Chlorperbenzoesäure in Portionen bei 0-5 °C zugesetzt und bis zum vollständigen Umsatz bei 0 bis 5°C nachgerührt. Dann werden 10 mL Wasser und 3 mL NaHSO₃-Lösung zugesetzt. Die organische Phase wird abgetrennt und zweimal mit 4 mL gesättigter NaHCO₃-Lösung gewaschen. Nach der Phasentrennung wird das Lösungsmittel am Rotationsverdampfer entfernt und der Rückstand wird mit CH₂Cl₂/MTBE (19:1) chromatographiert. Es werden 0,25 g (47,7 % d. Th) (I-A-4) erhalten.
logP (HCOOH): 2,19
M⁺ :353
¹H-NMR (CD₃CN): 8,58 (s,1H), 7,89 (s,1H), 7,40 (s,1H), 6,78-7,44 (m,1H), 3,55-3,75 (m, 2H), 2,42 (s,3H), 2,26 (s,3H).

Gemäß den oben beschriebenen Herstellverfahren lassen sich die Verbindungen der Formel (I) erhalten, beispielsweise die folgenden Verbindungen der Formel (I) (erfindungsgemäß sind Verbindungen mit A¹ = CHF₂, sofern nichts anderers angegeben ist, mit Ausnahme der Verbindungen I-A-1, I-A-2, I-A-3 und I-A-4):

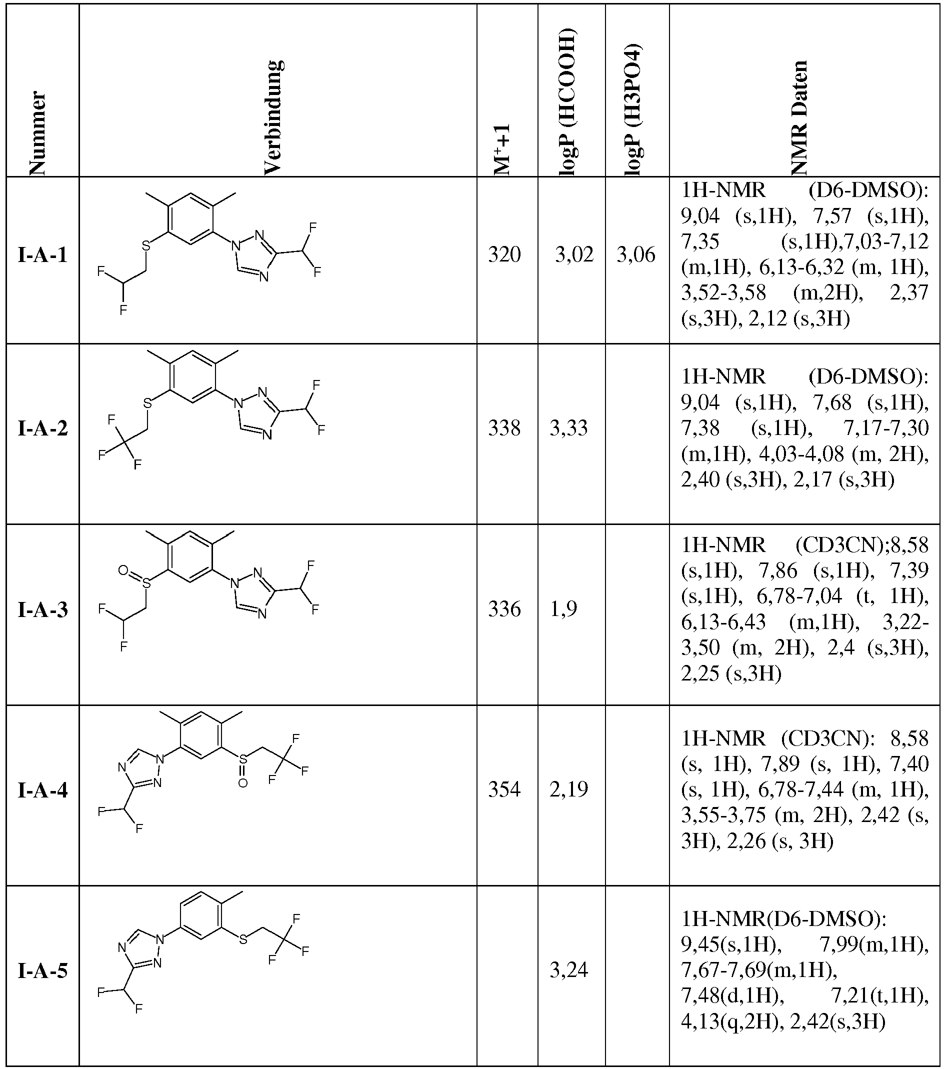

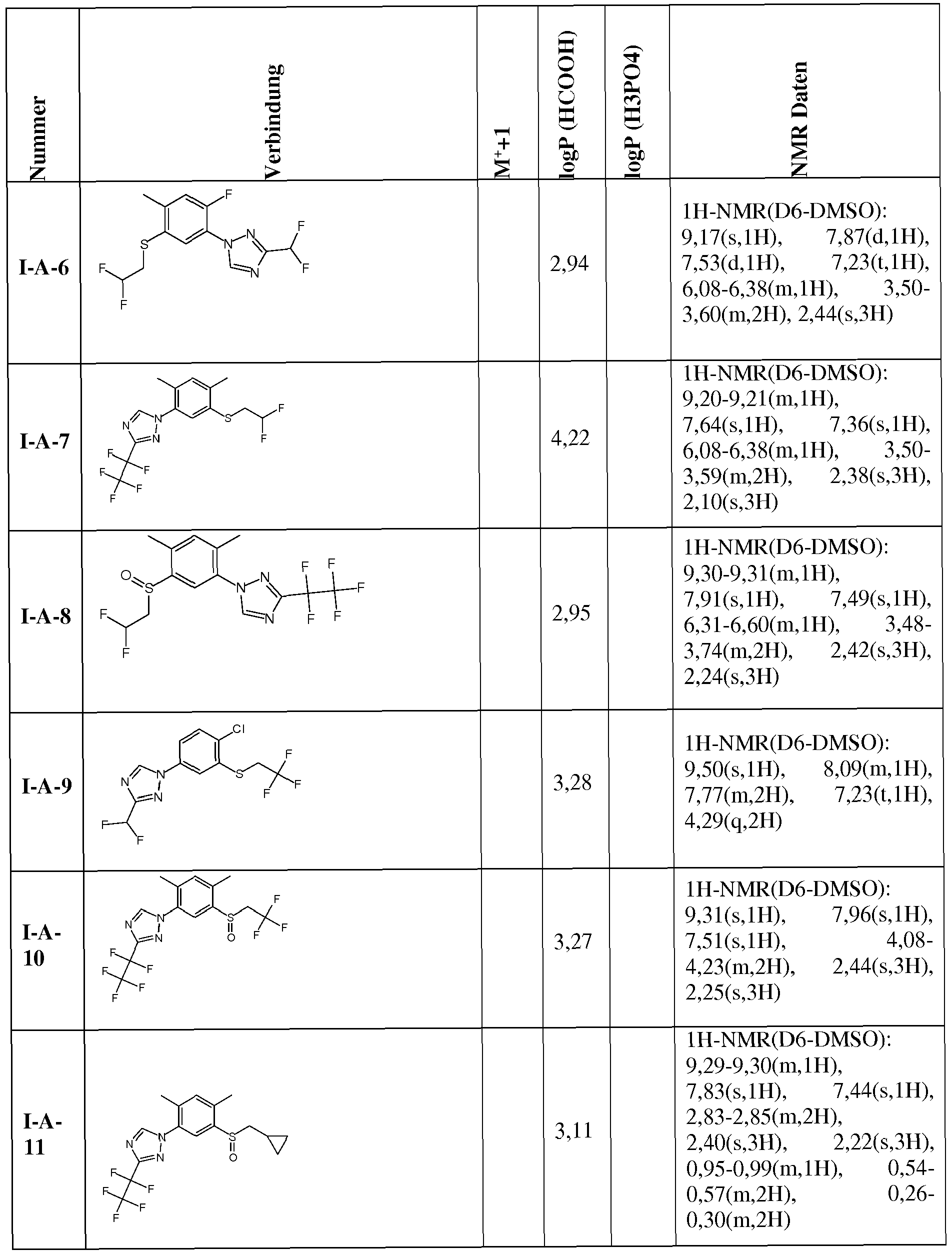

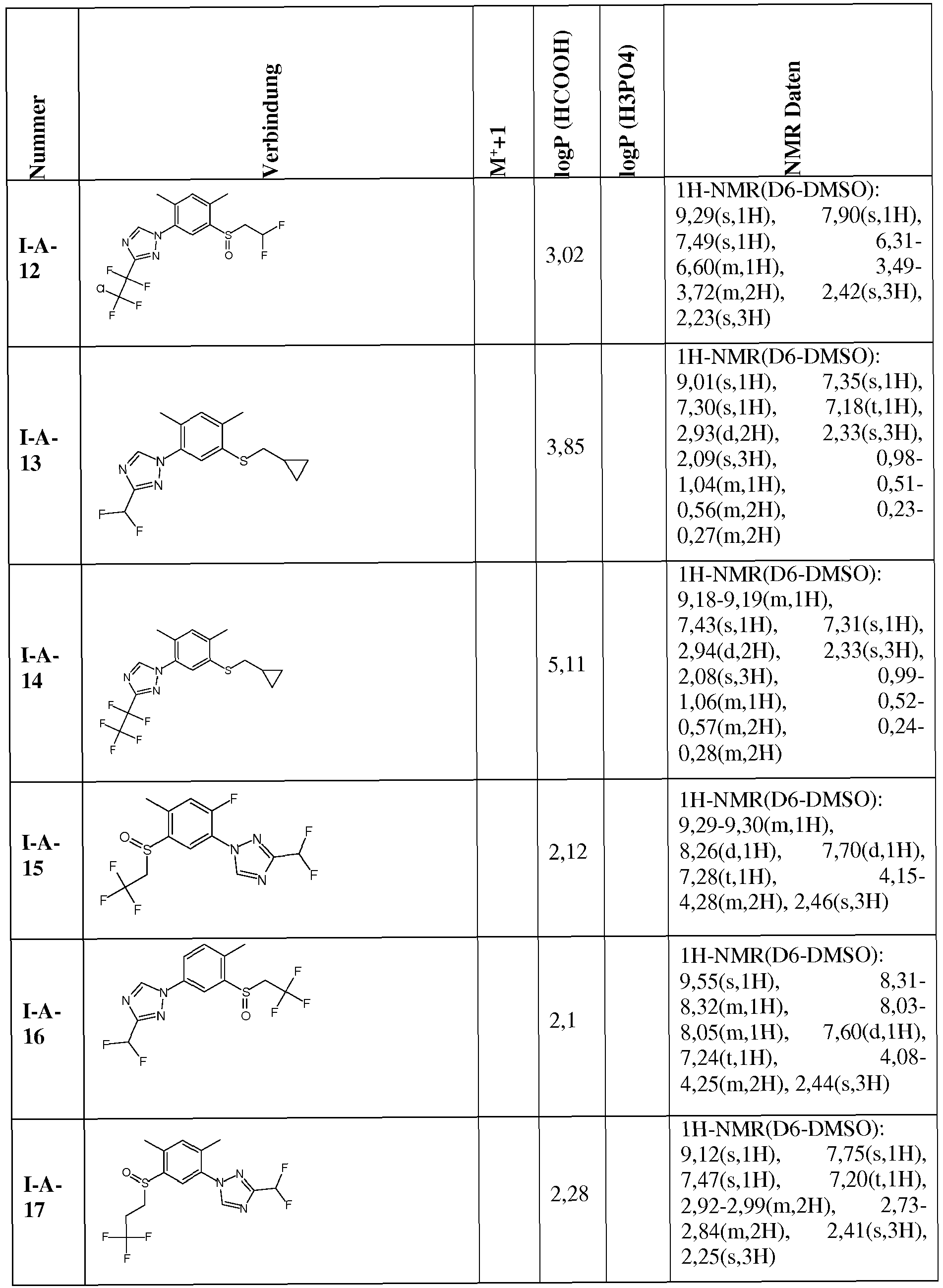

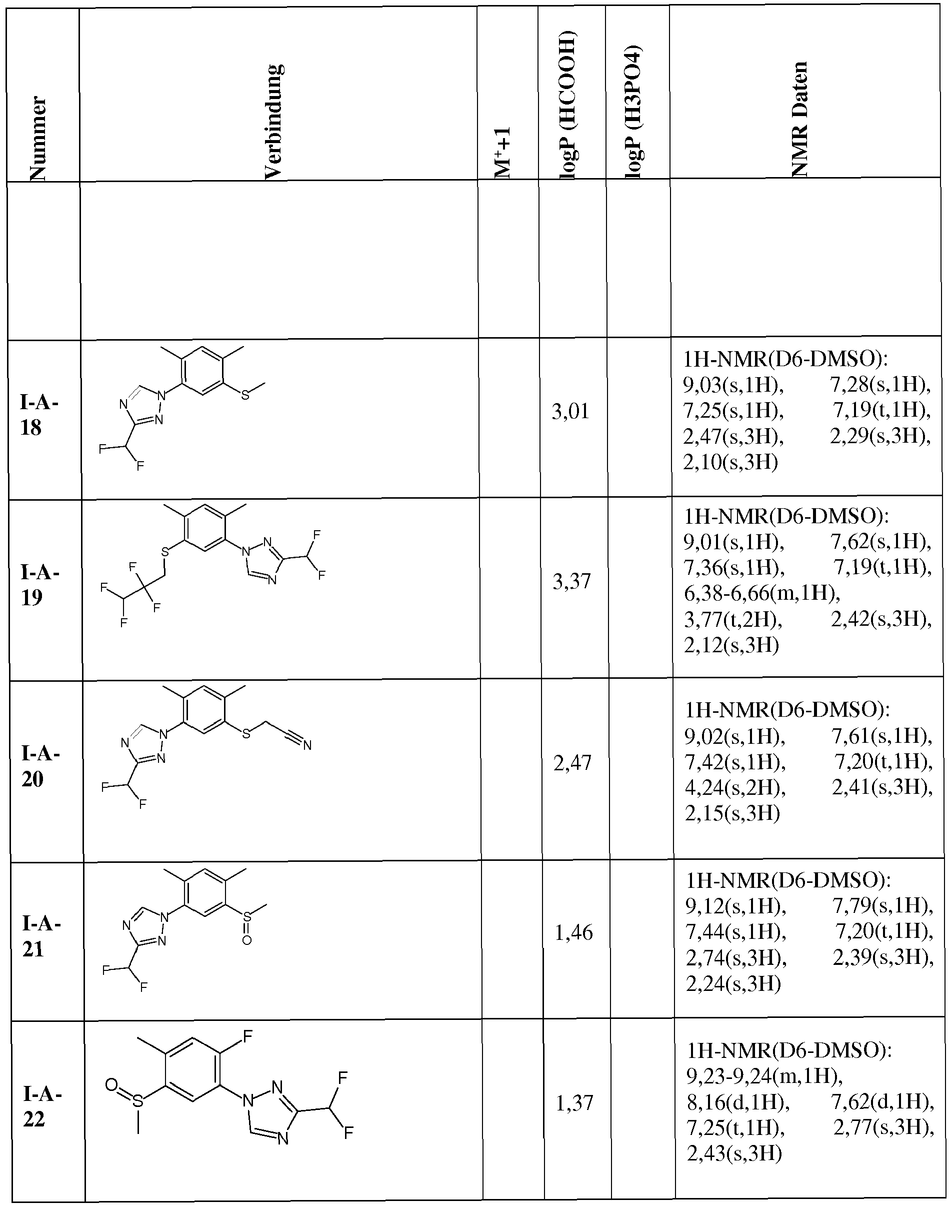

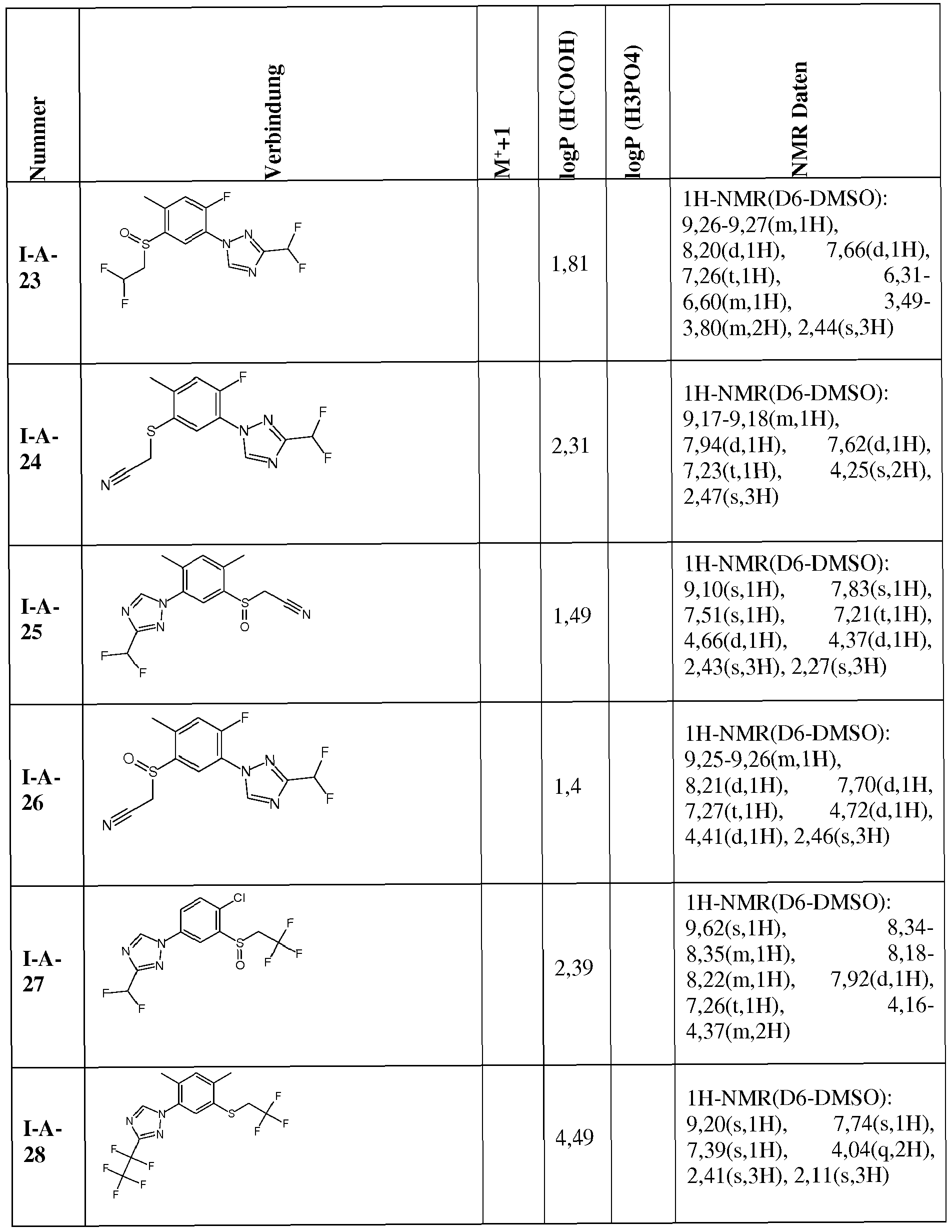

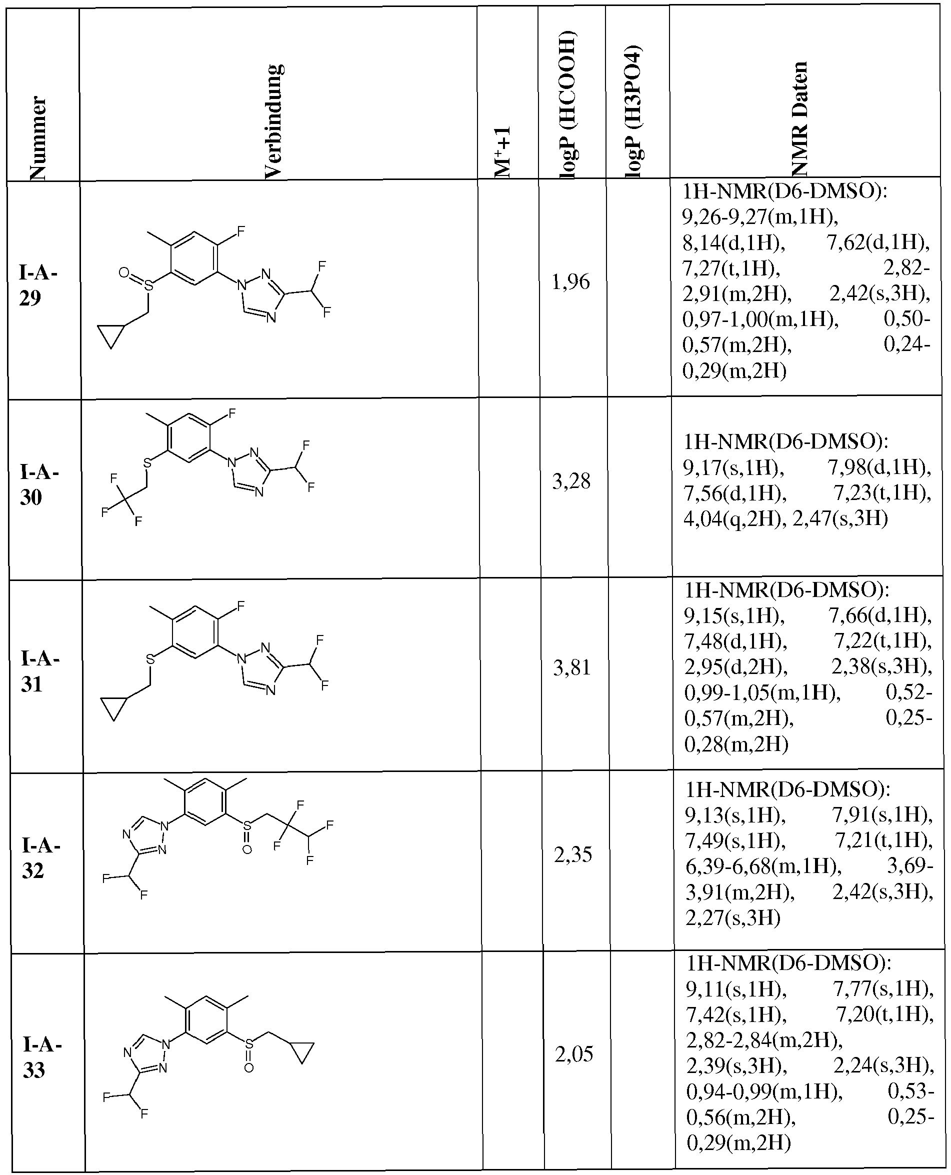

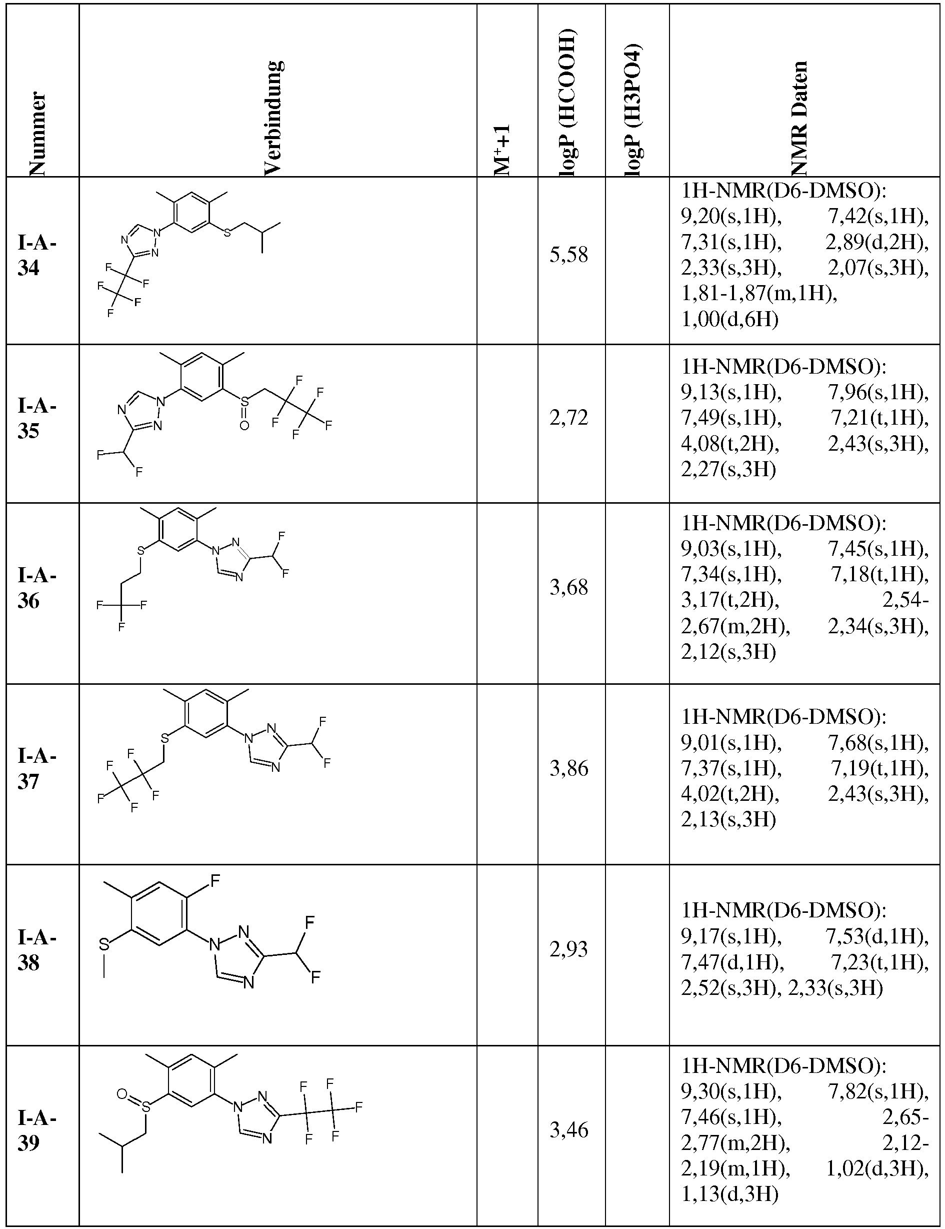

Gemäß den oben beschriebenen Herstellverfahren lassen sich die Verbindungen der Formel (II-A) erhalten, beispielsweise die folgenden Verbindungen der Formel (II-A) (erfindungsgemäß sind Verbindungen mit A¹ = CHF₂, sofern nichts anderers angegeben ist):

| **Nummer** | **Verbindung** | **logP (HCOOH)** | **NMR Daten** |
|---|---|---|---|
| **II-A-1** | 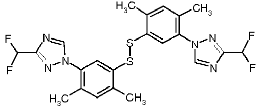 | 4,45 | 1H-NMR (D6-DMSO): 9,00 (s, 2H), 7,62(s, 2H), 7,40 (s, 2H), 7,09- 7,27 (m, 2H), 2,4 (s, 6H), 2,14 (s, 6H) |
| **II-A-2** | 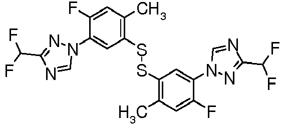 | 4,33 | 1H-NMR(D6-DMSO): 9,11(s,2H), 7,91(d,2H), 7,60(d,2H), 7,16(t,2H), 2,46(s,6H) |
| **II-A-3** | 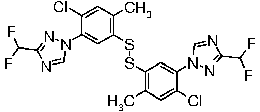 | 4,53 | 1H-NMR(D6-DMSO): 9,07(s,2H), 7,87(s,2H), 7,75(s,2H), 7,18(t,2H), 2,45(s,6H) |
| **II-A-4** | 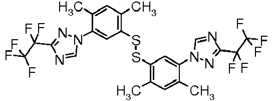 | 6,36 | 1H-NMR(D6-DMSO): 9,12(s,2H), 7,68(s,2H), 7,41(s,2H), 2,41(s,6H), 2,12(s,6H) |
| **II-A-5** | 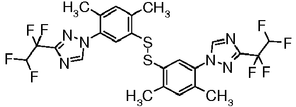 | 5,22 | 1H-NMR(D6-DMSO): 9,06(s,2H), 7,65(s,2H), 7,40(s,2H), 6,79-7,07(m,2H), 2,40(s,6H), 2,12(s,6H) |
| **II-A-6** | 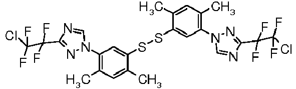 | 6,62 | 1H-NMR(D6-DMSO): 9,10(s,2H), 7,67(s,2H), 7,40(s,2H), 2,40(s,6H), 2,12(s,6H) |
| **II-A-7** | 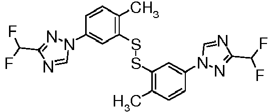 | 4,19 | 1H-NMR(D6-DMSO): 9,38(s,2H), 8,05-8,06(m,2H), 7,75-7,77(m,2H), 7,50(d,2H), 7,15(t,2H), 2,47(s,6H) |

Gemäß den oben beschriebenen Herstellverfahren lassen sich die Verbindungen der Formel (III-A) erhalten, beispielsweise die folgenden Verbindungen der Formel (III-A) (erfindungsgemäß sind Verbindungen mit A¹ = CHF₂, sofern nichts anderers angegeben ist):

| **Nummer** | **Verbindung** | **NMR Daten** |
|---|---|---|
| **III-A-1** | 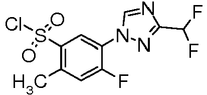 | 1H-NMR(D6-DMSO): 9,16(s,1H), 8,07-8,10(d,1H), 7,37-7,40(d,1H), 7,10-7,36(t,1H), 2,61(s,3H) |
| **III-A-2** | 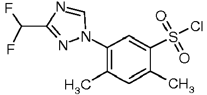 | 1H-NMR (CD₃CN): 8,61 (s, 1H), 8,11 (s, 1H), 7,61(s,1H), 6,92 (t, 1H), 2,79 (s, 3H), 2,32 (s,3H) |
| **III-A-3** | 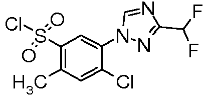 | 1H-NMR(D6-DMSO): 9,14(s,1H), 7,88(s,1H), 7,56(s,1H), 7,21(t,1H), 2,61(s,3H) |
| **III-A-4** | 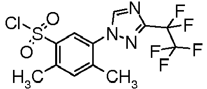 | 1H-NMR(D6-DMSO): 9,23(s,1H), 7,72(s,1H), 7,26(s,1H), 2,58(s,3H), 2,17(s,3H) |
| **III-A-5** | 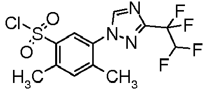 | 1H-NMR(D6-DMSO): : 9,16(s,1H), 7,71(s,1H), 7,26(s,1H), 6,83-7,11(m,1H), 2,58(s,3H), 2,13(s,3H) |
| **III-A-6** | 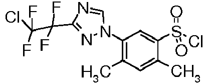 | 1H-NMR(D6-DMSO): 9,20-9,21(m,1H), 7,71(s,1H), 7,26(s,1H), 2,58(s,3H), 2,12(s,3H) |
| **III-A-7** | 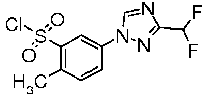 | 1H-NMR(D6-DMSO): 9,40(s,1H), 8,17-8,18(m,1H), 7,70-7,73(m,1H), 7,35(d,1H), 7,20(t,1H), 2,58(s,3H) |

Gemäß den oben beschriebenen Herstellverfahren lassen sich die Verbindungen der Formel (IV-A) erhalten, beispielsweise die folgenden Verbindungen der Formel (IV-A) (erfindungsgemäß sind Verbindungen mit A¹ = CHF₂, sofern nichts anderers angegeben ist):

| **Nummer** | **Verbindung** | **logP (HCOOH)** | **NMR Daten** |
|---|---|---|---|
| **IV-A-1** | 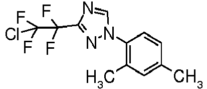 | 3,88 | 1H-NMR(D6-DMSO): 9,15-9,16(m,1H), 7,39-7,41(m,1H), 7,30(s,1H), 7,21-7,24(m,1H), 2,37(s,3H), 2,12(s,3H) |
| **IV-A-2** | 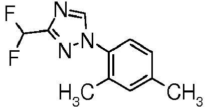 | 2,43 | 1H-NMR (CDCl₃); 8,27 (s, 1H), 7,12-7,26 (m, 3H), 6,67-6,93 (t, 1H), 2,4 (s, 3H), 2,19 (s, 3H) |
| **IV-A-3** | 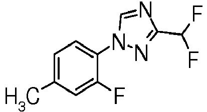 | 2,31 | 1H-NMR(D6-DMSO): 9,14(s,1H), 7,66-7,70(t,1H), 7,39-7,42(d,1H), 7,24-7,26(d,1H), 7,08-7,35(t,1H), 2,41(s,3H) |
| **IV-A-4** | 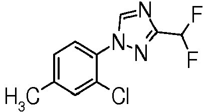 | 2,46 | 1H-NMR(D6-DMSO): 9,01(s,1H), 7,59-7,61(m,2H), 7,37-7,40(m,1H), 7,20(t,1H), 2,42 (s,3H) |
| **IV-A-5** | 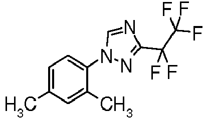 | 3,78 | 1H-NMR(D6-DMSO): 9,18(s,1H), 7,41(d,1H), 7,30(s,1H), 7,23(d,1H), 2,37(s,3H), 2,12(s,3H) |
| **IV-A-6** | 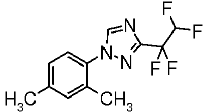 | 3,08 | 1H-NMR(D6-DMSO): 9,11(s,1H), 7,38(d,1H), 7,28-7,29(m,1H), 7,20-7,23(m,1H), 6,82-7,13(m,1H), 2,37(s,3H), 2,12(s,3H) |
| **IV-A-7** | 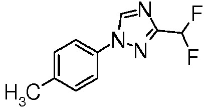 | 2,26 | 1H-NMR(D6-DMSO): 9,39(s,1H), 7,74-7,77(m,2H), 7,39-7,71(m,2H), 7,19(t,1H), 2,42 (s,3H) |

Gemäß den oben beschriebenen Herstellverfahren lassen sich die Verbindungen der Formel (V) erhalten, beispielsweise die folgenden Verbindungen der Formel (V) (erfindungsgemäß sind Verbindungen mit A¹ = CHF₂, sofern nichts anderers angegeben ist):

| **Nummer** | **Verbindung** | **logP (HCOOH)** | **NMR Daten** |
|---|---|---|---|
| **V-1** | 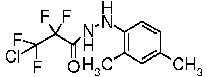 | 2,96 | 1H-NMR(D6-DMSO): 11,28(s,1H), 7,39(s,1H), 6,87(s,1H), 6,86(d,1H), 6,48(d,1H), 2,17(s,3H), 2,14(s,3H) |
| **V-2** | 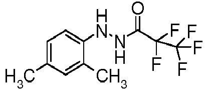 | 2,91 | 1H-NMR(D6-DMSO): 11,37(s,1H), 7,42(s,1H), 6,85-6,87(m,2H), 6,46(d,1H), 2,17(s,3H), 2,14(s,3H) |
| **V-3** | 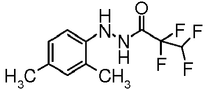 | 2,46 | 1H-NMR(D6-DMSO): 11,07(s,1H), 7,32(s,1H), 6,64-6,93(m,3H), 6,47(d,1H), 2,16(s,3H), 2,14(s,3H) |
| **V-4** | 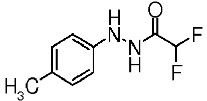 | 1,46 | 1H-NMR(D6-DMSO): 10,58-10,59(m,1H), 7,78-7,79(m,1H), 6,98(d,2H), 6,64(m,2H), 6,35(t,1H), 2,18(s,3H) |
| **V-5** | 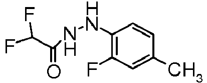 | 1,66 | 1H-NMR(D6-DMSO): 10,64(s,1H), 7,73(s,1H), 6,91-6,94(d,1H), 6,82-6,84(d,1H), 6,65-6,69(t,1H), 6,23-6,50(t,1H), 2,20(s,3H) |

Gemäß den oben beschriebenen Herstellverfahren lassen sich die Verbindungen der Formel (X) erhalten, beispielsweise die folgenden Verbindungen der Formel (X) (erfindungsgemäß sind Verbindungen mit A¹ = CHF₂, sofern nichts anderers angegeben ist):

| **Nummer** | **Verbindung** | **logP (HCOOH)** | **NMR Daten** |
|---|---|---|---|
| **X-1** | 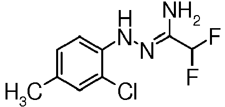 | 2,5 | |
| **X-2** | 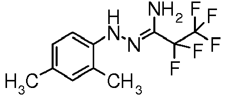 | 3,65 | 1H-NMR(D6-DMSO): 7,38(s,1H), 7,07(d,1H), 6,87(d,1H), 6,84(s,1H), 6,59(s,2H), 2,17(s,3H), 2,16(s,3H) |
| **X-3** | 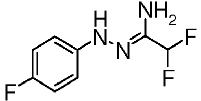 | 1,34 | |

### Analytische Methoden

Die Bestimmung der in der voranstehenden Tabelle und den Herstellungsbeispielen angegebenen logP Werten erfolgte gemäß EEC Directive 79/831 Annex V.A8 durch HPLC (High Performance Liquid Chromatography) an reversed-phase Säulen (C 18), mit nachfolgenden Methoden:
^{[a]} Die Bestimmung erfolgt im sauren Bereich bei pH 2.3 mit 0,1% wässriger Phosphorsäure und Acetonitril als Eluenten; linearer Gradient von 10% Acetonitril bis 95% Acetonitril.
^{[b]} Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril.

Die Kalibrierung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die lambda-maX Werte wurden an Hand der UV-Spektren von 200 nm bis 400 nm in den Maxima der chromatographischen Signale ermittelt.

Die MH⁺-Signale wurden mit einem Agilent MSD-System mit ESI und positiver oder negativer Ionisation bestimmt.

Die NMR-Spektren wurden mit einem Bruker Avance 400, ausgestattet mit einem Durchflussprobenkopf (60 µl Volumen), bestimmt. Als Lösungsmittel wurde d₆-DMSO oder CD₃CN verwendet, wobei als Referenz Tetramethylsilan (0.00 ppm) eingesetzt wurde. Die Meßtemperatur beträgt 303K, falls d₆-DMSO als Lösungsmittel verwendet wird, und 298K, falls CD₃CN als Lösungsmittel verwendet wird.

In Einzelfällen wurden die Proben mit einem Bruker Avance II 600 oder III 600 bestimmt.

Die Aufspaltung der Signale wurde wie folgt beschrieben: s (Singulett), d (Duplett), t (Triplett), q (Quartett), quin (Quintett), m (Multiplett).

### Anwendungsbeispiele

### Beispiel 1

### Boophilus microplus -Test (BOOPMI Injektion)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration. Die Wirkstofflösung wird in das Abdomen *(Boophilus microplus)* injiziert, die Tiere werden in Schalen überführt und in einem klimatisierten Raum aufbewahrt. Die Wirkungskontrolle erfolgt auf Ablage fertiler Eier.

Nach 7 Tagen wird die Wirkung in % bestimmt. Dabei bedeutet 100%, dass keine Zecke fertile Eier gelegt hat.

Bei diesem Test zeigen z.B. die folgenden Verbindungen der Herstellungsbeispiele eine Wirkung von 100% bei einer Aufwandmenge von 20µg/Tier: I-A-1, I-A-2, I-A-3, I-A-4,
1-A-5, 1-A-7, 1-A-8, 1-A-9, 1-A-10, I-A-11, I-A-12, I-A-17, I-A-27, I-A-36, I-A-39

### Beispiel 2

### Phaedon-Test (PHAECO Spritzbehandlung)

| | | |
|---|---|---|
| Lösungsmittel: | 78,0 | GewichtsteileAceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 100% bei einer Aufwandmenge von 500g/ha: I-A-5, I-A-6, I-A-9, I-A-10, I-A-12, I-A-15, I-A-16, I-A-23, I-A-27, I-A-30, I-A-31

### Beispiel 3

### Tetranychus-Test; OP-resistent (TETRUR Spritzbehandlung)

| | | |
|---|---|---|
| Lösungsmittel: | 78,0 | GewichtsteileAceton |
| | 1,5 | Gewichtsteile Dimethylformamid |
| Emulgator : | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 80% bei einer Aufwandmenge von 500g/ha: I-A-21, I-A-22, IV-A-2

Bei diesem Test zeigen z. B. die folgenden Verbindungen der Herstellungsbeispiele eine Wirkung von 90% bei einer Aufwandmenge von 500g/ha: IV-A-3,V-1

Bei diesem Test zeigen z. B. die folgenden Verbindungen der Herstellungsbeispiele eine Wirkung von 100% bei einer Aufwandmenge von 500g/ha: I-A-2, I-A-10, I-A-15, I-A-18,
I-A-20, I-A-23, I-A-24, I-A-25, I-A-26, I-A-29, I-A-33, I-A-38

Bei diesem Test zeigen z. B. die folgenden Verbindungen der Herstellungsbeispiele eine Wirkung von 100% bei einer Aufwandmenge von 100g/ha: I-A-1, I-A-3, I-A-4, I-A-5, I-A-6,
I-A-7, I-A-8, I-A-9, I-A-11, I-A-12, I-A-13, I-A-14, I-A-16, I-A-17, I-A-19, I-A-27, I-A-28,
I-A-30, I-A-31, I-A-32, I-A-34, I-A-35, I-A-36, I-A-37, I-A-39

### Beispiel 4

### Meloidogyne incognita-Test (MELGIN)

| | | |
|---|---|---|
| Lösungsmittel: | 78,0 | GewichtsteileAceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Gefäße werden mit Sand, Wirkstofflösung, Meloidogyne incognita-Ei-Larvensuspension und Salatsamen gefüllt. Die Salatsamen keimen und die Pflänzchen entwickeln sich. An den Wurzeln entwickeln sich die Gallen. Nach 14 Tagen wird die nematizide Wirkung an Hand der Gallenbildung in % bestimmt. Dabei bedeutet 100 %, dass keine Gallen gefunden wurden; 0 % bedeutet, dass die Zahl der Gallen an den behandelten Pflanzen der der unbehandelten Kontrolle entspricht.

Bei diesem Test zeigen z. B. die folgenden Verbindungen der Herstellungsbeispiele eine Wirkung von 90% bei einer Aufwandmenge von 20ppm: I-A-15, I-A-30

Bei diesem Test zeigen z. B. die folgenden Verbindungen der Herstellungsbeispiele eine Wirkung von 100% bei einer Aufwandmenge von 20ppm: I-A-2, I-A-4, I-A-9, I-A-16, I-A-27

### Beispiel 5

### Tetranychus-Test ; OP-resistent (TETRUR Spritzbehandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele überlegene Wirksamkeit gegenüber dem Stand der Technik:

**siehe Tabelle**

| Substanz | Struktur | Objekt | Konzentration | %Wirkung nach Applikation |
|---|---|---|---|---|
| VI-297 bekannt aus WO 1999/055668 | 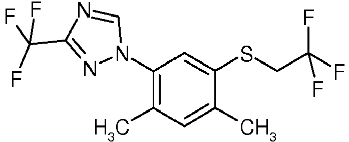 | TETRUR | 20 g / ha | 70 6d |
| I-A-2 erfindungsgemäß | 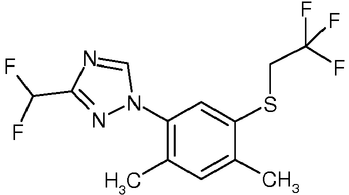 | TETRUR | 20 g / ha | 100 6d |

## Patentansprüche

1. 3-[1-(3-Haloalkyl)-triazolyl]-phenyl-Sulfid-Derivate der Formel (I) 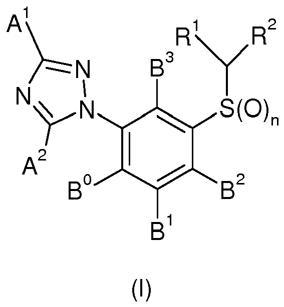 in welcher
A¹ für CHF₂ steht,
A² für Wasserstoff steht,
B⁰ für Wasserstoff, Amino, Halogen, Cyano, Nitro, Alkyl, Halogenalkyl, Alkylthio, Halogenalkylthio, Alkoxy oder Halogenalkoxy steht,
B¹, B², B³ unabhängig von einander für Wasserstoff, Halogen, Cyano, Nitro, Alkyl, Halogenalkyl, Cyanoalkyl, Hydroxyalkyl, Alkoxycarbonylalkyl, Alkoxyalkyl, Alkenyl, Halogenalkenyl Cyanoalkenyl, Alkinyl, Halogenalkinyl, Cyanoalkinyl, Alkoxy, Halogenalkoxy, Cyanoalkoxy, Alkoxycarbonylalkoxy, Alkoxyalkoxy, Alkylthio, Halogenalkylthio, Alkoxyalkylthio, Alkylsulfinyl, Halogenalkylsulfinyl, Alkoxyalkylsulfinyl, Alkylsulfonyl, Halogenalkylsulfonyl, Alkoxyalkylsulfonyl, Acyl, Halogenalkylcarbonyl, Carboxyl, Alkoxycarbonyl oder NR³R⁴, wobei R³ und R⁴ unabhängig voneinander für Wasserstoff, Alkyl, Halogenalkyl, Cyanoalkyl, Hydroxyalkyl, Alkoxyalkyl, Alkylthioalkyl, Alkenyl, Halogenalkenyl, Cyanoalkenyl, Alkinyl, Halogenalkinyl, Cyanoalkinyl, Acyl, Alkoxycarbonyl stehen oder R³ und R⁴ gemeinsam mit dem N-Atom, an dem sie gebunden sind, einen gegebenenfalls substituierten und gegebenenfalls durch Heteroatome unterbrochenen gesättigten oder ungesättigten fünf- bis achtgliedrigen Ring bilden können, stehen,
n für die Zahl 0, 1 oder 2 steht,
R¹ für Wasserstoff oder Alkyl steht,
R² für Wasserstoff, Halogen, Cyano, Nitro, Alkyl, Halogenalkyl, Cyanoalkyl, Alkoxyalkyl, Alkenyl, Halogenalkenyl, Alkinyl, Halogenalkinyl, Halogenalkoxyalkyl, gegebenenfalls durch ein oder mehrere Heteroatome unterbrochenes gegebenenfalls substituiertes Cycloalkyl oder Cycloalkenyl steht,
wobei die 3-[1-(3-Haloalkyl)-triazolyl]-phenyl-Sulfid-Derivate der Formel (I) nicht 3-(Difluormethyl)-1-{2,4-dimethyl-5-[(2,2,2-trifluorethyl)-sulfanyl]-phenyl}-1H-1,2,4-triazol, 3-(Difluormethyl)-1-{2,4-dimethyl-5-[(2,2,2-trifluorethyl)-sulfinyl]-phenyl}-1H-1,2,4-triazol, 1-{5-[(2,2-Difluorethyl)sulfanyl]-2,4-dimethylphenyl}-3-(difluor-methyl)-1H-1,2,4-triazol, und
1-{5-[(2,2-Difluorethyl)sulfinyl]-2,4-dimethylphenyl}-3-(difluormethyl)-1H-1,2,4-triazol sind.

2. Verbindungen der Formel (I) gemäß Anspruch 1, in welcher
A¹ für CHF₂ steht,
A² für Wasserstoff steht,
B⁰ für Wasserstoff, Amino, Halogen, Cyano, Nitro, (C₁-C₆)Alkyl, (C₁-C₆)Halogenalkyl, (C₁-C₆)Alkylthio, (C₁-C₆)Halogenalkylthio, (C₁-C₆)Alkoxy oder (C₁-C₆)Halogenalkoxy steht,
B¹, B², B³ unabhängig von einander für Wasserstoff, Halogen, Cyano, Nitro, (C₁-C₆)Alkyl, (C₁-C₆)Halogenalkyl, (C₁-C₆)Cyanoalkyl, (C₁-C₆)Hydroxyalkyl, (C₂-C₇)Alkoxycarbonyl-(C₁-C₆)Alkyl, (C₁-C₆)Alkoxy-(C₁-C₆)Alkyl, (C₂-C₆)Alkenyl, (C₂-C₆)Halogenalkenyl (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₂-C₅)Alkoxycarbonyl-(C₁-C₆)Alkoxy, (C₁-C₃)Alkoxy-(C₁-C₆)-Alkoxy, (C₁-C₆)Alkylthio, (C₁-C₆)Halogenalkylthio, (C₁-C₃)Alkoxy-(C₁-C₆)Alkylthio, (C₁-C₆)Alkylsulfinyl, (C₁-C₆)Halogenalkylsulfinyl, (C₁-C₃)Alkoxy-(C₁-C₆)Alkylsulfinyl, (C₁-C₆)Alkylsulfonyl, (C₁-C₆)Halogenalkylsulfonyl, (C₁-C₃)Alkoxy-(C₁-C₆)Alkylsulfonyl, (C₁-C₇)Acyl, (C₂-C₅)Halogenalkylcarbonyl, Carboxyl, (C₂-C₇)Alkoxycarbonyl oder NR³R⁴, wobei R³ und R⁴ unabhängig voneinander für Wasserstoff, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)Alkyl, (C₁-C₆)Alkylthio-(C₁-C₆)Alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₇)Acyl, (C₂-C₇)Alkoxycarbonyl stehen oder R³ und R⁴ gemeinsam mit dem N-Atom, an dem sie gebunden sind, einen gegebenenfalls durch (C₁-C₄)Alkyl, (C₁-C₄)Alkoxy, (C₁-C₄)Halogenalkyl substituierten und gegebenenfalls durch Heteroatome unterbrochenen gesättigten oder ungesättigten fünf- oder sechsgliedrigen Ring bilden können, stehen,
n für die Zahl 0, 1 oder 2 steht,
R¹ für Wasserstoff oder (C₁-C₄)Alkyl steht,
R² für Wasserstoff, Halogen, Cyano, Nitro, (C₁-C₆)Alkyl, (C₁-C₆)Halogenalkyl, (C₁-C₆)Cyanoalkyl, (C₂-C₆)Alkenyl, (C₂-C₆)Halogenalkenyl, (C₂-C₆)Alkinyl, (C₂-C₆)Halogenalkinyl, (C₁-C₆)Alkoxy-(C₁-C₆)Alkyl, (C₁-C₆)Halogenalkoxy-(C₁-C₆)Alkyl, gegebenenfalls durch ein oder mehrere Heteroatome unterbrochenes, gegebenenfalls substituiertes (C₃-C₈)Cycloalkyl oder (C₃-C₈)Cycloalkenyl steht.

3. Verfahren zur Herstellung von Verbindungen der Formel (I) gemäß einem der Ansprüche 1 bis 2, **dadurch gekennzeichnet, dass** man
(A) Aniline der Formel (VII) 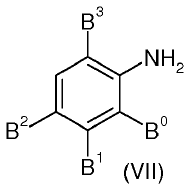 mit Natriumnitrit umsetzt zu Diazoniumsalzen und anschließend zu Hydrazinen der allgemeinen Formel (VI) 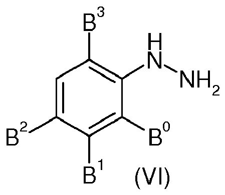 reduziert,
(B) die Hydrazine der Formel (VI) in Gegenwart von Estern der Formel (VIII) 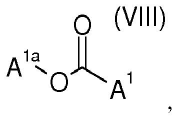 wobei A^{1a} für Alkyl steht,
in Hydrazide der allgemeinen Formel (V) 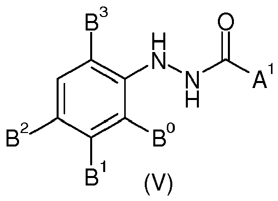 überführt,
(C) Hydrazide der Formel (V) mit Formamidin-Hydrochlorid in Gegenwart einer Base zu Triazolen der Formel (IV-A) 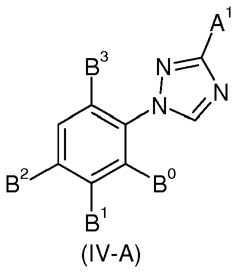 umsetzt,
(D) die Triazole der allgemeinen Formel (IV-A) mittels Sulfochlorierung zu Sulfonylchloriden der allgemeinen Formel (III-A) 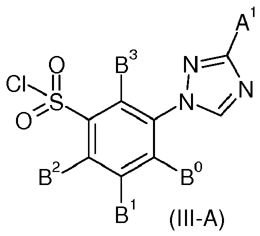 umsetzt,
(E) die Sulfonylchloride der Formel (III-A) durch Reduktion in Disulfide der allgemeinen Formel (II-A) 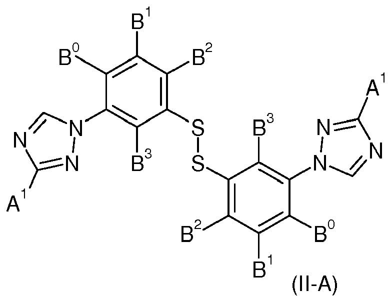 überführt,
(F) die Disulfide der allgemeinen Formel (II-A) mit Elektrophilen der allgemeinen Formel (IX) 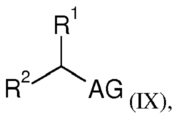 wobei AG für eine Abgangsgruppe wie Chlor, Brom, Tosylat, Mesylat oder Triflat steht,
zu Sulfiden der allgemeinen Formel (I-Aa) 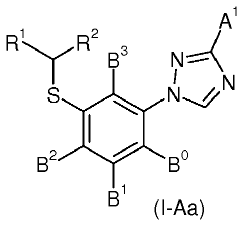 umsetzt,
(G) die Verbindungen der Formel (I-Aa) mit Oxidationsmitteln zu Sulfoxiden der allgemeinen Formeln (I-Ab) 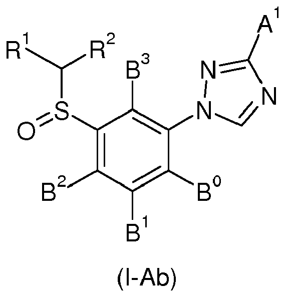 und Sulfone der allgemeinen Formeln (I-Ac) 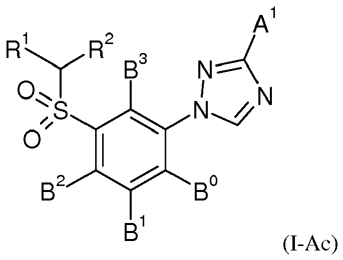 umsetzt,
wobei A¹, B⁰, B¹, B², B³, R¹, R² die Bedeutungen gemäß einem der Ansprüche 1 bis 2 haben.

4. Verfahren gemäß Anspruch 3, **dadurch gekennzeichnet, dass**
die Verbindungen der allgemeinen Formel (IV-A),
wobei A¹, B⁰, B¹, B² und B³ die Bedeutungen gemäß einem der Ansprüche 1 bis 2 haben, hergestellt werden,
indem man
Boronsäuren der allgemeinen Formel (VIII) 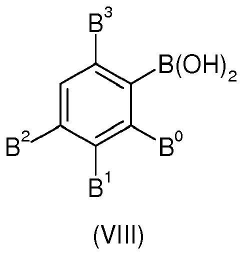 in einer Kupfer-katalysierten Kupplungsreaktion mit Triazolen der allgemeinen Formel (IX-A) 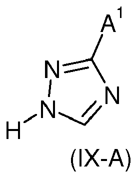 zu den Verbindungen der Formel (IV-A) umsetzt.

5. Verfahren gemäß Anspruch 3, **dadurch gekennzeichnet, dass**
die Verbindungen der allgemeinen Formel (IV-A),
wobei A¹, B⁰, B¹, B² und B³ die Bedeutungen gemäß einem der Ansprüche 1 bis 2 haben, hergestellt werden,
indem man Hydrazine der allgemeinen Formel (VI) 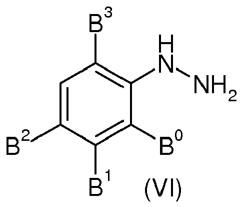 in Gegenwart von Amidinen der Formel (XI) oder deren Salze 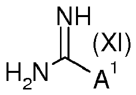 zu Amidrazonen der Formel (X) 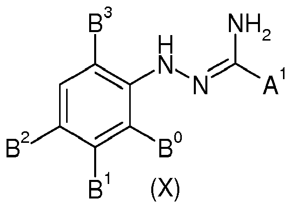 umsetzt, und diese mit einem Orthoformat zu Triazolen der Formel (IV-A) umsetzt.

6. Wirkstoffzusammensetzung enthaltend mindestens ein 3-[1-(3-Haloalkyl)-triazolyl]-phenyl-Sulfid-Derivat der Formel (I) gemäß einem der Ansprüche 1 bis 2 und mindestens einen weiteren insektiziden, akariziden oder nematiziden Wirkstoff ausgewählt aus der Gruppe bestehend aus
(1) Acetylcholinesterase (AChE) Inhibitoren, wie beispielsweise
Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; oder
Organophosphate, z.B. Acephate, Azamethiphos, Azinphos (-methyl, -ethyl), Cadusafos, Chlorethoxyfos, Chlorfenvinphos, Chlormephos, Chlorpyrifos (-methyl), Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Isofenphos, Isopropyl *O*-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion (-methyl), Phenthoate, Phorate, Phosalone, Phosmet, Phosphamidon, Phoxim, Pirimiphos (-methyl), Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Antagonisten, wie beispielsweise
Organochlorine, z.B. Chlordane und Endosulfan (alpha-); oder
Fiprole (Phenylpyrazole), z.B. Ethiprole, Fipronil, Pyrafluprole und Pyriprole.
(3) Natrium-Kanal-Modulatoren / Spannungsabhängige Natrium-Kanal-Blocker, wie beispielsweise
Pyrethroide, z.B. Acrinathrin, Allethrin (d-cis-trans, d-trans), Bifenthrin, Bioallethrin, Bioallethrin-S-cyclopentenyl, Bioresmethrin, Cycloprothrin, Cyfluthrin (beta-), Cyhalothrin (gamma-, lambda-), Cypermethrin (alpha-, beta-, theta-, zeta-), Cyphenothrin [(1*R*)-*trans-*Isomere], Deltamethrin, Dimefluthrin, Empenthrin [(*EZ*)-(1*R*)-Isomere], Esfenvalerate, Etofenprox, Fenpropathrin, Fenvalerate, Flucythrinate, Flumethrin, Fluvalinate (tau-), Halfenprox, Imiprothrin, Metofluthrin, Permethrin, Phenothrin [(1*R*)-trans-Isomer], Prallethrin, Profluthrin, Pyrethrine (pyrethrum), Resmethrin, RU 15525, Silafluofen, Tefluthrin, Tetramethrin [(1*R*)-Isomere], Tralomethrin, Transfluthrin und ZXI 8901; oder
DDT; oder Methoxychlor.
(4) Nikotinerge Acetylcholin-Rezeptor-Agonisten, wie beispielsweise
Neonikotinoide, z.B. Acetamiprid, Clothianidin, Dinotefuran, Imidacloprid, Nitenpyram, Thiacloprid, Thiamethoxam; oder
Nikotin.
(5) Allosterische Acetylcholin-Rezeptor-Modulatoren (Agonisten), wie beispielsweise Spinosyne, z.B. Spinetoram und Spinosad.
(6) Chlorid-Kanal-Aktivatoren, wie beispielsweise
Avermectine/Milbemycine, z.B. Abamectin, Emamectin-benzoate, Lepimectin und Milbemectin.
(7) Juvenilhormon-Analoge, z.B. Hydroprene, Kinoprene, Methoprene; oder Fenoxycarb; Pyriproxyfen.
(8) Wirkstoffe mit unbekannten oder nicht spezifischen Wirkmechanismen, wie beispielsweise
Begasungsmittel, z.B. Methylbromid und andere Alkylhalogenide; oder
Chloropicrin; Sulfurylfluorid; Borax; Brechweinstein.
(9) Selektive Fraßhemmer, z.B. Pymetrozine; oder Flonicamid.
(10) Milbenwachstumsinhibitoren, z.B. Clofentezine, Diflovidazin, Hexythiazox, Etoxazole.
(11) Mikrobielle Disruptoren der Insektendarmmembran, wie beispielsweise *Bacillus thuringiensis* Subspezies *israelerisis, Bacillus sphaericus*, *Bacillus thuringiensis* Subspezies *aizawai, Bacillus thuringiensis* Subspezies *kurstaki, Bacillus thuringiensis* Subspezies *tenebrionis,* und BT-Pflanzen-Proteine, z.B. Cry1Ab, Cry1Ac, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron; oder
Organozinnverbindungen, z.B. Azocyclotin, Cyhexatin, Fenbutatin oxide; oder
Propargite; Tetradifon.
(13) Entkoppler der oxidativen Phoshorylierung durch Unterbrechung des H-Protongradienten, wie beispielsweise Chlorfenapyr und DNOC.
(14) Nikotinerge Acetylcholin-Rezeptor-Antagonisten, wie beispielsweise Bensultap, Cartap (-Hydrochlorid), Thiocylam, und Thiosultap (-sodium).
(15) Inhibitoren der Chitinbiosynthese, Typ 0, wie beispielsweise Benzoylharnstoffe, z.B. Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungsstörende Wirkstoffe, wie beispielsweise Cyromazine.
(18) Ecdysonagonisten/-disruptoren, wie beispielsweise
Diacylhydrazine, z.B. Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon; Acequinocyl; Fluacrypyrim.
(21) Komplex-I-Elektronentransportinhibitoren, beispielsweise aus der Gruppe der METI-Akarizide, z.B. Fenazaquin, Fenpyroximate, Pyrimidifen, Pyridaben, Tebufenpyrad, Tolfenpyrad; oder
Rotenone (Derris).
(22) Spannungsabhängige Natriumkanal-Blocker, z.B. Indoxacarb; Metaflumizone.
(23) Inhibitoren der Acetyl-CoA-Carboxylase, wie beispielsweise Tetronsäure-Derivate, z.B. Spirodiclofen und Spiromesifen; oder Tetramsäure-Derivate, z.B. Spirotetramat.
(24) Komplex-IV-Elektronentransportinhibitoren, wie beispielsweise Phosphine, z.B. Aluminiumphosphid, Kalziumphosphid, Phosphin, Zinkphosphid; oder Cyanid.
(25) Komplex-II-Elektronentransportinhibitoren, wie beispielsweise Cyenopyrafen.
(28) Ryanodinrezeptor-Effektoren, wie beispielsweise Diamide, z.B. Flubendiamide, Chlorantraniliprole (Rynaxypyr), Cyantraniliprole (Cyazypyr) sowie 3-Brom-N-{2-brom-4-chlor-6-[(1-cyclopropylethyl)carbamoyl]phenyl}-1-(3-chlorpyridin-2-yl)-1H-pyrazol-5-carboxamid oder Methyl-2-[3,5-dibrom-2-({[3-brom-1-(3-chlorpyridin-2-yl)-1H-pyrazol-5-yl]carbonyl}amino)benzoyl]-1,2-dimethylhydrazincarboxylat.
Weitere Wirkstoffe mit unbekanntem Wirkmechanismus, wie beispielsweise Azadirachtin, Amidoflumet, Benzoximate, Bifenazate, Chinomethionat, Cryolite, Cyflumetofen, Dicofol, Fluensulfone (5-chloro-2-[(3,4,4-trifluorobut-3-en-1-yl)sulfonyl]-1,3-thiazole), Flufenerim, Pyridalyl und Pyrifluquinazon; desweiteren Präparate auf Basis von Bacillus firmus (I-1582, BioNeem, Votivo) sowie folgende bekannte wirksame Verbindungen
4-{[(6-Brompyrid-3-yl)methyl](2-fluorethyl)amino}furan-2(5H)-on, 4-{[(6-Fluorpyrid-3-yl)methyl](2,2-difluorethyl)amino}furan-2(5H)-on, 4-{[(2-Chlor-1,3-thiazol-5-yl)methyl](2-fluorethyl)amino}furan-2(5H)-on, 4-{[(6-Chlorpyrid-3-yl)methyl](2-fluorethyl)amino}furan-2(5H)-on, 4-{[(6-Chlorpyrid-3-yl)methyl](2,2-difluorethyl)amino}furan-2(5H)-on, 4-{[(6-Chlor-5-fluorpyrid-3-yl)methyl](methyl)amino}furan-2(5H)-on, 4-{[(5,6-Dichlorpyrid-3-yl)methyl](2-fluorethyl)amino}furan-2(5H)-on, 4-{[(6-Chlor-5-fluorpyrid-3-yl)methyl](cyclopropyl)amino}furan-2(5H)-on, 4-{[(6-Chlorpyrid-3-yl)methyl](cyclopropyl)amino}furan-2(5H)-on, 4-{[(6-Chlorpyrid-3-yl)methyl](methyl)amino}furan-2(5H)-on, [(6-Chlorpyridin-3-yl)methyl](methyl)oxido-λ⁴-sulfanylidencyanamid, [1-(6-Chlorpyridin-3-yl)ethyl](methyl)oxido-λ⁴-sulfanylidencyanamid und seine Diastereomere (A) und (B) 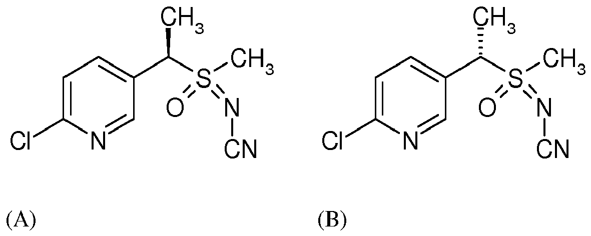 [(6-Trifluormethylpyridin-3-yl)methyl](methyl)oxido-λ⁴-sulfanylidencyanamid, Sulfoxaflor
11-(4-Chlor-2,6-dimethylphenyl)-12-hydroxy-1,4-dioxa-9-azadispiro[4.2.4.2]tetradec-11-en-10-on, 3-(4'-Fluor-2,4-dimethylbiphenyl-3-yl)-4-hydroxy-8-oxa-1-azaspiro[4.5]dec-3-en-2-on,
1-[2-fluoro-4-methyl-5-[(2,2,2-trifluoroethyl)sulfinyl]phenyl]-3-(trifluoromethyl)-1H-1,2,4-Triazol-5-amine,
[(3S,4aR,12R,12aS,12bS)-3-[(Cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methylcyclopropancarboxylat,
2-Cyan-3-(difluormethoxy)-N,N-dimethylbenzolsulfonamid,
2-Cyan-3-(difluormethoxy)-N-methylbenzolsulfonamid, 2-Cyan-3-(difluormethoxy)-N-ethylbenzolsulfonamid, 4-(Difluormethoxy)-N-ethyl-N-methyl-1,2-benzothiazol-3-amin-1,1-dioxid und
N-[1-(2,3-Dimethylphenyl)-2-(3,5-dimethylphenyl)ethyl]-4,5-dihydro-1,3-thiazol-2-amin,
und/oder mindestens einen weiteren fungiziden Wirkstoff ausgewählt aus der Gruppe bestehend aus
(1) Inhibitoren der Ergosterol-Biosynthese, wie beispielsweise Aldimorph, Azaconazol, Bitertanol, Bromuconazol, Cyproconazol, Diclobutrazol, Difenoconazol, Diniconazol, Diniconazol-M, Dodemorph, Dodemorph Acetat, Epoxiconazol, Etaconazol, Fenarimol, Fenbuconazol, Fenhexamid, Fenpropidin, Fenpropimorph, Fluquinconazol, Flurprimidol, Flusilazol, Flutriafol, Furconazol, Furconazol-Cis, Hexaconazol, Imazalil, Imazalil Sulfat, Imibenconazol, Ipconazol, Metconazol, Myclobutanil, Naftifin, Nuarimol, Oxpoconazol, Paclobutrazol, Pefurazoat, Penconazol, Piperalin, Prochloraz, Propiconazol, Prothioconazol, Pyributicarb, Pyrifenox, Quinconazol, Simeconazol, Spiroxamin, Tebuconazol, Terbinafin, Tetraconazol, Triadimefon, Triadimenol, Tridemorph, Triflumizol, Triforin, Triticonazol, Uniconazol, Uniconazol-p, Viniconazol, Voriconazol, 1-(4-Chlorphenyl)-2-(1H-1,2,4-triazol-1-yl)cycloheptanol, Methyl-1-(2,2-dimethyl-2,3-dihydro-1H-inden-1-yl)-1H-imidazol-5-carboxylat, N'-{5-(Difluormethyl)-2-methyl-4-[3-(trimethylsilyl)propoxy]phenyl}-N-ethyl-N-methylimidoformamid, N-Ethyl-N-methyl-N'-{2-methyl-5-(trifluormethyl)-4-[3-(trimethylsilyl)propoxy]phenyl} imidoformamid und O-[1-(4-Methoxyphenoxy)-3,3-dimethylbutan-2-yl]-1H-imidazol-1-carbothioat.
(2) Inhibitoren der Respiration (Atmungsketten-Inhibitoren), wie beispielsweise Bixafen, Boscalid, Carboxin, Diflumetorim, Fenfuram, Fluopyram, Flutolanil, Fluxapyroxad, Furametpyr, Furmecyclox, Isopyrazam Mischung des syn-epimeren Razemates 1RS,4SR,9RS und des anti-empimeren Razemates 1RS,4SR,9SR, Isopyrazam (anti-epimeres Razemat ), Isopyrazam (anti-epimeres Enantiomer 1R,4S,9S), Isopyrazam (anti-epimeres Enantiomer 1S,4R,9R), Isopyrazam (syn-epimeres Razemat 1RS,4SR,9RS), Isopyrazam (syn-epimeres Enantiomer 1R,4S,9R), Isopyrazam (syn-epimeres Enantiomer 1S,4R,9S), Mepronil, Oxycarboxin, Penflufen, Penthiopyrad, Sedaxane, Thifluzamid, 1-Methyl-N-[2-(1,1,2,2-tetrafluorethoxy)phenyl]-3-(trifluormethyl)-1H-pyrazol-4-carboxamid, 3-(Difluormethyl)-1-methyl-N-[2-(1,1,2,2-tetrafluorethoxy)phenyl]-1H-pyrazol-4-carboxamid, 3-(Difluormethyl)-N-[4-fluor-2-(1,1,2,3,3,3-hexafluorpropoxy)phenyl]-1-methyl-1H-pyrazol-4-carboxamid und N-[1-(2,4-Dichlorphenyl)-1-methoxypropan-2-yl]-3-(difluormethyl)-1-methyl-1H-pyrazol-4-carboxamid.
(3) Inhibitoren der Respiration (Atmungsketten-Inhibitoren) am Komplex III der Atumungskette, wie beispielsweise Ametoctradin, Amisulbrom, Azoxystrobin, Cyazofamid, Dimoxystrobin, Enestroburin, Famoxadon, Fenamidon, Fluoxastrobin, Kresoxim-Methyl, Metominostrobin, Orysastrobin, Picoxystrobin, Pyraclostrobin, Pyrametostrobin, Pyraoxystrobin, Pyribencarb, Trifloxystrobin, (2E)-2-(2-{[6-(3-Chlor-2-methylphenoxy)-5-fluorpyrimidin-4-yl]oxy}phenyl)-2-(methoxyimino)-N-methylethanamid, (2E)-2-(Methoxyimino)-N-methyl-2-(2-{[({(1E)-1-[3-(trifluormethyl)phenyl]ethyliden}amino)oxy]methyl}phenyl)ethanamid, (2E)-2-(Methoxyimino)-N-methyl-2-{2-[(E)-({1-[3-(trifluormethyl)phenyl]ethoxy}imino)methyl]phenyl}ethanamid, (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethanamid, (2E)-2-{2-[({[(2E,3E)-4-(2,6-Dichlorphenyl)but-3-en-2-yliden]amino}oxy)methyl]phenyl}-2-(methoxyimino)-N-methylethanamid, 2-Chlor-N-(1,1,3-trimethyl-2,3-dihydro-1H-inden-4-yl)pyridin-3-carboxamid, 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, Methyl-(2E)-2-{2-[({cyclopropyl[(4-methoxyphenyl)imino]methyl}sulfanyl)methyl]phenyl}-3-methoxyprop-2-enoat, N-(3-Ethyl-3,5,5-trimethylcyclohexyl)-3-(formylamino)-2-hydroxybenzamid, 2-{2-[(2,5-Dimethylphenoxy)methyl]phenyl}-2-methoxy-N-methylacetamid und (2R)-2-{2-[(2,5-Dimethylphenoxy)methyl]phenyl}-2-methoxy-N-methylacetamid.
(4) Inhibitoren der Mitose und Zellteilung, wie beispielsweise Benomyl, Carbendazim, Chlorfenazol, Diethofencarb, Ethaboxam, Fluopicolid, Fuberidazol, Pencycuron, Thiabendazol, Thiophanat-Methyl, Thiophanat, Zoxamid, 5-Chlor-7-(4-methylpiperidin-1-yl)-6-(2,4,6-trifluorphenyl)[1,2,4]triazolo[1,5-a]pyrimidin und 3-Chlor-5-(6-chlorpyridin-3-yl)-6-methyl-4-(2,4,6-trifluorphenyl)pyridazin.
(5) Verbindungen mit Multisite-Aktivität, wie beispielsweise Bordeauxmischung, Captafol, Captan, Chlorothalonil, Kupferzubereitungen wie Kupferhydroxid, Kupfernaphthenat, Kupferoxid, Kupferoxychlorid, Kupfersulfat, Dichlofluanid, Dithianon, Dodine, Dodine freie Base, Ferbam, Fluorofolpet, Folpet, Guazatin, Guazatinacetat, Iminoctadin, Iminoctadinalbesilat, Iminoctadintriacetat, Mankupfer, Mancozeb, Maneb, Metiram, Zinkmetiram, Kupfer-Oxin, Propamidin, Propineb, Schwefel und Schwefelzubereitungen wie beispielsweise Calciumpolysulfid, Thiram, Tolylfluanid, Zineb und Ziram.
(6) Resistenzinduktoren, wie beispielsweise Acibenzolar-S-Methyl, Isotianil, Probenazol und Tiadinil.
(7) Inhibitoren der Aminosäure- und Protein-Biosynthese, wie beispielsweise Andoprim, Blasticidin-S, Cyprodinil, Kasugamycin, Kasugamycin Hydrochlorid Hydrat, Mepanipyrim und Pyrimethanil.
(8) Inhibitoren der ATP Produktion, wie beispielsweise Fentin Acetat, Fentin Chlorid, Fentin Hydroxid und Silthiofam.
(9) Inhibitoren der Zellwandsynthese, wie beispielsweise Benthiavalicarb, Dimethomorph, Flumorph, Iprovalicarb, Mandipropamid, Polyoxins, Polyoxorim, Validamycin A und Valifenalat.
(10) Inhibitoren der Lipid- und Membran-Synthese, wie beispielsweise Biphenyl, Chloroneb, Dicloran, Edifenphos, Etridiazol, Iodocarb, Iprobenfos, Isoprothiolan, Propamocarb, Propamocarb Hydrochlorid, Prothiocarb, Pyrazophos, Quintozen, Tecnazene und Tolclofos-Methyl.
(11) Inhibitoren der Melanin-Biosynthese, wie beispielsweise Carpropamid, Diclocymet, Fenoxanil, Fthalid, Pyroquilon und Tricyclazol.
(12) Inhibitoren der Nukleinsäuresynthese, wie beispielsweise Benalaxyl, Benalaxyl-M (Kiralaxyl), Bupirimat, Clozylacon, Dimethirimol, Ethirimol, Furalaxyl, Hymexazol, Metalaxyl, Metalaxyl-M (Mefenoxam), Ofurace, Oxadixyl, Oxolinsäure.
(13) Inhibitoren der Signaltransduktion, wie beispielsweise Chlozolinat, Fenpiclonil, Fludioxonil, Iprodion, Procymidon, Quinoxyfen und Vinclozolin.
(14) Entkoppler, wie beispielsweise Binapacryl, Dinocap, Ferimzon, Fluazinam und Meptyldinocap.
(15) Weitere Verbindungen, wie beispielsweise Benthiazol, Bethoxazin, Capsimycin, Carvon, Chinomethionat, Chlazafenon, Cufraneb, Cyflufenamid, Cymoxanil, Cyprosulfamide, Dazomet, Debacarb, Dichlorophen, Diclomezin, Difenzoquat, Difenzoquat Methylsulphat, Diphenylamin, Ecomat, Fenpyrazanlln, Flumetover, Fluoromid, Flusulfamid, Flutianil, Fosetyl-Aluminium, Fosetyl-Calcium, Fosetyl-Natrium, Hexachlorbenzol, Irumamycin, Methasulfocarb, Methylisothiocyanat, Metrafenon, Mildiomycin, Natamycin, Nickel Dimethyldithiocarbamat, Nitrothal-Isopropyl, Octhilinone, Oxamocarb, Oxyfenthiin, Pentachlorphenol und dessen Salze, Phenothrin, Phosphorsäure und deren Salze, Propamocarb-Fosetylat, Propanosin-Natrium, Proquinazid, Pyrrolnitrin, Tebufloquin, Tecloftalam, Tolnifanid, Triazoxid, Trichlamid, Zarilamid, 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-Methoxyphenoxy)-3,3-dimethylbutan-2-yl-1H-imidazol-1-carboxylat, 2,3,5,6-Tetrachlor-4-(methylsulfonyl)pyridin, 2,3-Dibutyl-6-chlorthieno[2,3-d]pyrimidin-4(3H)-on, 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, 2-Butoxy-6-iod-3-propyl-4H-chromen-4-on, 2-Chlor-5-[2-chlor-1-(2,6-difluor-4-methoxyphenyl)-4-methyl-1H-imidazol-5-yl]pyridin, 2-Phenylphenol und dessen Salze, 3,4,5-Trichlorpyridin-2,6-dicarbonitril, 3-[5-(4-Chlorphenyl)-2,3-dimethyl-1,2-oxazolidin-3-yl]pyridin, 3-Chlor-5-(4-chlorphenyl)-4-(2,6-difluorphenyl)-6-methylpyridazin, 4-(4-Chlorphenyl)-5-(2,6-difluorphenyl)-3,6-dimethylpyridazin, 5-Amino-1,3,4-thiadiazol-2-thiol, 5-Chlor-N'-phenyl-N'-(prop-2-in-1-yl)thiophen-2-sulfonohydrazid, 5-Methyl-6-octyl[1,2,4]triazolo[1,5-a]pyrimidin-7-amin, Ethyl-(2Z)-3-amino-2-cyan-3-phenylprop-2-enoat, N-(4-Chlorbenzyl)-3-[3-methoxy-4-(prop-2-in-1-yloxy)phenyl]propanamid, N-[(4-Chlorphenyl)(cyan)methyl]-3-[3-methoxy-4-(prop-2-in-1-yloxy)phenyl]propanamid, N-[(5-Brom-3-chlorpyridin-2-yl)methyl]-2,4-dichlorpyridin-3-carboxamid, N-[1-(5-Brom-3-chlorpyridin-2-yl)ethyl]-2,4-dichlorpyridm-3-carboxamid, N-[1-(5-Brom-3-chlorpyridin-2-yl)ethyl]-2-fluor-4-iodpyridin-3-carboxamid, N-{(E)-[(Cyclopropylmethoxy)imino][6-(difluormethoxy)-2,3-difluorphenyl]methyl}-2-phenylacetamid, N-{(Z)-[(Cyclopropylmethoxy)imino][6-(difluormethoxy)-2,3-difluorphenyl]methyl-2-phenylacetamid, N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazol-4-carboxamid, N-Methyl-2-(1-{[5-methyl-3-(trifluormethyl)-1H-pyrazol-1-yl]acetyl}piperidin-4-yl)-N-[(1R)-1,2,3,4-tetrahydronaphthalen-1-yl]-1,3-thiazol-4-carboxamid, N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazol-4-carboxamid, Pentyl-{6-[({[(1-methyl-1H-tetrazol-5-yl)(phenyl)methyliden]amino}oxy)methyl]pyridin-2-yl}carbamat, Phenazin-1-carbonsäure, Chinolin-8-ol und Chinolin-8-olsulfat(2:1).
(16) Weitere Verbindungen, wie beispielsweise 1-Methyl-3-(trifluormethyl)-N-[2'-(trifluormethyl)biphenyl-2-yl]-1H-pyrazol-4-carboxamid, N-(4'-Chlorbiphenyl-2-yl)-3-(difluormethyl)-1-methyl-1H-pyrazol-4-carboxamid, N-(2',4'-Dichlorbiphenyl-2-yl)-3-(difluormethyl)-1-methyl-1H-pyrazol-4-carboxamid, 3-(Difluormethyl)-1-methyl-N-[4'-(trifluormethyl)biphenyl-2-yl]-1H-pyrazol-4-carboxamid, N-(2',5'-Difluorbiphenyl-2-yl)-1-methyl-3-(trifluormethyl)-1H-pyrazol-4-carboxamid, 3-(Difluormethyl)-1-methyl-N-[4'-(prop-1-in-1-yl)biphenyl-2-yl]-1H-pyrazol-4-carboxamid, 5-Fluor-1,3-dimethyl-N-[4'-(prop-1-in-1-yl)biphenyl-2-yl]-1H-pyrazol-4-carboxamid, 2-Chlor-N-[4'-(prop-1-in-1-yl)biphenyl-2-yl]pyridin-3-carboxamid, 3-(Difluormethyl)-N-[4'-(3,3-dimethylbut-1-in-1-yl)biphenyl-2-yl]-1-methyl-1H-pyrazol-4-carboxamid, N-[4'-(3,3-Dimethylbut-1-in-1-yl)biphenyl-2-yl]-5-fluor-1,3-dimethyl-1H-pyrazol-4-carboxamid, 3-(Difluormethyl)-N-(4'-ethinylbiphenyl-2-yl)-1-methyl-1H-pyrazol-4-carboxamid, N-(4'-Ethinylbiphenyl-2-yl)-5-fluor-1,3-dimethyl-1H-pyrazol-4-carboxamid, 2-Chlor-N-(4'-ethinylbiphenyl-2-yl)pyridin-3-carboxamid, 2-Chlor-N-[4'-(3,3-dimethylbut-1-in-1-yl)biphenyl-2-yl]pyridin-3-carboxamid, 4-(Difluormethyl)-2-methyl-N-[4'-(trifluormethyl)biphenyl-2-yl]-1,3-thiazol-5-carboxamid, 5-Fluor-N-[4'-(3-hydroxy-3-methylbut-1-in-1-yl)biphenyl-2-yl]-1,3-dimethyl-1H-pyrazol-4-carboxamid, 2-Chlor-N-[4'-(3-hydroxy-3-methylbut-1-in-1-yl)biphenyl-2-yl]pyridin-3-carboxamid, 3-(Difluormethyl)-N-[4'-(3-methoxy-3-methylbut-1-in-1-yl)biphenyl-2-yl]-1-methyl-1H-pyrazol-4-carboxamid, 5-Fluor-N-[4'-(3-methoxy-3-methylbut-1-in-1-yl)biphenyl-2-yl]-1,3-dimethyl-1H-pyrazol-4-carboxamid, 2-Chlor-N-[4'-(3-methoxy-3-methylbut-1-in-1-yl)biphenyl-2-yl]pyridin-3-carboxamid, (5-Brom-2-methoxy-4-methylpyridin-3-yl)(2,3,4-trimethoxy-6-methylphenyl)methanon und N-[2-(4-{[3-(4-Chlorphenyl)prop-2-in-1-yl]oxy}-3-methoxyphenyl)ethyl]-N2-(methylsulfonyl)valinamid.

7. Zusammensetzung enthaltend mindestens eine Verbindung der Formel (I) gemäß einem der Ansprüche 1 bis 2 oder eine Wirkstoffzusammensetzung gemäß Anspruch 6, sowie mindestens einen Penetrationsförderer.

8. Verbindungen der allgemeinen Formel (II-A) gemäß Anspruch 3 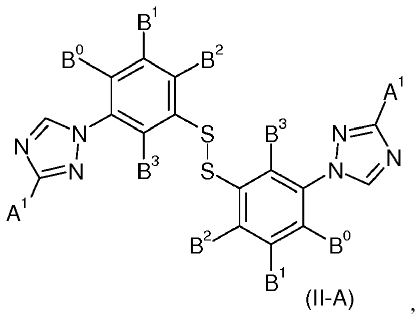 in welcher A¹, B⁰, B² die Bedeutungen gemäß einem der Ansprüche 1 bis 2 haben, und und B¹ und B³ für Wasserstoff stehen.

9. Verbindungen der allgemeinen Formel (III-A) gemäß Anspruch 3, 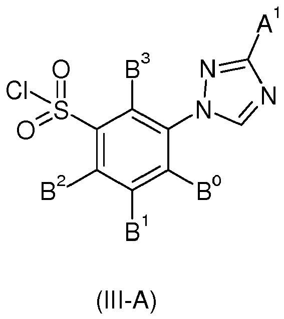 wobei A¹, B⁰ und B²die Bedeutung gemäß einem der Ansprüche 1 bis 2 haben und B¹ und B³ für Wasserstoff stehen.

10. Verbindungen der allgemeinen Formel (IV-A) gemäß Anspruch 3, 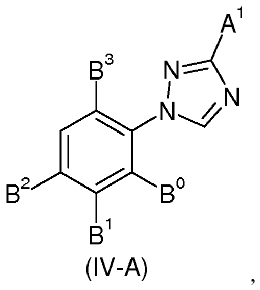 wobei
B¹ und B³ für Wasserstoff stehen, und
B⁰ steht für Wasserstoff, Methyl, Ethyl, Fluor, Chlor, Methoxy, Cyano, CHF₂, CF₃ oder OCF₃,
B² steht für Wasserstoff, Methyl, Ethyl, Fluor, Chlor, Methoxy, Cyano, CHF₂, CF₃ oder OCF₃.

11. Verbindung der allgemeinen Formel (V) gemäß Anspruch 3 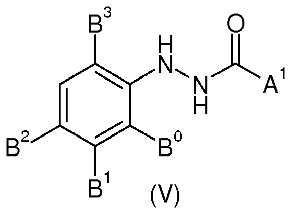 wobei
B¹ und B³ für Wasserstoff stehen,
A¹ für CHF₂ steht,
B⁰ für Methyl, Ethyl, Fluor, Chlor, Methoxy, Cyano, CHF₂, CF₃ oder OCF₃ steht,
B² für Wasserstoff, Ethyl, Fluor, Chlor, Cyano, CHF₂, CF₃ oder OCF₃ steht.

12. Agrochemische Zusammensetzungen, **dadurch gekennzeichnet, dass** sie mindestens eine Verbindung der Formel (I) gemäß den Ansprüchen 1 bis 2 oder eine Zusamennsetzung gemäß einem der Ansprüche 6 bis 7, sowie Streckmittel und/oder oberflächenaktive Stoffe enthalten.

13. Verwendung von Verbindungen der Formel (I) gemäß einem der Ansprüche 1 bis 2 oder einer Zusammensetzung gemäß einem der Ansprüche 6,7 oder 12 zur Bekämpfung tierischer Schädlinge im Pflanzenschutz oder im Materialschutz.

14. Verbindung der Formel (I) gemäß einem der Ansprüche 1 bis 2 oder einer Zusammensetzung gemäß einem der Ansprüche 6,7 oder 12 zur Anwendung in einem Verfahren im veterinärmedizinischen Sektor.

## Claims

1. 3-[1-(3-Haloalkyl)triazolyl]phenyl sulphide derivatives of the formula (I) 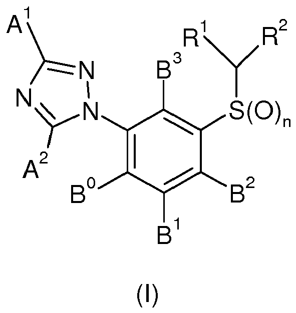 in which
A¹ is CHF₂,
A² is hydrogen,
B⁰ is hydrogen, amino, halogen, cyano, nitro, alkyl, haloalkyl, alkylthio, haloalkylthio, alkoxy or haloalkoxy,
B¹, B² and B³ independently of one another are each hydrogen, halogen, cyano, nitro, alkyl, haloalkyl, cyanoalkyl, hydroxyalkyl, alkoxycarbonylalkyl, alkoxyalkyl, alkenyl, haloalkenyl, cyanoalkenyl, alkynyl, haloalkynyl, cyanoalkynyl, alkoxy, haloalkoxy, cyanoalkoxy, alkoxycarbonylalkoxy, alkoxyalkoxy, alkylthio, haloalkylthio, alkoxyalkylthio, alkylsulphinyl, haloalkylsulphinyl, alkoxyalkylsulphinyl, alkylsulphonyl, haloalkylsulphonyl, alkoxyalkylsulphonyl, acyl, haloalkylcarbonyl, carboxyl, alkoxycarbonyl or NR³R⁴, where R³ and R⁴ independently of one another are hydrogen, alkyl, haloalkyl, cyanoalkyl, hydroxyalkyl, alkoxyalkyl, alkylthioalkyl, alkenyl, haloalkenyl, cyanoalkenyl, alkynyl, haloalkynyl, cyanoalkynyl, acyl or alkoxycarbonyl, or R³ and R⁴, together with the N atom to which they are attached, may form an optionally substituted saturated or unsaturated, five- to eight-membered ring which is optionally interrupted by heteroatoms,
n is the number 0, 1 or 2,
R¹ is hydrogen or alkyl,
R² is hydrogen, halogen, cyano, nitro, alkyl, haloalkyl, cyanoalkyl, alkoxyalkyl, alkenyl, haloalkenyl, alkynyl, haloalkynyl or haloalkoxyalkyl, or is optionally substituted cycloalkyl or cycloalkenyl each of which is optionally interrupted by one or more heteroatoms,
where the 3-[1-(3-haloalkyl)triazolyl]phenyl sulphide derivatives of the formula (I) are not 3-(difluoromethyl)-1-{2,4-dimethyl-5-[(2,2,2-trifluoroethyl)sulphanyl]phenyl}-1H-1,2,4-triazole, 3-(difluoromethyl)-1-{2,4-dimethyl-5-[(2,2,2-trifluoroethyl)sulphinyl]phenyl}-1H-1,2,4-triazole, 1-{5-[(2,2-difluoroethyl)sulphanyl]2,4-dimethylphenyl}-3-(difluoromethyl)-1H-1,2,4-triazole and 1-{5-(2,2-difluoroethyl)sulphinyl]-2,4-dimethylphenyl}-3-(difluoromethyl)-1H-1,2,4-triazole.

2. Compounds of the formula (I) according to Claim 1, in which
A¹ is CHF₂,
A² is hydrogen,
B⁰ is hydrogen, amino, halogen, cyano, nitro, (C₁-C₆) alkyl, (C₁-C₆) haloalkyl, (C₁-C₆)alkylthio, (C₁-C₆)haloalkylthio, (C₁-C₆)alkoxy or (C₁-C₆)haloalkoxy,
B¹, B² and B³ independently of one another are each hydrogen, halogen, cyano, nitro, (C₁-C₆)alkyl, (C₁-C₆) haloalkyl, (C₁-C₆) cyanoalkyl, (C₁-C₆) hydroxyalkyl, (C₂-C₇) alkoxycarbonyl- (C₁-C₆)alkyl, (C₁-C₆)alkoxy-(C₁-C₆)alkyl, (C₂-C₆)alkenyl, (C₂-C₆) haloalkenyl (C₂-C₆)cyanoalkenyl, (C₂-C₆) alkynyl, (C₂-C₆)haloalkynyl, (C₂-C₆)cyanoalkynyl, (C₁-C₆)alkoxy, (C₁-C₆) haloalkoxy, (C₁-C₆)cyanoalkoxy, (C₂-C₅) alkoxycarbonyl- (C₁-C₆)alkoxy, (C₁-C₃) alkoxy- (C₁-C₆) alkoxy, (C₁-C₆)alkylthio, (C₁-C₆)haloalkylthio, (C₁-C₃)alkoxy-(C₁-C₆)alkylthio, (C₁-C₆)alkylsulphinyl, (C₁-C₆)haloalkylsulphinyl, (C₁-C₃)alkoxy-(C₁-C₆)alkylsulphinyl, (C₁-C₆)alkylsulphonyl, (C₁-C₆)haloalkylsulphonyl, (C₁-C₃)alkoxy-(C₁-C₆)alkylsulphonyl, (C₁-C₇)acyl, (C₂-C₅) haloalkylcarbonyl, carboxyl, (C₂-C₇) alkoxycarbonyl or NR³R⁴, where R³ and R⁴ independently of one another are hydrogen, (C₁-C₆)alkyl, (C₁-C₆) haloalkyl, (C₁-C₆)cyanoalkyl, (C₁-C₆) hydroxyalkyl, (C₁-C₆)alkoxy-(C₁-C₆)alkyl, (C₁-C₆)alkylthio-(C₁-C₆)alkyl, (C₂-C₆) alkenyl, (C₂-C₆) haloalkenyl, (C₂-C₆) cyanoalkenyl, (C₂-C₆) alkynyl, (C₂-C₆)haloalkynyl, (C₂-C₆)cyanoalkynyl, (C₁-C₇)acyl, (C₂-C₇) alkoxycarbonyl, or R³ and R⁴, together with the N atom to which they are attached, may form an optionally (C₁-C₄)alkyl-, (C₁-C₄) alkoxy-, (C₁-C₄) haloalkyl-substituted saturated or unsaturated, five- or six-membered ring which is optionally interrupted by heteroatoms,
n is the number 0, 1 or 2,
R¹ is hydrogen or (C₁-C₄)alkyl,
R² is hydrogen, halogen, cyano, nitro, (C₁-C₆)alkyl, (C₁-C₆) haloalkyl, (C₁-C₆) cyanoalkyl, (C₂-C₆)alkenyl, (C₂-C₆)haloalkenyl, (C₂-C₆)alkynyl, (C₂-C₆) haloalkynyl, (C₁-C₆) alkoxy-(C₁-C₆)alkyl or (C₁-C₆)haloalkoxy-(C₁-C₆)alkyl, or is optionally substituted (C₃-C₈)cycloalkyl or (C₃-C₈) cycloalkenyl each of which is optionally interrupted by one or more heteroatoms.

3. Process for preparing compounds of the formula (I) according to either of Claims 1 and 2, **characterized in that**
(A) anilines of the formula (VII) 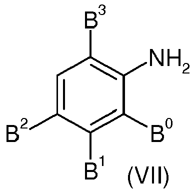 are reacted with sodium nitrite to give diazonium salts, which are then reduced to give hydrazines of the general formula (VI) 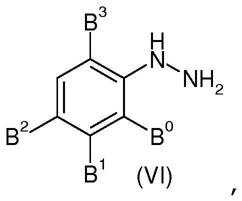
(B) the hydrazines of the formula (VI) are converted in the presence of esters of the formula (VIII) 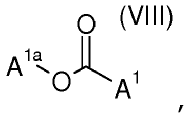 where A^{1a} is alkyl,
into hydrazides of the general formula (V) 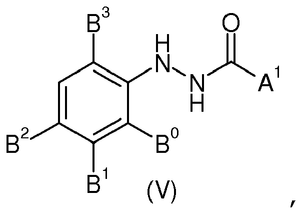
(C) hydrazides of the formula (V) are reacted with formamidine hydrochloride in the presence of a base to give triazoles of the formula (IV-A) 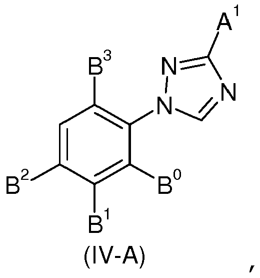
(D) the triazoles of the general formula (IV-A) are reacted by sulphochlorination to give sulphonyl chlorides of the general formula (III-A) 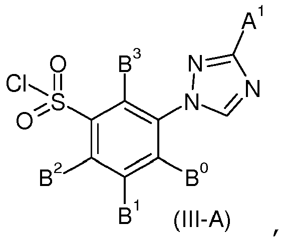
(E) the sulphonyl chlorides of the formula (III-A) are converted by reduction into disulphides of the general formula (II-A) 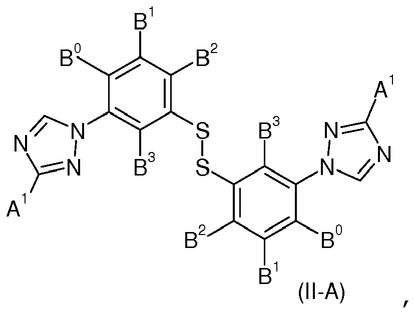
(F) the disulphides of the general formula (II-A) are reacted with electrophiles of the general formula (IX) 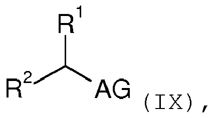 where AG is a leaving group such as chloro, bromo, tosylate, mesylate or triflate,
to give sulphides of the general formula (I-Aa) 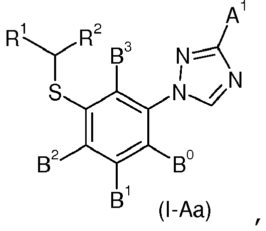
(G) the compounds of the formula (I-Aa) are reacted with oxidizing agents to give sulphoxides of the general formula (I-Ab) 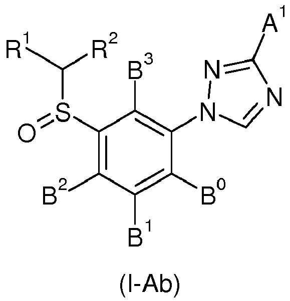 and sulphones of the general formula (I-Ac) 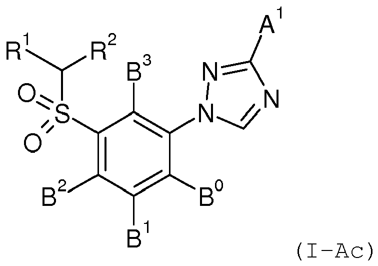 where A¹, B⁰, B¹, B², B³, R¹ and R² are as defined according to either of Claims 1 and 2.

4. Process according to Claim 3, **characterized in that**
the compounds of the general formula (IV-A) where A¹, B⁰, B¹, B² and B³ are as defined according to either of Claims 1 and 2
are prepared by
reacting boronic acids of the general formula (VIII) 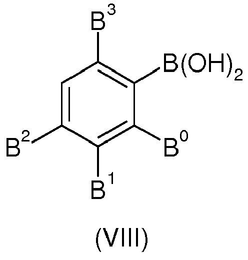 in a copper-catalyzed coupling reaction with triazoles of the general formula (IX-A) 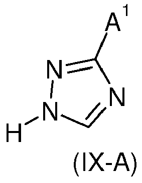 to give the compounds of the formula (IV-A).

5. Process according to Claim 3, **characterized in that**
the compounds of the general formula (IV-A)
where A¹, B⁰, B¹, B² and B³ are as defined according to either of Claims 1 and 2
are prepared by reacting
hydrazines of the general formula (VI) 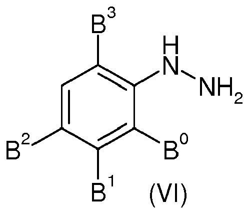 in the presence of amidines of the formula (XI) or salts thereof 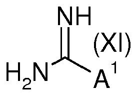 to give amidrazones of the formula (X) 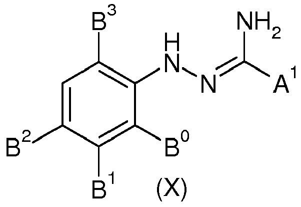 which are reacted with an orthoformate to give triazoles of the formula (IV-A).

6. Active compound composition comprising at least one 3-[1-(3-haloalkyl)triazolyl]phenyl sulphide derivative of the formula (I) according to either of Claims 1 and 2 and at least one other active insecticidal, acaricidal or nematicidal compound selected from the group consisting of
(1) Acetylcholinesterase (AChE) inhibitors, such as, for example,
carbamates, for example alanycarb, aldicarb, bendiocarb, benfuracarb, butocarboxim, butoxy-carboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, triazamate, trimethacarb, XMC and xylylcarb; or
organophosphates, for example acephate, azamethiphos, azinphos (-methyl, -ethyl), cadusafos, chlorethoxyfos, chlorfenvinphos, chlormephos, chlorpyrifos (-methyl), coumaphos, cyanophos, demeton-S-methyl, diazinon, dichlorvos/DDVP, dicrotophos, dimethoate, dimethylvinphos, disulfoton, EPN, ethion, ethoprophos, famphur, fenamiphos, fenitrothion, fenthion, fosthiazate, heptenophos, isofenphos, isopropyl O-(methoxyaminothiophosphoryl) salicylate, isoxathion, malathion, mecarbam, methamidophos, methidathion, mevinphos, monocrotophos, naled, omethoate, oxydemeton-methyl, parathion (-methyl), phenthoate, phorate, phosalone, phosmet, phosphamidon, phoxim, pirimiphos (-methyl), profenofos, propetamphos, prothiofos, pyraclofos, pyridaphenthion, quinalphos, sulfotep, tebupirimfos, temephos, terbufos, tetrachlorvinphos, thiometon, triazophos, triclorfon and vamidothion.
(2) GABA-gated chloride channel antagonists, such as, for example,
organochlorines, for example chlordane and endosulfan (alpha-); or
fiproles (phenylpyrazoles), for example ethiprole, fipronil, pyrafluprole and pyriprole.
(3) Sodium channel modulators/voltage-dependent sodium channel blockers, such as, for example, pyrethroids, for example acrinathrin, allethrin (d-cis-trans, d-trans), bifenthrin, bioallethrin, bioallethrin-S-cyclopentenyl, bioresmethrin, cycloprothrin, cyfluthrin (beta-), cyhalothrin (gamma-, lambda-), cypermethrin (alpha-, beta-, theta-, zeta-), cyphenothrin [(1*R*)-*trans*-isomers], deltamethrin, dimefluthrin, empenthrin [(*EZ*)*-*(1*R*)-isomers], esfenvalerate, etofenprox, fenpropathrin, fenvalerate, flucythrinate, flumethrin, fluvalinate (tau-), halfenprox, imiprothrin, metofluthrin, permethrin, phenothrin [(1*R*)-trans-isomer], prallethrin, profluthrin, pyrethrins (pyrethrum), resmethrin, RU 15525, silafluofen, tefluthrin, tetramethrin [(1*R*)-isomers], tralomethrin, transfluthrin and ZXI 8901; or
DDT; or methoxychlor.
(4) Nicotinergic acetylcholine receptor agonists, such as, for example,
neonicotinoids, for example acetamiprid, clothianidin, dinotefuran, imidacloprid, nitenpyram, thiacloprid, thiamethoxam; or
nicotine.
(5) Allosteric acetylcholine receptor modulators (agonists), such as, for example,
spinosyns, for example spinetoram and spinosad.
(6) Chloride channel activators, such as, for example,
avermectins/milbemycins, for example abamectin, emamectin benzoate, lepimectin and milbemectin.
(7) Juvenile hormone analogues, for example hydroprene, kinoprene, methoprene; or fenoxycarb; pyriproxyfen.
(8) Active compounds with unknown or non-specific mechanisms of action, such as, for example, fumigants, for example methyl bromide and other alkyl halides; or
chloropicrin; sulphuryl fluoride; borax; tartar emetic.
(9) Selective antifeedants, for example pymetrozine; or flonicamid.
(10) Mite growth inhibitors, for example clofentezine, diflovidazin, hexythiazox, etoxazole.
(11) Microbial disruptors of the insect gut membrane, such as, for example, *Bacillus thuringiensis* subspecies *israelensis, Bacillus sphaericus, Bacillus thuringiensis* subspecies *aizawai, Bacillus thuringiensis* subspecies *kurstaki, Bacillus thuringiensis* subspecies *tenebrionis,* and BT plant proteins, for example Cry1Ab, Cry1Ac, Cry1Fa, Cry2Ab, mCry3A, Cry3Ab, Cry3Bb, Cry34/35Ab1.
(12) Oxidative phosphorylation inhibitors, ATP disruptors, such as, for example, diafenthiuron; or
organotin compounds, for example azocyclotin, cyhexatin, fenbutatin oxide; or
propargite; tetradifon.
(13) Oxidative phoshorylation decouplers acting by interrupting the H proton gradient, such as, for example, chlorfenapyr and DNOC.
(14) Nicotinergic acetylcholine receptor antagonists, such as, for example, bensultap, cartap (hydrochloride), thiocylam, and thiosultap (sodium).
(15) Chitin biosynthesis inhibitors, type 0, such as, for example, benzoylureas, for example bistrifluron, chlorfluazuron, diflubenzuron, flucycloxuron, flufenoxuron, hexaflumuron, lufenuron, novaluron, noviflumuron, teflubenzuron and triflumuron.
(16) Chitin biosynthesis inhibitors, type 1, such as, for example, buprofezin.
(17) Moulting disruptors, such as, for example, cyromazine.
(18) Ecdysone agonists/disruptors, such as, for example,
diacylhydrazines, for example chromafenozide, halofenozide, methoxyfenozide and tebufenozide.
(19) Octopaminergic agonists, such as, for example, amitraz.
(20) Complex-III electron transport inhibitors, such as, for example, hydramethylnone; acequinocyl; fluacrypyrim.
(21) Complex-I electron transport inhibitors, for example from the group of the METI acaricides, for example fenazaquin, fenpyroximate, pyrimidifen, pyridaben, tebufenpyrad, tolfenpyrad; or
rotenone (Derris).
(22) Voltage-dependent sodium channel blockers, for example indoxacarb; metaflumizone.
(23) Inhibitors of acetyl-CoA carboxylase, such as, for example, tetronic acid derivatives, for example spirodiclofen and spiromesifen; or tetramic acid derivatives, for example spirotetramat.
(24) Complex-IV electron transport inhibitors, such as, for example, phosphines, for example aluminium phosphide, calcium phosphide, phosphine, zinc phosphide; or cyanide.
(25) Complex-II electron transport inhibitors, such as, for example, cyenopyrafen.
(28) Ryanodine receptor effectors, such as, for example, diamides, for example flubendiamide, chlorantraniliprole (Rynaxypyr), cyantraniliprole (Cyazypyr) and also 3-bromo-N-{2-bromo-4-chloro-6-[(1-cyclopropylethyl)carbamoyl]phenyl}-1-(3-chloropyridin-2-yl)-1H-pyrazole-5-carboxamide or methyl 2-[3,5-dibromo-2-({[3-bromo-1-(3-chloropyridin-2-yl)-1H-pyrazol-5-yl]carbonyl}amino)benzoyl]-1,2-dimethylhydrazinecarboxylate.
Further active compounds with unknown mechanism of action, such as, for example, azadirachtin, amidoflumet, benzoximate, bifenazate, chinomethionat, cryolite, cyflumetofen, dicofol, fluensulfone (5-chloro-2-[(3,4,4-trifluorobut-3-en-1-yl)sulphonyl]-1,3-thiazole), flufenerim, pyridalyl and pyrifluquinazon; and also products based on Bacillus firmus (1-1582, BioNeem, Votivo) and also the known active compounds below 4-{[(6-bromopyrid-3-yl)methyl](2-fluoroethyl)amino}furan-2(5H)-one, 4-{[(6-fluoropyrid-3-yl)methyl] (2,2-difluoroethyl)amino}furan-2(5H)-one, 4-{[(2-chloro-1,3-thiazol-5-yl)methyl](2-fluoroethyl)amino}furan-2(5H)-one, 4-{[(6-chloropyrid-3-yl)methyl](2-fluoroethyl)amino}furan-2(5H)-one, 4-{[(6-chloropyrid-3-yl)methyl](2,2-difluoroethyl)amino}furan-2(5H)-one, 4-{[(6-chloro-5-fluoropyrid-3-yl)methyl](methyl)amino}furan-2(5H)-one, 4-{[(5,6-dichloropyrid-3-yl)methyl](2-fluoroethyl)amino}furan-2(5H)-one, 4-{[(6-chloro-5-fluoropyrid-3-yl)methyl](cyclopropyl)amino}furan-2(5H)-one, 4-{[(6-chloropyrid-3-yl)methyl](cyclopropyl)amino}furan-2(5H)-one, 4-{[(6-chloropyrid-3-yl)methyl](methyl)amino}furan-2(5H)-one, [(6-chloropyridin-3-yl)methyl](methyl)oxido-λ⁴-sulphanylidenecyanamide, [1-(6-chloropyridin-3-yl)ethyl](methyl)oxido-λ⁴-sulphanylidenecyanamide and its diastereomers (A) and (B) 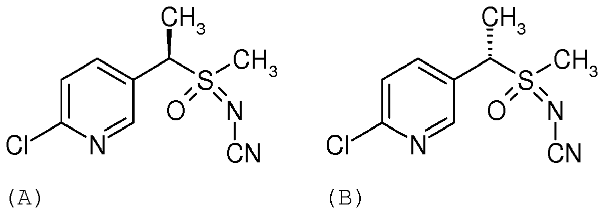 [(6-trifluoromethylpyridin-3-yl)methyl](methyl)oxido-λ⁴-sulphanylidenecyanamide, sulfoxaflor
11-(4-chloro-2,6-dimethylphenyl)-12-hydroxy-1,4-dioxa-9-azadispiro[4.2.4.2]tetradec-11-en-10-one,
3-(4'-fluoro-2,4-dimethylbiphenyl-3-yl)-4-hydroxy-8-oxa-1-azaspiro[4.5]dec-3-en-2-one,
1-[2-fluoro-4-methyl-5-[(2,2,2-trifluoroethyl)sulphinyl]phenyl]-3-(trifluoromethyl)-1H-1,2,4-triazole-5-amine, [(3S,4aR,12R,12aS,12bS)-3-[(cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methyl cyclopropanecarboxylate, 2-cyano-3-(difluoromethoxy)-N,N-dimethylbenzenesulphonamide, 2-cyano-3-(difluoromethoxy)-N-methylbenzenesulphonamide, 2-cyano-3-(difluoromethoxy)-N-ethylbenzenesulphonamide, 4-(difluoromethoxy)-N-ethyl-N-methyl-1,2-benzothiazole-3-amine 1,1-dioxide and N-[1-(2,3-dimethylphenyl)-2-(3,5-dimethylphenyl)ethyl]-4,5-dihydro-1,3-thiazole-2-amine,
and/or at least one further active fungicidal compound selected from the group consisting of
(1) Ergosterol biosynthesis inhibitors, such as, for example, aldimorph, azaconazole, bitertanol, bromuconazole, cyproconazole, diclobutrazole, difenoconazole, diniconazole, diniconazole-M, dodemorph, dodemorph acetate, epoxiconazole, etaconazole, fenarimol, fenbuconazole, fenhexamid, fenpropidin, fenpropimorph, fluquinconazole, flurprimidol, flusilazole, flutriafol, furconazole, furconazole-cis, hexaconazole, imazalil, imazalil sulphate, imibenconazole, ipconazole, metconazole, myclobutanil, naftifine, nuarimol, oxpoconazole, paclobutrazol, pefurazoate, penconazole, piperalin, prochloraz, propiconazole, prothioconazole, pyributicarb, pyrifenox, quinconazole, simeconazole, spiroxamine, tebuconazole, terbinafine, tetraconazole, triadimefon, triadimenol, tridemorph, triflumizole, triforine, triticonazole, uniconazole, uniconazole-p, viniconazole, voriconazole, 1-(4-chlorophenyl)-2-(1H-1,2,4-triazol-1-yl)cycloheptanol, methyl 1-(2,2-dimethyl-2,3-dihydro-1H-inden-1-yl)-1H-imidazole-5-carboxylate, N'-{5-(difluoromethyl)-2-methyl-4-[3-(trimethylsilyl)propoxy]phenyl}-N-ethyl-N-methylimidoformamide, N-ethyl-N-methyl-N'-{2-methyl-5-(trifluoromethyl)-4-[3-(tri-methylsilyl)propoxy]phenyl}imidoformamide and O-[1-(4-methoxyphenoxy)-3,3-dimethylbutan-2-yl]-1H-imidazole-1-carbothioate.
(2) Respiration inhibitors (respiratory-chain inhibitors), such as, for example, bixafen, boscalid, carboxin, diflumetorim, fenfuram, fluopyram, flutolanil, fluxapyroxad, furametpyr, furmecyclox, isopyrazam mixture of the syn-epimeric racemate 1RS,4SR,9RS and of the anti-epimeric racemate 1RS,4SR,9SR, isopyrazam (anti-epimeric racemate), isopyrazam (anti-epimeric enantiomer 1R,4S,9S), isopyrazam (anti-epimeric enantiomer 1S,4R,9R), isopyrazam (syn-epimeric racemate 1RS,4SR,9RS), isopyrazam (syn-epimeric enantiomer 1R,4S,9R), isopyrazam (syn-epimeric enantiomer 1S,4R,9S), mepronil, oxycarboxin, penflufen, penthiopyrad, sedaxane, thifluzamid, 1-methyl-N-[2-(1,1,2,2-tetrafluoroethoxy)phenyl]-3-(trifluoromethyl)-1H-pyrazole-4-carboxamide, 3-(difluoromethyl)-1-methyl-N-[2-(1,1,2,2-tetrafluoroethoxy)phenyl]-1H-pyrazole-4-carboxamide, 3-(difluoromethyl)-N-[4-fluoro-2-(1,1,2,3,3,3-hexafluoropropoxy)phenyl]-1-methyl-1H-pyrazole-4-carboxamide and N-[1-(2,4-dichlorophenyl)-1-methoxypropan-2-yl]-3-(difluoromethyl)-1-methyl-1H-pyrazole-4-carboxamide.
(3) Respiration inhibitors (respiratory-chain inhibitors) on the complex III of the respiratory chain, such as, for example, ametoctradin, amisulbrom, azoxystrobin, cyazofamid, dimoxystrobin, enestroburin, famoxadon, fenamidon, fluoxastrobin, kresoxim-methyl, metominostrobin, orysastrobin, picoxystrobin, pyraclostrobin, pyrametostrobin, pyraoxystrobin, pyribencarb, trifloxystrobin, (2E)-2-(2-{[6-(3-chloro-2-methylphenoxy)-5-fluoropyrimidin-4-yl]oxy}phenyl)-2-(methoxyimino)-N-methylethanamide, (2E)-2-(methoxyimino)-N-methyl-2-(2-{[({(1E)-1-[3-(trifluoromethyl)phenyl]ethylidene}amino)oxy]methy 1}phenyl)ethanamide, (2E)-2-(methoxyimino)-N-methyl-2-{2-[(E)-({1-[3-(trifluoromethyl)phenyl]ethoxy}imino)methyl]phenyl}ethanamid e, (2E)-2-{2-[({[(1E)-1-(3-{[(E)-1-fluoro-2-phenylethenyl]oxy}phenyl)ethylidene]amino}oxy)meth yl]phenyl}-2-(methoxyimino)-N-methylethanamide, (2E)-2-{2-[({[(2E,3E)-4-(2,6-dichlorophenyl)but-3-en-2-ylidene]amino}oxy)methyl]phenyl}-2-(methoxyimino)-N-methylethanamide, 2-chloro-N-(1,1,3-trimethyl-2,3-dihydro-1H-inden-4-yl)pyridine-3-carboxamide, 5-methoxy-2-methyl-4-(2-{[({(1E)-1-[3-(trifluoromethyl)phenyl]ethylidene}amino)oxy]methy 1}phenyl)-2,4-dihydro-3H-1,2,4-triazol-3-one, methyl (2E)-2-{2-[({cyclopropyl[(4-methoxyphenyl)imino]-methyl}sulphanyl)methyl]phenyl}-3-methoxyprop-2-enoate, N-(3-ethyl-3,5,5-trimethylcyclohexyl)-3-(formylamino)-2-hydroxybenzamide, 2-{2-[(2,5-dimethylphenoxy)methyl]phenyl}-2-methoxy-N-methylacetamide and (2R)-2-{2-[(2,5-dimethylphenoxy)methyl]phenyl}-2-methoxy-N-methylacetamide.
(4) Mitosis and cell division inhibitors, such as, for example, benomyl, carbendazim, chlorfenazole, diethofencarb, ethaboxam, fluopicolid, fuberidazole, pencycuron, thiabendazole, thiophanate-methyl, thiophanate, zoxamide, 5-chloro-7-(4-methylpiperidin-1-yl)-6-(2,4,6-trifluorophenyl)[1,2,4]triazolo[1,5-a]pyrimidine and 3-chloro-5-(6-chloropyridin-3-yl)-6-methyl-4-(2,4,6-trifluorophenyl)pyridazine.
(5) Compounds with multi-site activity, such as, for example, Bordeaux mixture, captafol, captan, chlorothalonil, copper preparations such as copper hydroxide, copper naphthenate, copper oxide, copper oxychloride, copper sulphate, dichlofluanid, dithianon, dodine, dodine free base, ferbam, fluorofolpet, folpet, guazatine, guazatine acetate, iminoctadine, iminoctadine albesilate, iminoctadine triacetate, man copper, mancozeb, maneb, metiram, metiram-zinc, oxine-copper, propamidine, propineb, sulphur and sulphur preparations such as, for example, calcium polysulphide, thiram, tolylfluanid, zineb and ziram.
(6) Resistance inductors, such as, for example, acibenzolar-S-methyl, isotianil, probenazole and tiadinil.
(7) Amino acid and protein biosynthesis inhibitors, such as, for example, andoprim, blasticidin-S, cyprodinil, kasugamycin, kasugamycin hydrochloride hydrate, mepanipyrim and pyrimethanil.
(8) ATP production inhibitors, such as, for example, fentin acetate, fentin chloride, fentin hydroxide and silthiofan.
(9) Cell wall synthesis inhibitors, such as, for example, benthiavalicarb, dimethomorph, flumorph, iprovalicarb, mandipropamid, polyoxins, polyoxorim, validamycin A and valifenalate.
(10) Lipid and membrane synthesis inhibitors, such as, for example, biphenyl, chloroneb, dicloran, edifenphos, etridiazole, iodocarb, iprobenfos, isoprothiolane, propamocarb, propamocarb hydrochloride, prothiocarb, pyrazophos, quintozene, tecnazene and tolclofos-methyl.
(11) Melanin biosynthesis inhibitors, such as, for example, carpropamid, diclocymet, fenoxanil, fthalide, pyroquilon and tricyclazole.
(12) Nucleic acid synthesis inhibitors, such as, for example, benalaxyl, benalaxyl M (kiralaxyl), bupirimate, clozylacon, dimethirimol, ethirimol, furalaxyl, hymexazol, metalaxyl, metalaxyl-M (mefenoxam), ofurace, oxadixyl and oxolinic acid.
(13) Signal transduction inhibitors, such as, for example, chlozolinate, fenpiclonil, fludioxonil, iprodione, procymidon, quinoxyfen and vinclozoline.
(14) Decouplers, such as, for example, binapacryl, dinocap, ferimzone, fluazinam and meptyldinocap.
(15) Further compounds, such as, for example, benthiazole, bethoxazin, capsimycin, carvone, chinomethionat, chlazafenon, cufraneb, cyflufenamid, cymoxanil, cyprosulfamide, dazomet, debacarb, dichlorophen, diclomezine, difenzoquat, difenzoquat methylsulphate, diphenylamine, ecomat, fenpyrazamine, flumetover, fluoromid, flusulfamide, flutianil, fosetyl-aluminium, fosetyl-calcium, fosetyl-sodium, hexachlorobenzene, irumamycin, methasulphocarb, methyl isothiocyanate, metrafenone, mildiomycin, natamycin, nickel dimethyldithiocarbamate, nitrothal-isopropyl, octhilinone, oxamocarb, oxyfenthiin, pentachlorophenol and its salts, phenothrin, phosphoric acid and its salts, propamocarb-fosetylate, propanosine-sodium, proquinazid, pyrrolnitrin, tebufloquin, tecloftalam, tolnifanid, triazoxide, trichlamide, zarilamide, 1-(4-{4-[(5R)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, 1-(4-{4-[(5S)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, 1-(4-{4-[5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, 1-(4-methoxyphenoxy)-3,3-dimethylbutan-2-yl 1H-imidazole-1-carboxylate, 2,3,5,6-tetrachloro-4-(methylsulphonyl)pyridine, 2,3-dibutyl-6-chlorothieno[2,3-d]pyrimidin-4(3H)-one, 2-[5-methyl-3-(trifluoromethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanone, 2-[5-methyl-3-(trifluoromethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanone, 2-[5-methyl-3-(trifluoromethyl)-1H-pyrazol-1-yl]-1-{4-[4-(5-phenyl-4,5-dihydro-1,2-oxazol-3-yl)-1,3-thiazol-2-yl]piperidin-1-yl}ethanone, 2-butoxy-6-iodo-3-propyl-4H-chromen-4-one, 2-chloro-5-[2-chloro-1-(2,6-difluoro-4-methoxyphenyl)-4-methyl-1H-imidazol-5-yl]pyridine, 2-phenylphenol and its salts, 3,4,5-trichloropyridine-2,6-dicarbonitrile, 3-[5-(4-chlorophenyl)-2,3-dimethyl-1,2-oxazolidin-3-yl]pyridine, 3-chloro-5-(4-chlorophenyl)-4-(2,6-difluorophenyl)-6-methylpyridazine, 4-(4-chlorophenyl)-5-(2,6-difluorophenyl)-3,6-dimethylpyridazine, 5-amino-1,3,4-thiadiazole-2-thiol, 5-chloro-N'-phenyl-N'-(prop-2-yn-1-yl)thiophene-2-sulphonohydrazide, 5-methyl-6-octyl[1,2,4]triazolo[1,5-a]pyrimidin-7-amine, ethyl (2Z)-3-amino-2-cyano-3-phenylprop-2-enoate, N-(4-chlorobenzyl)-3-[3-methoxy-4-(prop-2-yn-1-yloxy)phenyl]propanamide, N-[(4-chlorophenyl)(cyano)methyl]-3-[3-methoxy-4-(prop-2-yn-1-yloxy)phenyl]propanamide, N-[(5-bromo-3-chloropyridin-2-yl)methyl]-2,4-dichloropyridine-3-carboxamide, N-[1-(5-bromo-3-chloropyridin-2-yl)ethyl]-2,4-dichloropyridine-3-carboxamide, N-[1-(5-bromo-3-chloropyridin-2-yl)ethyl]-2-fluoro-4-iodopyridine-3-carboxamide, N-{(E)-[(cyclopropylmethoxy)imino][6-(difluoromethoxy)-2,3-difluorophenyl]methyl}-2-phenylacetamide, N-{(Z)-[(cyclopropylmethoxy)imino][6-(difluoromethoxy)-2,3-difluorophenyl]methyl}-2-phenylacetamide, N-methyl-2-(1-{[5-methyl-3-(trifluoromethyl)-1H-pyrazol-1-yl]acetyl}piperidin-4-yl)-N-(1,2,3,4-tetrahydronaphthalen-1-yl)-1,3-thiazole-4-carboxamide, N-methyl-2-(1-{[5-methyl-3-(trifluoromethyl)-1H-pyrazol-1-yl]acetyl}piperidin-4-yl)-N-[(1R)-1,2,3,4-tetrahydronaphthalen-1-yl]-1,3-thiazole-4-carboxamide, N-methyl-2-(1-{[5-methyl-3-(trifluoromethyl)-1H-pyrazol-1-yl]acetyl}piperidin-4-yl)-N-[(1S)-1,2,3,4-tetrahydronaphthalen-1-yl]-1,3-thiazole-4-carboxamide, pentyl {6-[({[(1-methyl-1H-tetrazol-5-yl)(phenyl)methylidene]amino}oxy)methyl]pyridin-2-yl}carbamate, phenazine-1-carboxylic acid, quinoline-8-ol and quinoline-8-ol sulphate (2:1).
(16) Further compounds, such as, for example, 1-methyl-3-(trifluoromethyl)-N-[2'-(trifluoromethyl)biphenyl-2-yl]-1H-pyrazole-4-carboxamide, N-(4'-chlorobiphenyl-2-yl)-3-(difluoromethyl)-1-methyl-1H-pyrazole-4-carboxamide, N-(2',4'-dichlorobiphenyl-2-yl)-3-(difluoromethyl)-1-methyl-1H-pyrazole-4-carboxamide, 3-(difluoromethyl)-1-methyl-N-[4'-(trifluoromethyl)biphenyl-2-yl]-1H-pyrazole-4-carboxamide, N-(2',5'-difluorobiphenyl-2-yl)-1-methyl-3-(trifluoromethyl)-1H-pyrazole-4-carboxamide, 3-(difluoromethyl)-1-methyl-N-[4'-(prop-1-yn-1-yl)biphenyl-2-yl]-1H-pyrazole-4-carboxamide, 5-fluoro-1,3-dimethyl-N-[4'-(prop-1-yn-1-yl)biphenyl-2-yl]-1H-pyrazole-4-carboxamide, 2-chloro-N-[4'-(prop-1-yn-1-yl)biphenyl-2-yl]pyridine-3-carboxamide, 3-(difluoromethyl)-N-[4'-(3,3-dimethylbut-1-yn-1-yl)biphenyl-2-yl]-1-methyl-1H-pyrazole-4-carboxamide, N-[4'-(3,3-dimethylbut-1-yn-1-yl)biphenyl-2-yl]-5-fluoro-1,3-dimethyl-1H-pyrazole-4-carboxamide, 3-(difluoromethyl)-N-(4'-ethynylbiphenyl-2-yl)-1-methyl-1H-pyrazole-4-carboxamide, N-(4'-ethynylbiphenyl-2-yl)-5-fluoro-1,3-dimethyl-1H-pyrazole-4-carboxamide, 2-chloro-N-(4'-ethynylbiphenyl-2-yl)pyridine-3-carboxamide, 2-chloro-N-[4'-(3,3-dimethylbut-1-yn-1-yl)biphenyl-2-yl]pyridine-3-carboxamide, 4-(difluoromethyl)-2-methyl-N-[4'-(trifluoromethyl)biphenyl-2-yl]-1,3-thiazole-5-carboxamide, 5-fluoro-N-[4'-(3-hydroxy-3-methylbut-1-yn-1-yl)biphenyl-2-yl]-1,3-dimethyl-1H-pyrazole-4-carboxamide, 2-chloro-N-[4'-(3-hydroxy-3-methylbut-1-yn-1-yl)biphenyl-2-yl]pyridine-3-carboxamide, 3-(difluoromethyl)-N-[4'-(3-methoxy-3-methylbut-1-yn-1-yl)biphenyl-2-yl]-1-methyl-1H-pyrazole-4-carboxamide, 5-fluoro-N-[4'-(3-methoxy-3-methylbut-1-yn-1-yl)biphenyl-2-yl]-1,3-dimethyl-1H-pyrazole-4-carboxamide, 2-chloro-N-[4'-(3-methoxy-3-methylbut-1-yn-1-yl)biphenyl-2-yl]pyridine-3-carboxamide, (5-bromo-2-methoxy-4-methylpyridin-3-yl)(2,3,4-trimethoxy-6-methylphenyl)methanone and N-[2-(4-{[3-(4-chlorophenyl)prop-2-yn-1-yl]oxy}-3-methoxyphenyl)ethyl]-N2-(methylsulphonyl)valinamide.

7. Composition comprising at least one compound of the formula (I) according to either of Claims 1 and 2 or an active compound composition according to Claim 6, and at least one penetrant.

8. Compounds of the general formula (II-A) according to Claim 3 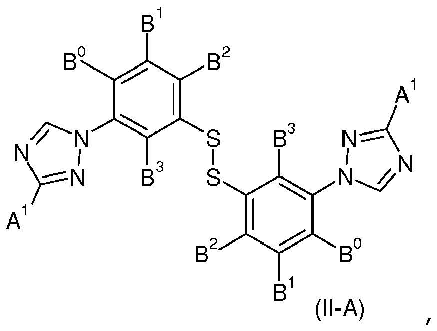 in which A¹, B⁰ and B² are as defined according to either of Claims 1 and 2, and B¹ and B³ are hydrogen.

9. Compounds of the general formula (III-A) according to Claim 3 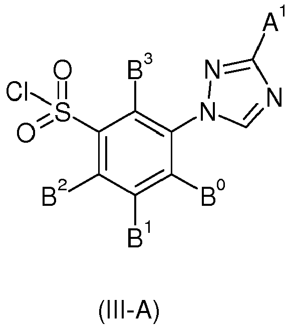 where A¹, B⁰ and B² are as defined in either of Claims 1 and 2
aund B¹ and B³ are hydrogen.

10. Compounds of the general formula (IV-A) according to Claim 3 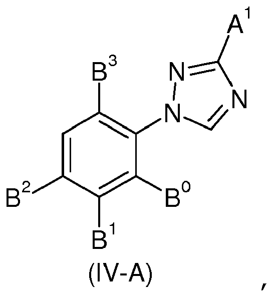 where
B¹ and B³ are hydrogen, and
B⁰ is hydrogen, methyl, ethyl, fluoro, chloro, methoxy, cyano, CHF₂, CF₃ or OCF₃,
B² is hydrogen, methyl, ethyl, fluoro, chloro, methoxy, cyano, CHF₂, CF₃ or OCF₃.

11. Compound of the general formula (V) according to Claim 3 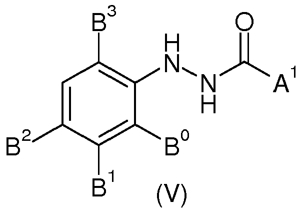 where
B¹ and B³ are hydrogen,
A¹ is CHF₂,
B⁰ is methyl, ethyl, fluoro, chloro, methoxy, cyano, CHF₂, CF₃ or OCF₃,
B² is hydrogen, ethyl, fluoro, chloro, cyano, CHF₂, CF₃ or OCF₃.

12. Agrochemical compositions **characterized in that** they comprise at least one compound of the formula (I) according to Claims 1 to 2 or a composition according to either of Claims 6 and 7, and also extenders and/or surface-active substances.

13. Use of compounds of the formula (I) according to either of Claims 1 and 2 or of a composition according to any of Claims 6, 7 and 12 for controlling animal pests in crop protection or in the protection of materials.

14. Compound of the formula (I) according to either of Claims 1 and 2 or a composition according to any of Claims 6, 7 and 12 for use in a process in the veterinary sector.

## Revendications

1. Dérivés de sulfure de 3-[1-(3-haloalkyl)-triazolyl]-phényle de formule (I) 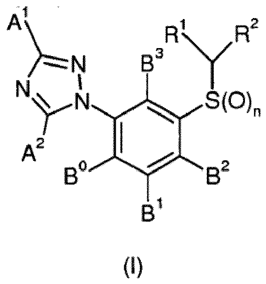 dans laquelle
A¹ représente CHF₂,
A² représente hydrogène,
B⁰ représente hydrogène, amino, halogène, cyano, nitro, alkyle, halogénoalkyle, alkylthio, halogénoalkylthio, alcoxy ou halogénoalcoxy,
B¹, B², B³ représentent indépendamment les uns des autres hydrogène, halogène, cyano, nitro, alkyle, halogénoalkyle, cyanoalkyle, hydroxyalkyle, alcoxycarbonylalkyle, alcoxyalkyle, alcényle, halogénalcényle cyanoalcényle, alcynyle, halogénalcynyle, cyanoalcynyle, alcoxy, halogénalcoxy, cyanoalcoxy, alcoxycarbonylalcoxy, alcoxyalcoxy, alkylthio, halogénoalkylthio, alcoxyalkylthio, alkylsulfinyle, halogénoalkylsulfinyle, alcoxyalkylsulfinyle, alkylsulfonyle, halogénoalkylsulfonyle, alcoxyalkylsulfonyle, acyle, halogénoalkylcarbonyle, carboxyle, alcoxycarbonyle ou NR³R⁴, R³ et R⁴ représentant indépendamment l'un de l'autre hydrogène, alkyle, halogénoalkyle, cyanoalkyle, hydroxyalkyle, alcoxyalkyle, alkylthioalkyle, alcényle, halogénoalcényle, cyanoalcényle, alcynyle, halogénoalcynyle, cyanoalcynyle, acyle, alcoxycarbonyle, ou R³ et R⁴ pouvant former ensemble avec l'atome N auquel ils sont reliés un cycle saturé ou insaturé de cinq à huit éléments éventuellement substitué et éventuellement interrompu par des hétéroatomes,
n représente le nombre 0, 1 ou 2,
R¹ représente hydrogène ou alkyle,
R² représente hydrogène, halogène, cyano, nitro, alkyle, halogénoalkyle, cyanoalkyle, alcoxyalkyle, alcényle, halogénoalcényle, alcynyle, halogénoalcynyle, halogénoalcoxyalkyle ; cycloalkyle ou cycloalcényle éventuellement substitué, éventuellement interrompu par un ou plusieurs hétéroatomes,
les dérivés de sulfure de 3-[1-(3-haloalkyl)-triazolyl]-phényle de formule (I) n'étant pas le 3-(difluorométhyl)-1-{2,4-diméthyl-5-[(2,2,2-trifluoroéthyl)-sulfanyl]-phényl}-1H-1,2,4-triazol, le 3-(difluorométhyl)-1-{2,4-diméthyl-5-[(2,2,2-trifluoréthyl)-sulfinyl]-phényl}-1H-1,2,4-triazol, le 1-{5-[(2,2-difluoréthyl)sulfanyl]-2,4-diméthylphényl}-3-(difluorométhyl)-1H-1,2,4-triazol et le 1-{5-[(2,2-difluoréthyl)sulfinyl]-2,4-diméthylphényl}-3-(difluorométhyl)-1H-1,2,4-triazol.

2. Composés de formule (I) selon la revendication 1, dans lesquels
A¹ représente CHF₂,
A² représente hydrogène,
B⁰ représente hydrogène, amino, halogène, cyano, nitro, alkyle en (C₁-C₆), halogénoalkyle en (C₁-C₆), alkylthio en (C₁-C₆), halogénoalkylthio en (C₁-C₆), alcoxy en (C₁-C₆) ou halogénoalcoxy en (C₁-C₆),
B¹, B², B³ représentent indépendamment les uns des autres hydrogène, halogène, cyano, nitro, alkyle en (C₁-C₆), halogénoalkyle en (C₁-C₆), cyanoalkyle en (C₁-C₆), hydroxyalkyle en (C₁-C₆), alcoxycarbonyle en (C₂-C₇)-alkyle en (C₁-C₆), alcoxy en (C₁-C₆)-alkyle en (C₁-C₆), alcényle en (C₂-C₆), halogénalcényle en (C₂-C₆), cyanoalcényle en (C₂-C₆), alcynyle en (C₂-C₆), halogénalcynyle en (C₂-C₆), cyanoalcynyle en (C₂-C₆), alcoxy en (C₁-C₆), halogénalcoxy en (C₁-C₆), cyanoalcoxy en (C₁-C₆), alcoxycarbonyle en (C₂-C₅)-alcoxy en (C₁-C₆), alcoxy en (C₁-C₃)-alcoxy en (C₁-C₆), alkylthio en (C₁-C₆), halogénoalkylthio en (C₁-C₆), alcoxy en (C₁-C₃)-alkylthio en (C₁-C₆), alkylsulfinyle en (C₁-C₆), halogénoalkylsulfinyle en (C₁-C₆), alcoxy en (C₁-C₃)-alkylsulfinyle en (C₁-C₆), alkylsulfonyle en (C₁-C₆), halogénoalkylsulfonyle en (C₁-C₆), alcoxy en (C₁-C₃)-alkylsulfonyle en (C₁-C₆), acyle en (C₁-C₇), halogénoalkylcarbonyle en (C₂-C₅), carboxyle, alcoxycarbonyle en (C₂-C₇) ou NR³R⁴, R³ et R⁴ représentant indépendamment l'un de l'autre hydrogène, alkyle en (C₁-C₆), halogénoalkyle en (C₁-C₆), cyanoalkyle en (C₁-C₆), hydroxyalkyle en (C₁-C₆), alcoxy en (C₁-C₆)-alkyle en (C₁-C₆), alkylthio en (C₁-C₆)-alkyle en (C₁-C₆), alcényle en (C₂-C₆), halogénoalcényle en (C₂-C₆), cyanoalcényle en (C₂-C₆), alcynyle en (C₂-C₆), halogénoalcynyle en (C₂-C₆), cyanoalcynyle en (C₂-C₆), acyle en (C₁-C₇), alcoxycarbonyle en (C₂-C₇), ou R³ et R⁴ pouvant former ensemble avec l'atome N auquel ils sont reliés un cycle saturé ou insaturé à cinq ou six éléments éventuellement substitué par alkyle en (C₁-C₄), alcoxy en (C₁-C₄), halogénoalkyle en (C₁-C₄) et éventuellement interrompu par des hétéroatomes,
n représente le nombre 0, 1 ou 2,
R¹ représente hydrogène ou alkyle en (C₁-C₄),
R² représente hydrogène, halogène, cyano, nitro, alkyle en (C₁-C₆), halogénoalkyle en (C₁-C₆), cyanoalkyle en (C₁-C₆), alcényle en (C₂-C₆), halogénoalcényle en (C₂-C₆), alcynyle en (C₂-C₆), halogénoalcynyle en (C₂-C₆), alcoxy en (C₁-C₆)-alkyle en (C₁-C₆), halogénoalcoxy en (C₁-C₆)-alkyle en (C₁-C₆) , cycloalkyle en (C₃-C₈) ou cycloalcényle en (C₃-C₈) éventuellement substitué, éventuellement interrompu par un ou plusieurs hétéroatomes.

3. Procédé de fabrication de composés de formule (I) selon l'une quelconque des revendications 1 à 2, **caractérisé en ce que**
(A) des anilines de formule (VII) 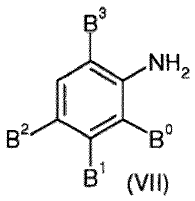 sont mises en réaction avec du nitrite de sodium pour former des sels de diazonium, puis réduites en hydrazines de formule générale (VI) 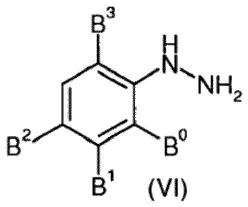
(B) les hydrazines de formule (VI) sont transformées en présence d'esters de formule (VIII) 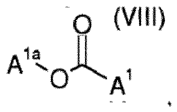 dans laquelle A^{1a} représente alkyle, en hydrazides de formule générale (V) 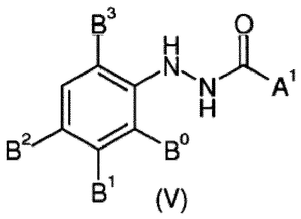
(C) les hydrazides de formule (V) sont mis en réaction avec du chlorhydrate de formamidine en présence d'une base pour former des triazoles de formule (IV-A) 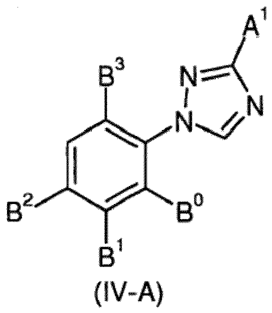
(D) les triazoles de formule générale (IV-A) sont transformés par sulfochloration en chlorures de sulfonyle de formule générale (III-A) 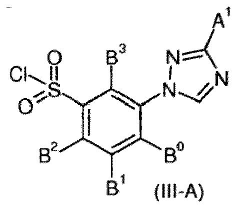
(E) les chlorures de sulfonyle de formule (III-A) sont transformés par réduction en disulfures de formule générale (II-A) 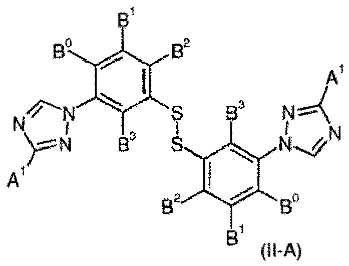
(F) les disulfures de formule générale (II-A) sont mis en réaction avec des électrophiles de formule générale (IX) 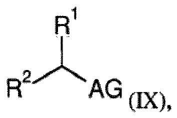 dans laquelle AG représente un groupe partant tel que chlore, brome, tosylate, mésylate ou triflate,
pour former des sulfures de formule générale (I-Aa) 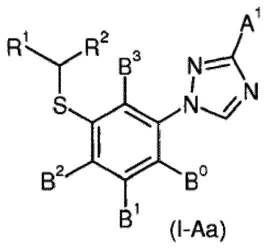
(G) les composés de formule (I-Aa) sont mis en réaction avec des oxydants pour former des sulfoxydes de formule générale (I-Ab) 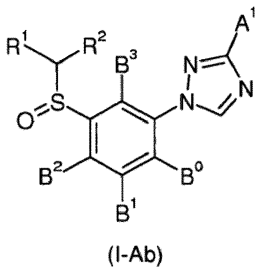 et des sulfones de formule générale (I-Ac) 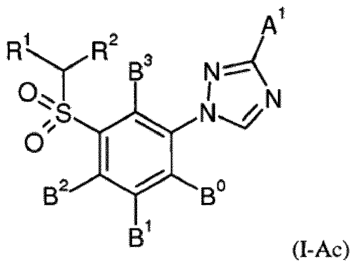 dans lesquelles A¹, B⁰, B¹, B², B³, R¹, R² ont les significations selon l'une quelconque des revendications 1 à 2.

4. Procédé selon la revendication 3, **caractérisé en ce que**
les composés de formule générale (IV-A),
dans laquelle A¹, B⁰, B¹, B² et B³ ont les significations selon l'une quelconque des revendications 1 à 2,
sont préparés par
mise en réaction d'acides boroniques de formule générale (VIII) 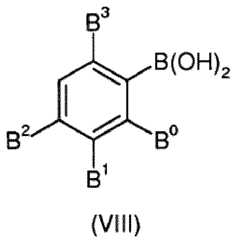 par une réaction de couplage catalysée par du cuivre, avec des triazoles de formule générale (IX-A) 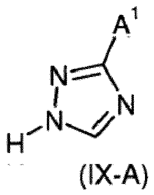 pour former les composés de formule (IV-A).

5. Procédé selon la revendication 3, **caractérisé en ce que**
les composés de formule générale (IV-A)
dans laquelle A¹, B⁰, B¹, B² et B³ ont les significations selon l'une quelconque des revendications 1 à 2,
sont préparés par
mise en réaction d'hydrazines de formule générale (VI) 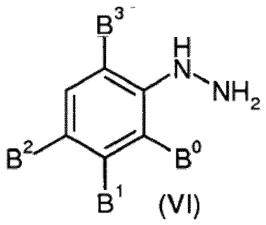 en présence d'amidines de formule (XI) ou leurs sels 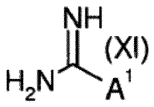 pour former des amidrazones de formule (X) 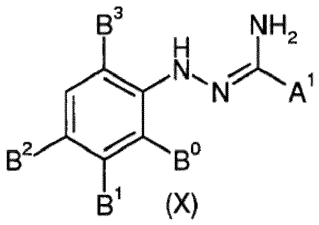 et celles-ci sont mises en réaction avec un orthoformate pour former des triazoles de formule (IV-A).

6. Composition d'agents actifs contenant au moins un dérivé de sulfure de 3-[1-(3-haloalkyl)-triazolyl]-phényle de formule (I) selon l'une quelconque des revendications 1 à 2, et au moins un autre agent actif insecticide, acaricide ou nématicide choisi dans le groupe constitué par
(1) les inhibiteurs d'acétylcholinestérase (AChE), tels que par exemple
les carbamates, p. ex. l'alanycarb, l'aldicarb, le bendiocarb, le benfuracarb, le butocarboxime, le butoxycarboxime, le carbaryl, le carbofurane, le carbosulfane, l'éthiofencarb, le fénobucarb, le formétanate, le furathiocarb, l'isoprocarb, le méthiocarb, le méthomyl, le métolcarb, l'oxamyl, le pirimicarb, le propoxur, le thiodicarb, le thiofanox, le triazamate, le triméthacarb, le XMC et le xylylcarb ; ou
les organophosphates, p. ex. l'acéphate, l'azaméthiphos, l'azinphos (-méthyl, -éthyl), le cadusafos, le chloréthoxyfos, le chlorfenvinphos, le chlorméphos, le chlorpyrifos (-méthyl), le coumaphos, le cyanophos, le déméton-S-méthyl, le diazinon, le dichlorvos/DDVP, le dicrotophos, le diméthoate, le diméthylvinphos, le disulfoton, l'EPN, l'éthion, l'éthoprophos, le famphur, le fénamiphos, le fénitrothion, le fenthion, le fosthiazate, l'hepténophos, l'isofenphos, l'*O*-(méthoxyaminothio-phosphoryl)salicylate d'isopropyle, l'isoxathion, le malathion, le mécarbam, le méthamidophos, le méthidathion, le mévinphos, le monocrotophos, le naled, l'ométhoate, l'oxydéméton-méthyl, le parathion (-méthyl), le phenthoate, le phorate, la phosalone, le phosmet, la phosphamidone, le phoxim, le pirimiphos (-méthyl), le profénofos, le propétamphos, le prothiofos, le pyraclofos, le pyridaphenthion, le quinalphos, le sulfotep, le tébupirimfos, le téméphos, le terbufos, le tétrachlorvinphos, le thiométon, le triazophos, le triclorfon et le vamidothion ;
(2) les antagonistes des canaux chlorure contrôlés par GABA, tels que par exemple
les organochlores, p. ex. le chlordane et l'endosulfane (alpha-) ; ou
les fiproles (phénylpyrazoles), p. ex. l'éthiprole, le fipronil, le pyrafluprole et le pyriprole ;
(3) les modulateurs des canaux sodiques/bloquants des canaux sodiques dépendants de la tension, tels que par exemple
les pyréthroïdes, p. ex. l'acrinathrine, l'alléthrine (d-cis-trans, d-trans), la bifenthrine, la bioalléthrine, la bioalléthrine-S-cyclopentényle, la bioresméthrine, la cycloprothrine, la cyfluthrine (bêta-), la cyhalothrine (gamma-, lambda-), la cyperméthrine (alpha-, bêta-, thêta-, zêta-), la cyphénothrine [isomères (1*R)-trans*]*,* la deltaméthrine, la diméfluthrine, l'empenthrine [isomères (*EZ*)-(1*R*)], l'esfenvalérate, l'étofenprox, la fenpropathrine, le fenvalérate, le flucythrinate, la fluméthrine, le fluvalinate (tau-), l'halfenprox, l'imiprothrine, la métofluthrine, la perméthrine, la phénothrine [isomère (1*R*)-trans], la pralléthrine, la profluthrine, la pyréthrine (pyrèthre), la resméthrine, RU 15525, le silafluofen, la téfluthrine, la tétraméthrine [isomères (1*R*)], la tralométhrine, la transfluthrine et ZXI 8901 ; ou
DDT ; ou le méthoxychlor ;
(4) les agonistes des récepteurs d'acétylcholine nicotinergiques, tels que par exemple
les néonicotinoïdes, p. ex. l'acétamiprid, la clothianidine, le dinotéfurane, l'imidacloprid, le nitenpyram, le thiacloprid, le thiaméthoxam ; ou
la nicotine ;
(5) les modulateurs des récepteurs d'acétylcholine allostériques (agonistes), tels que par exemple les spinosynes, p. ex. le spinétoram et le spinosad ;
(6) les activateurs des canaux chlorure, tels que par exemple
les avermectines/milbémycines, p. ex. l'abamectine, le benzoate d'émamectine, la lépimectine et la milbémectine ;
(7) les analogues de l'hormone juvénile, p. ex. l'hydroprène, le kinoprène, le méthoprène ; ou le fénoxycarb ; le pyriproxyfen ;
(8) les agents actifs à mécanisme d'action inconnu ou non spécifique, tels que par exemple
les agents fumigants, p. ex. le bromure de méthyle et d'autres halogénures d'alkyle ; ou
la chloropicrine ; le fluorure de sulfuryle ; le borax ; le tartrate d'antimoine et de potassium ;
(9) les anorexigènes sélectifs, p. ex. la pymétrozine ; ou le flonicamide ;
(10) les inhibiteurs de croissance des acariens, p. ex. la clofentézine, la diflovidazine, l'hexythiazox, l'étoxazole ;
(11) les perturbateurs microbiens de la membrane intestinale des insectes, tels que p. ex. *Bacillus thuringiensis* sous-espèce *israelensis, Bacillus sphaericus, Bacillus thuringiensis* sous-espèce *aizawai, Bacillus thuringiensis* sous-espèce *kurstaki, Bacillus thuringiensis* sous-espèce *tenebrionis,* et les protéines végétales BT, p. ex. Cry1Ab, Cry1Ac, Cry1Fa, Cry2Ab, mCry3A, Cry3Ab, Cry3Bb, Cry34/35Ab1 ;
(12) les inhibiteurs de la phosphorylation oxydative, les perturbateurs d'ATP, tels que par exemple le diafenthiuron ; ou
les composés d'organoétain, p. ex. l'azocyclotine, la cyhexatine, l'oxyde de fenbutatine ; ou
le propargite ; le tétradifon ;
(13) les découpleurs de la phosphorylation oxydative par interruption des gradients de protons H, tels que par exemple le chlorfénapyr et DNOC ;
(14) les antagonistes des récepteurs d'acétylcholine nicotinergiques, tels que par exemple le bensultap, le cartap (chlorhydrate de), le thiocylam et le thiosultap (-sodium) ;
(15) les inhibiteurs de la biosynthèse de chitine, de type 0, tels que par exemple les benzoylurées, p. ex. le bistrifluron, le chlorfluazuron, le diflubenzuron, le flucycloxuron, le flufénoxuron, l'hexaflumuron, le lufénuron, le novaluron, le noviflumuron, le téflubenzuron et le triflumuron ;
(16) les inhibiteurs de la biosynthèse de chitine, de type 1, tels que par exemple la buprofézine ;
(17) les agents actifs perturbant la mue, tels que par exemple la cyromazine ;
(18) les agonistes/perturbateurs d'ecdysone, tels que par exemple
les diacylhydrazines, p. ex. le chromafénozide, l'halofénozide, le méthoxyfénozide et le tébufénozide ;
(19) les agonistes octopaminergiques, tels que par exemple l'amitraz ;
(20) les inhibiteurs du complexe III du transport d'électrons, tels que par exemple l'hydraméthylnon ; l'acéquinocyl ; le fluacrypyrim ;
(21) les inhibiteurs du complexe I du transport d'électrons, par exemple du groupe des acaricides METI, p. ex. le fénazaquin, le fenpyroximate, le pyrimidifen, le pyridaben, le tébufenpyrad, le tolfenpyrad ; ou la roténone (Derris);
(22) les bloquants des canaux sodiques dépendants de la tension, p. ex. l'indoxacarb ; la métaflumizone ;
(23) les inhibiteurs d'acétyl-CoA-carboxylase, tels que par exemple les dérivés de l'acide tétronique, p. ex. le spirodiclofen et le spiromésifen ; ou les dérivés de l'acide tétramique, p. ex. le spirotétramate ;
(24) les inhibiteurs du complexe IV du transport d'électrons, tels que p. ex. les phosphines, p. ex. le phosphure d'aluminium, le phosphure de calcium, la phosphine, le phosphure de zinc ; ou le cyanure ;
(25) les inhibiteurs du complexe II du transport d'électrons, p. ex. le cyénopyrafen ;
(28) les effecteurs du récepteur de ryanodine, tels que par exemple les diamides, p. ex. le flubendiamide, le chlorantraniliprole (rynaxypyr), le cyantraniliprole (cyazypyr), ainsi que le 3-bromo-N-{2-bromo-4-chloro-6-[(1-cyclopropyléthyl)carbamoyl]phényl}-1-(3-chloropyridin-2-yl)-1H-pyrazol-5-carboxamide ou le carboxylate de méthyl-2-[3,5-dibromo-2-({[3-bromo-1-(3-chloropyridin-2-yl)-1H-pyrazol-5-yl]carbonyl}amino)benzoyl]-1,2-diméthylhydrazine ;
d'autres agents actifs de mécanismes d'action inconnu, tels que par exemple l'azadirachtine, l'amidoflumet, le benzoximate, le bifénazate, le chinométhionate, le cryolite, le cyflumétofen, le dicofol, la fluensulfone (5-chloro-2-[(3,4,4-trifluorobut-3-én-1-yl)sulfonyl]-1,3-thiazole), le flufénérim, le pyridalyle et le pyrifluquinazon ; ainsi que les préparations à base de Bacillus firmus (1-1582, BioNeem, Votivo), ainsi que les composés actifs connus suivants :
la 4-{[(6-bromopyrid-3-yl)méthyl](2-fluoroéthyl)amino}furan-2(5H)-one, la 4-{[(6-fluoropyrid-3-yl)méthyl](2,2-difluoroéthyl)amino}furan-2(5H)-one, la 4-{[(2-chloro-1,3-thiazol-5-yl)méthyl](2-fluoroéthyl)amino}furan-2(5H)-one, la 4-{[(6-chloropyrid-3-yl)méthyl](2-fluoroéthyl)amino}furan-2(5H)-one, la 4-{[(6-chloropyrid-3-yl)méthyl](2,2-difluoroéthyl)amino}furan-2(5H)-one, la 4-{[(6-chloro-5-fluoropyrid-3-yl)méthyl](méthyl)amino}furan-2(5H)-one, la 4-{[(5,6-dichloropyrid-3-yl)méthyl](2-fluoroéthyl)amino}furan-2(5H)-one, la 4-{[(6-chloro-5-fluoropyrid-3-yl)méthyl](cyclopropyl)amino}furan-2(5H)-one, la 4-{[(6-chloropyrid-3-yl)méthyl](cyclopropyl)amino}furan-2(5H)-one, la 4-{[(6-chloropyrid-3-yl)méthyl](méthyl)amino}furan-2(5H)-one, le [(6-chloropyridin-3-yl)méthyl](méthyl)oxido-λ⁴-sulfanylidène-cyanamide, le [1-(6-chloropyridin-3-yl)éthyl](méthyl)oxydo-λ⁴-sulfanylidène-cyanamide et ses diastéréomères (A) et (B) 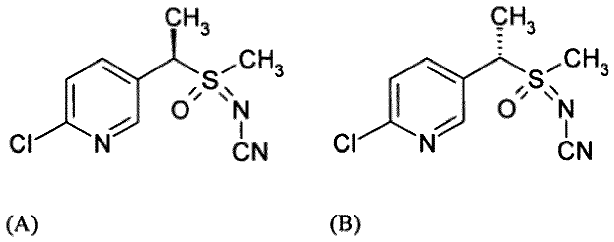
le [(6-trifluorométhylpyridin-3-yl)méthyl](méthyl)oxido-λ⁴-sulfanylidène-cyanamide, le sulfoxaflor, la 11-(4-chloro-2,6-diméthylphényl)-12-hydroxy-1,4-dioxa-9-azadispiro[4.2.4.2]tétradéc-11-én-10-one, la 3-(4'-fluoro-2,4-diméthylbiphényl-3-yl)-4-hydroxy-8-oxa-1-azaspiro[4.5]déc-3-én-2-one, la 1-[2-fluoro-4-méthyl-5-[(2,2,2-trifluoroéthyl)sulfinyl]phényl]-3-(trifluorométhyl)-1H-1,2,4-triazol-5-amine,
le carboxylate de [(3S,4aR,12R,12aS,12bS)-3-[(cyclopropylcarbonyl)oxy]-6,12-dihydroxy-4,12b-diméthyl-11-oxo-9-(pyridin-3-yl)-1,3,4,4a,5,6,6a,12,12a,12b-décahydro-2H,11H-benzo[f]pyrano[4,3-b]chromén-4-yl]méthylcyclopropane,
le 2-cyano-3-(difluorométhoxy)-N,N-diméthylbenzènesulfonamide,
le 2-cyano-3-(difluorométhoxy)-N-méthylbenzènesulfonamide, le 2-cyano-3-(difluorométhoxy)-N-éthylbenzènesulfonamide, le 4-(difluorométhoxy)-N-éthyl-N-méthyl-1,2-benzothiazol-3-amine-1,1-dioxyde et
la N-[1-(2,3-diméthylphényl)-2-(3,5-diméthylphényl)éthyl]-4,5-dihydro-1,3-thiazol-2-amine,
et/ou au moins un agent actif fongicide supplémentaire choisi dans le groupe constitué par :
(1) les inhibiteurs de la biosynthèse d'ergostérol, tels que par exemple l'aldimorph, l'azaconazole, le bitertanol, le bromuconazole, le cyproconazole, le diclobutrazole, le difénoconazole, le diniconazole, le diniconazol-M, le dodémorph, l'acétate de dodémorph, l'époxiconazole, l'étaconazole, le fénarimol, le fenbuconazole, le fenhexamide, la fenpropidine, le fenpropimorph, le fluquinconazole, le flurprimidol, le flusilazol, le flutriafol, le furconazole, le furconazole-cis, l'hexaconazole, l'imazalil, le sulfate d'imazalil, l'imibenconazole, l'ipconazole, le metconazole, le myclobutanil, la naftifine, le nuarimol, l'oxpoconazole, le paclobutrazole, le péfurazoate, le penconazole, la pipéraline, le prochloraz, le propiconazole, le prothioconazole, le pyributicarb, le pyrifénox, le quinconazole, le siméconazole, la spiroxamine, le tébuconazole, la terbinafine, le tétraconazole, le triadiméfon, le triadiménol, le tridémorph, le triflumizole, la triforine, le triticonazole, l'uniconazole, l'uniconazole-p, le viniconazole, le voriconazole, le 1-(4-chlorophényl)-2-(1H-1,2,4-triazol-1-yl)cycloheptanol, le méthyl-1-(2,2-diméthyl-2,3-dihydro-1H-indén-1-yl)-1H-imidazole-5-carboxylate, le N'-{5-(difluorométhyl)-2-méthyl-4-[3-(triméthylsilyl)propoxy]phényl}-N-éthyl-N-méthylimidoformamide, le N-éthyl-N-méthyl-N'-{2-méthyl-5-(trifluorométhyl)-4-[3-(triméthylsilyl)propoxy]phényl}imidoformamide et l'O-[1-(4-méthoxyphénoxy)-3,3-diméthylbutan-2-yl]-1H-imidazole-1-carbothioate ;
(2) les inhibiteurs de la respiration (inhibiteurs de la chaîne respiratoire), tels que par exemple le bixafen, le boscalid, la carboxine, le diflumétorim, le fenfuram, le fluopyram, le flutolanil, le fluxapyroxad, le furametpyr, le furmecyclox, l'isopyrazam, mélange du racémat syn-épimère 1RS,4SR,9RS et du racémat anti-épimère 1RS,4SR,9SR, l'isopyrazam (racémat anti-épimère), l'isopyrazam (énantiomère anti-épimère 1R,4S,9S), l'isopyrazam (énantiomère anti-épimère 1S,4R,9R), l'isopyrazam (racémat syn-épimère 1RS,4SR,9RS) l'isopyrazam (énantiomère syn-épimère 1R,4S,9R), l'isopyrazam (énantiomère syn-épimère 1S,4R,9S), le mépronil, l'oxycarboxine, le penflufen, le penthiopyrad, le sédaxane, le thifluzamide, le 1-méthyl-N-[2-(1,1,2,2-tétrafluoroéthoxy)phényl]-3-(trifluorométhyl)-1H-pyrazole-4-carboxamide, le 3-(difluorométhyl)-1-méthyl-N-[2-(1,1,2,2-tétrafluoroéthoxy)phényl]-1H-pyrazole-4-carboxamide, le 3-(difluorométhyl)-N-[4-fluoro-2-(1,1,2,3,3,3-hexafluoropropoxy)phényl]-1-méthyl-1H-pyrazole-4-carboxamide et le N-[1-(2,4-dichlorophényl)-1-méthoxypropan-2-yl]-3-(difluorométhyl)-1-méthyl-1H-pyrazole-4-carboxamide ;
(3) les inhibiteurs de la respiration (inhibiteurs de la chaîne respiratoire) agissant sur le complexe III de la chaîne respiratoire, tels que par exemple l'amétoctradine, l'amisulbrom, l'azoxystrobine, le cyazofamide, la dimoxystrobine, l'énestroburine, la famoxadone, la fénamidone, la fluoxastrobine, le krésoxim-méthyl, la métominostrobine, l'orysastrobine, la picoxystrobine, la pyraclostrobine, la pyramétostrobine, la pyraoxystrobine, le pyribencarb, la trifloxystrobine, le (2E)-2-(2-{[6-(3-chloro-2-méthylphénoxy)-5-fluoropyrimidin-4-yl]oxy}phényl)-2-(méthoxyimino)-N-méthyléthanamide, le (2E)-2-(méthoxyimino)-N-méthyl-2-(2-{[({(1E)-1-[3-(trifluorométhyl)phényl]éthylidène}amino)oxy]méthyl}phé nyl)éthanamide, le (2E)-2-(méthoxyimino)-N-méthyl-2-{2-[(E)-({1-[3-(trifluorométhyl)phényl]éthoxy}imino)méthyl]phényl}étha namide, le (2E)-2-{2-[({[(1E)-1-(3-{[(E)-1-fluoro-2-phényléthényl]oxy}phényl)éthylidène]amino}oxy)méthyl]ph ényl}-2-(méthoxyimino)-N-méthyléthanamide, le (2E)-2-{2-[({[(2E,3E)-4-(2,6-dichlorophényl)but-3-én-2-ylidène]amino}oxy)méthyl]phényl}-2-(méthoxyimino)-N-méthyléthanamide, le 2-chloro-N-(1,1,3-triméthyl-2,3-dihydro-1H-indén-4-yl)pyridine-3-carboxamide, la 5-méthoxy-2-méthyl-4-(2-{[({(1E)-1-[3-(trifluorométhyl)phényl]éthylidène}amino)oxy]méthyl}phé nyl)-2,4-dihydro-3H-1,2,4-triazol-3-one, le (2E)-2-{2-[({cyclopropyl[(4-méthoxyphényl)imino]méthyl}sulfanyl)méthyl]phényl}-3-méthoxyprop-2-énoate de méthyle, le N-(3-éthyl-3,5,5-triméthylcyclohexyl)-3-(formylamino)-2-hydroxybenzamide, le 2-{2-[(2,5-diméthylphénoxy)méthyl]phényl}-2-méthoxy-N-méthylacétamide et le (2R)-2-{2-[(2,5-diméthylphénoxy)méthyl]phényl}-2-méthoxy-N-méthylacétamide ;
(4) les inhibiteurs de la mitose et de la division cellulaire, tels que par exemple le bénomyl, le carbendazim, le chlorfénazole, le diéthofencarb, l'éthaboxam, le fluopicolid, le fubéridazole, le pencycuron, le thiabendazole, le thiophanate-méthyl, le thiophanate, le zoxamide, la 5-chloro-7-(4-méthylpipéridin-1-yl)-6-(2,4,6-trifluorophényl)[1,2,4]triazolo[1,5-a]pyrimidine et la 3-chloro-5-(6-chloropyridin-3-yl)-6-méthyl-4-(2,4,6-trifluorophényl)pyridazine ;
(5) les composés à activité multi-site, tels que par exemple le mélange de Bordeaux, le captafol, le captane, le chlorothalonil, les préparations à base de cuivre telles que l'hydroxyde de cuivre, le naphténate de cuivre, l'oxyde de cuivre, l'oxychlorure de cuivre, le sulfate de cuivre, le dichlofluanide, le dithianon, la dodine, la base libre de dodine, le ferbam, le fluorofolpet, le folpet, la guazatine, l'acétate de guazatine, l'iminoctadine, l'albésilate d'iminoctadine, le triacétate d'iminoctadine, le mankupfer, le mancozeb, le maneb, le métiram, le métiram de zinc, l'oxine-cuivre, la propamidine, le propineb, le soufre et les préparations à base de soufre telles que par exemple le polysulfure de calcium, le thiram, le tolylfluanide, le zineb et le ziram ;
(6) les inducteurs de résistance, tels que par exemple l'acibenzolar-S-méthyl, l'isotianil, le probénazole et le tiadinil ;
(7) les inhibiteurs de la biosynthèse d'acides aminés et de protéines, tels que par exemple l'andoprim, la blasticidine-S, le cyprodinil, la kasugamycine, le chlorhydrate de kasugamycine hydraté, le mépanipyrim et le pyriméthanil ;
(8) les inhibiteurs de la production d'ATP, tels que par exemple l'acétate de fentine, le chlorure de fentine, l'hydroxyde de fentine et le silthiofam ;
(9) les inhibiteurs de synthèse de la paroi cellulaire, tels que par exemple le benthiavalicarb, le diméthomorph, le flumorph, l'iprovalicarb, le mandipropamide, les polyoxines, le polyoxorim, la validamycine A et le valifénalate ;
(10) les inhibiteurs de la synthèse de lipides et de membranes, tels que par exemple le biphényle, le chloroneb, le dicloran, l'édifenphos, l'étridiazole, l'iodocarb, l'iprobenfos, l'isoprothiolan, le propamocarb, le chlorhydrate de propamocarb, le prothiocarb, le pyrazophos, le quintozène, le tecnazène et le tolclofos-méthyl ;
(11) les inhibiteurs de la biosynthèse de mélanine, tels que par exemple le carpropamide, le diclocymet, le fénoxanil, le phthalide, la pyroquilone et le tricyclazole ;
(12) les inhibiteurs de la synthèse d'acides nucléiques, tels que par exemple le bénalaxyl, le bénalaxyl-M (kiralaxyl), le bupirimate, le clozylacon, le diméthirimol, l'éthirimol, le furalaxyl, l'hymexazole, le métalaxyl, le métalaxyl-M (méfénoxam), l'ofurace, l'oxadixyl, l'acide oxolique ;
(13) les inhibiteurs de la transduction de signaux, tels que par exemple le chlozolinate, le fenpiclonil, le fludioxonil, l'iprodione, la procymidone, le quinoxyfen et la vinclozoline ;
(14) les découpleurs, tels que par exemple le binapacryl, le dinocap, la férimzone, le fluazinam et le meptyldinocap ;
(15) d'autres composés, tels que par exemple le benthiazole, la béthoxazine, la capsimycine, la carvone, le chinométhionate, la chlazafénone, le cufraneb, le cyflufénamide, le cymoxanil, le cyprosulfamide, le dazomet, le débacarb, le dichlorophène, la diclomézine, le difenzoquat, le méthylsulfate de difenzoquat, la diphénylamine, l'écomat, la fenpyrazamine, le flumétover, le fluoromid, le flusulfamide, le flutianil, le fosétyl-aluminium, le fosétyl-calcium, le fosétyl-sodium, l'hexachlorobenzène, l'irumamycine, le méthasulfocarb, l'isothiocyanate de méthyle, la métrafénone, la mildiomycine, la natamycine, le diméthyldithiocarbamate de nickel, le nitrothal-isopropyl, l'octhilinone, l'oxamocarb, l'oxyfenthiine, le pentachlorophénol et ses sels, la phénothrine, l'acide phosphorique et ses sels, le fosétylate de propamocarb, la propanosine-sodium, le proquinazide, la pyrrolnitrine, la tébufloquine, le técloftalam, le tolnifanid, le triazoxyde, le trichlamide, le zarilamide, la 1-(4-{4-[(5R)-5-(2,6-difluorophényl)-4,5-dihydro-1,2-oxazol-3-yl]-1,3-thiazol-2-yl}pipéridin-1-yl)-2-[5-méthyl-3-(trifluorométhyl)-1H-pyrazol-1-yl]éthanone, la 1-(4-{4-[(5S)-5-(2,6-difluorophényl)-4,5-dihydro-1,2-oxazol-3-yl]-1,3-thiazol-2-yl}pipéridin-1-yl)-2-[5-méthyl-3-(trifluorométhyl)-1H-pyrazol-1-yl]éthanone, la 1-(4-{4-[5-(2,6-difluorophényl)-4,5-dihydro-1,2-oxazol-3-yl]-1,3-thiazol-2-yl}pipéridin-1-yl)-2-[5-méthyl-3-(trifluorométhyl)-1H-pyrazol-1-yl]éthanone, le 1-(4-méthoxyphénoxy)-3,3-diméthylbutan-2-yl-1H-imidazol-1-carboxylate, la 2,3,5,6-tétrachloro-4-(méthylsulfonyl)pyridine, la 2,3-dibutyl-6-chlorothiéno[2,3-d]pyrimidin-4(3H)-one, la 2-[5-méthyl-3-(trifluorométhyl)-1H-pyrazol-1-yl]-1-(4-{4-[(5R)-5-phényl-4,5-dihydro-1,2-oxazol-3-yl]-1,3-thiazol-2-yl}pipéridin-1-yl)éthanone, la 2-[5-méthyl-3-(trifluorométhyl)-1H-pyrazol-1-yl]-1-(4-{4-[(5S)-5-phényl-4,5-dihydro-1,2-oxazol-3-yl]-1,3-thiazol-2-yl}pipéridin-1-yl)éthanone, la 2-[5-méthyl-3-(trifluorométhyl)-1H-pyrazol-1-yl]-1-{4-[4-(5-phényl-4,5-dihydro-1,2-oxazol-3-yl)-1,3-thiazol-2-yl]pipéridin-1-yl}éthanone, la 2-butoxy-6-iodo-3-propyl-4H-chromén-4-one, la 2-chloro-5-[2-chloro-1-(2,6-difluoro-4-méthoxyphényl)-4-méthyl-1H-imidazol-5-yl]pyridine, le 2-phénylphénol et ses sels, le 3,4,5-trichloropyridine-2,6-dicarbonitrile, la 3-[5-(4-chlorophényl)-2,3-diméthyl-1,2-oxazolidin-3-yl]pyridine, la 3-chloro-5-(4-chlorophényl)-4-(2,6-difluorophényl)-6-méthylpyridazine, la 4-(4-chlorophényl)-5-(2,6-difluorophényl)-3,6-diméthylpyridazine, le 5-amino-1,3,4-thiadiazole-2-thiol, le 5-chloro-N'-phényl-N'-(prop-2-yn-1-yl)thiophène-2-sulfonohydrazide, la 5-méthyl-6-octyl[1,2,4]triazolo[1,5-a]pyrimidin-7-amine, le (2Z)-3-amino-2-cyano-3-phénylprop-2-énoate d'éthyle, le N-(4-chlorobenzyl)-3-[3-méthoxy-4-(prop-2-yn-1-yloxy)phényl]propanamide, le N-[(4-chlorophényl)(cyano)méthyl]-3-[3-méthoxy-4-(prop-2-yn-1-yloxy)phényl]propanamide, le N-[(5-bromo-3-chloropyridin-2-yl)méthyl]-2,4-dichloropyridine-3-carboxamide, le N-[1-(5-bromo-3-chloropyridin-2-yl)éthyl]-2,4-dichloropyridine-3-carboxamide, le N-[1-(5-bromo-3-chloropyridin-2-yl)éthyl]-2-fluoro-4-iodopyridine-3-carboxamide, le N-{(E)-[(cyclopropylméthoxy)imino][6-(difluorométhoxy)-2,3-difluorophényl]méthyl}-2-phénylacétamide, le N-{(Z)-[(cyclopropylméthoxy)imino][6-(difluorométhoxy)-2,3-difluorophényl]méthyl}-2-phénylacétamide, le N-méthyl-2-(1-{[5-méthyl-3-(trifluorométhyl)-1H-pyrazol-1-yl]acétyl}pipéridin-4-yl)-N-(1,2,3,4-tétrahydronaphtalén-1-yl)-1,3-thiazol-4-carboxamide, le N-méthyl-2-(1-{[5-méthyl-3-(trifluorométhyl)-1H-pyrazol-1-yl]acétyl}pipéridin-4-yl)-N-[(1R)-1,2,3,4-tétrahydronaphtalén-1-yl]-1,3-thiazole-4-carboxamide, le N-méthyl-2-(1-{[5-méthyl-3-(trifluorométhyl)-1H-pyrazol-1-yl]acétyl}pipéridin-4-yl)-N-[(1S)-1,2,3,4-tétrahydronaphtalén-1-yl]-1,3-thiazol-4-carboxamide, le pentyl-{6-[({[(1-méthyl-1H-tétrazol-5-yl)(phényl)méthylidène]amino}oxy)méthyl]pyridin-2-yl}carbamate, l'acide phénazine-1-carboxylique, le quinolin-8-ol et le sulfate de quinolin-8-ol (2:1) ;
(16) d'autres composés, tels que par exemple le 1-méthyl-3-(trifluorométhyl)-N-[2'-(trifluorométhyl)biphényl-2-yl]-1H-pyrazol-4-carboxamide, le N-(4'-chlorobiphényl-2-yl)-3-(difluorométhyl)-1-méthyl-1H-pyrazole-4-carboxamide, le N-(2',4'-dichlorobiphényl-2-yl)-3-(difluorométhyl)-1-méthyl-1H-pyrazole-4-carboxamide, le 3-(difluorométhyl)-1-méthyl-N-[4'-(trifluorométhyl)biphényl-2-yl]-1H-pyrazole-4-carboxamide, le N-(2',5'-difluorobiphényl-2-yl)-1-méthyl-3-(trifluorométhyl)-1H-pyrazole-4-carboxamide, le 3-(difluorométhyl)-1-méthyl-N-[4'-(prop-1-yn-1-yl)biphényl-2-yl]-1H-pyrazole-4-carboxamide, le 5-fluoro-1,3-diméthyl-N-[4'-(prop-1-yn-1-yl)biphényl-2-yl]-1H-pyrazole-4-carboxamide, le 2-chloro-N-[4'-(prop-1-yn-1-yl)biphényl-2-yl]pyridine-3-carboxamide, le 3-(difluorométhyl)-N-[4'-(3,3-diméthylbut-1-yn-1-yl)biphényl-2-yl]-1-méthyl-1H-pyrazole-4-carboxamide, le N-[4'-(3,3-diméthylbut-1-yn-1-yl)biphényl-2-yl]-5-fluoro-1,3-diméthyl-1H-pyrazole-4-carboxamide, le 3-(difluorométhyl)-N-(4'-éthynylbiphényl-2-yl)-1-méthyl-1H-pyrazole-4-carboxamide, le N-(4'-éthynylbiphényl-2-yl)-5-fluoro-1,3-diméthyl-1H-pyrazole-4-carboxamide, le 2-chloro-N-(4'-éthynylbiphényl-2-yl)pyridine-3-carboxamide, le 2-chloro-N-[4'-(3,3-diméthylbut-1-yn-1-yl)biphényl-2-yl]pyridine-3-carboxamide, le 4-(difluorométhyl)-2-méthyl-N-[4'-(trifluorométhyl)biphényl-2-yl]-1,3-thiazole-5-carboxamide, le 5-fluoro-N-[4'-(3-hydroxy-3-méthylbut-1-yn-1-yl)biphényl-2-yl]-1,3-diméthyl-1H-pyrazole-4-carboxamide, le 2-chloro-N-[4'-(3-hydroxy-3-méthylbut-1-yn-1-yl)biphényl-2-yl]pyridine-3-carboxamide, le 3-(difluorométhyl)-N-[4'-(3-méthoxy-3-méthylbut-1-yn-1-yl)biphényl-2-yl]-1-méthyl-1H-pyrazole-4-carboxamide, le 5-fluoro-N-[4'-(3-méthoxy-3-méthylbut-1-yn-1-yl)biphényl-2-yl]-1,3-diméthyl-1H-pyrazole-4-carboxamide, le 2-chloro-N-[4'-(3-méthoxy-3-méthylbut-1-yn-1-yl)biphényl-2-yl]pyridine-3-carboxamide, la (5-bromo-2-méthoxy-4-méthylpyridin-3-yl)(2,3,4-triméthoxy-6-méthylphényl)méthanone et le N-[2-(4-{[3-(4-chlorophényl)prop-2-yn-1-yl]oxy}-3-méthoxyphényl)éthyl]-N2-(méthylsulfonyl)valinamide.

7. Composition contenant au moins un composé de formule (I) selon l'une quelconque des revendications 1 à 2 ou une composition d'agents actifs selon la revendication 6, ainsi qu'au moins un promoteur de pénétration.

8. Composés de formule générale (II-A) selon la revendication 3 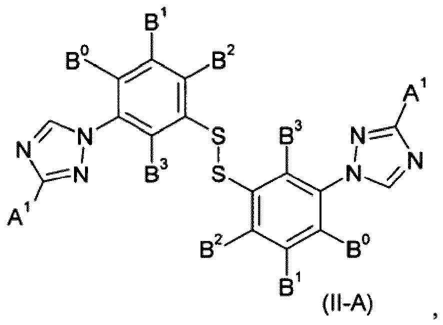 dans laquelle A¹, B⁰, B² ont les significations selon l'une quelconque des revendications 1 à 2, et B¹ et B³ représentent l'hydrogène.

9. Composé de formule générale (III-A) selon la revendication 3 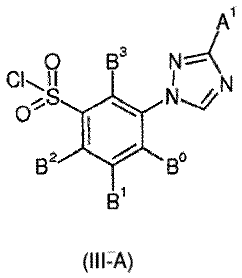 dans laquelle A¹, B⁰ et B² ont les significations selon l'une quelconque des revendications 1 à 2, et B¹ et B³ représentent l'hydrogène.

10. Composés de formule générale (IV-A) selon la revendication 3 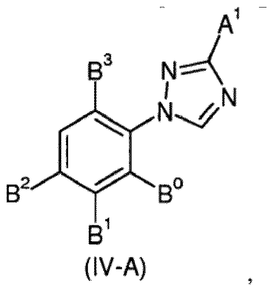 dans laquelle
B¹ et B³ représentent l'hydrogène, et
B⁰ représente hydrogène, méthyle, éthyle, fluor, chlore, méthoxy, cyano, CHF₂, CF₃ ou OCF₃,
B² représente hydrogène, méthyle, éthyle, fluor, chlore, méthoxy, cyano, CHF₂, CF₃ ou OCF₃.

11. Composé de formule générale (V) selon la revendication 3 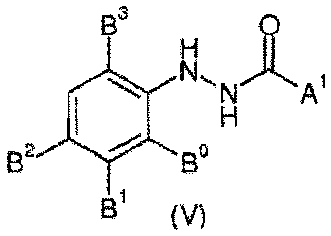 dans laquelle
B¹ et B³ représentent l'hydrogène,
A¹ représente CHF₂,
B⁰ représente méthyle, éthyle, fluor, chlore, méthoxy, cyano, CHF₂, CF₃ ou OCF₃,
B² représente hydrogène, éthyle, fluor, chlore, cyano, CHF₂, CF₃ ou OCF₃.

12. Compositions agrochimiques, **caractérisées en ce qu'**elles contiennent au moins un composé de formule (I) selon les revendications 1 à 2 ou une composition selon l'une quelconque des revendications 6 à 7, ainsi que des extendeurs et/ou des substances tensioactives.

13. Utilisation de composés de formule (I) selon l'une quelconque des revendications 1 à 2 ou d'une composition selon l'une quelconque des revendications 6, 7 ou 12, pour lutter contre les animaux nuisibles dans le cadre de la protection des plantes ou de la protection des matériaux.

14. Composé de formule (I) selon l'une quelconque des revendications 1 à 2 ou composition selon l'une quelconque des revendications 6, 7 ou 12, destiné à une utilisation dans un procédé dans le secteur de la médecine vétérinaire.
